(19) Europäisches Patentamt
European Patent Office
Office européen des brevets

(11) **EP 2 872 637 B1**

(12) **EUROPEAN PATENT SPECIFICATION**

(45) Date of publication and mention
of the grant of the patent:
**26.11.2025 Bulletin 2025/48**

(21) Application number: **13737542.4**

(22) Date of filing: **15.07.2013**

(51) International Patent Classification (IPC):
**C12P 5/02** (2006.01)

(52) Cooperative Patent Classification (CPC):
**C12P 5/023; Y02E 50/30; Y02P 20/133; Y02P 20/59**

(86) International application number:
**PCT/EP2013/002098**

(87) International publication number:
**WO 2014/009026 (16.01.2014 Gazette 2014/03)**

(54) **METHOD AND SYSTEM FOR PRODUCING METHANE USING HIGH GAS FEED RATES**

VERFAHREN UND ANLAGE ZUR HERSTELLUNG VON METHAN UNTER VERWENDUNG EINER HOHEN GASZUFUHR

PROCÉDÉ ET SYSTÈME PERMETTANT DE PRODUIRE DU MÉTHANE AU MOYEN DE FORTS DÉBITS D'INJECTION DE GAZ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.07.2012 EP 12176297**

(43) Date of publication of application:
**20.05.2015 Bulletin 2015/21**

(73) Proprietor: **Krajete GmbH**
**4061 Pasching (AT)**

(72) Inventors:
• **KRAJETE, Alexander**
**4020 Linz (AT)**
• **HERWIG, Christoph**
**A-1130 Wien (AT)**
• **RITTMANN, Simon**
**1030 Wien (AT)**
• **SEIFERT, Arne**
**1030 Wien (AT)**

(74) Representative: **Graf von Stosch**
**Patentanwaltsgesellschaft mbH**
**Triftstraße 5**
**80538 München (DE)**

(56) References cited:
EP-A2- 1 574 581       WO-A1-2012/110256
WO-A1-2014/128300      US-A1- 2009 130 734

• **NORIHIRO NISHIMURA ET AL: "Growth of thermophilic methanogen KN-15 on H2-Co2 under batch and continuous conditions", JOURNAL OF FERMENTATION AND BIOENGINEERING, vol. 72, 1991, pages 280 - 284, XP023573159**
• **YANO ET AL: "Selection of sulfur source for methane production by Methanobacterium thermoautotrophicum", JOURNAL OF FERMENTATION TECHNOLOGY, vol. 64, 1986, pages 383 - 387, XP025705928**
• **SEIFERT ET AL: "Biomethane production from gaseous substrates using methanogenic Archaea", TECHNISCHE UNIVERSITÄT WIEN (POSTER FOR THE 19TH EUROPEAN BIOMASS CONFERENCE AND EXHIBITION, BERLIN, JUNE 2011), 2011, Wien, Austria, pages 1, XP002713916, Retrieved from the Internet <URL:http://www.vt.tuwien.ac.at/fileadmin/t/vt/ Bioverfahrenstechnik/BioProzesstechnik/Bilder/ Projects/Rittmann_Seifert/ Poster_Biomass_Conference_ASE.pdf> [retrieved on 20130930]**

- RITTMANN ET AL: "Quantitative analysis of media dilution rate effects on Methanothermobacter marburgensis grown in continuous culture on H2 and CO2", BIOMASS AND BIOENERGY, vol. 36, 21 November 2011 (2011-11-21), pages 293 - 301, XP028346471
- PENNINGS ET AL: "Isolation and characterization of Methanobacterium thermoautotrophicum deltaH mutants unable to grow under hydrogen-deprived conditions", JOURNAL OF BACTERIOLOGY, vol. 180, 1998, pages 2676 - 2681, XP002713928
- RITTMANN ET AL: "A comprehensive and quantitative review of dark fermentative biohydrogen production", MICROBIAL CELL FACTORIES, vol. 11, 27 August 2012 (2012-08-27), pages 115 - 132, XP021115885
- HERWIG: "Integrated bioprocess development of biohydrogen, bioethanol and biomethane production aiming at a CO2 neutral process", WORLD CONGRESS OF BIOENERGY, DALIAN, CHINA, APRIL 2011, 2011, pages 1 - 3, XP002713917, Retrieved from the Internet <URL:http://www.bitlifesciences.com/wcbe2011/ fullprogram_track6.asp> [retrieved on 20130930]

**Description**

In view of the decreasing reserves of fossil fuels and environmental awareness of global warming and carbon dioxide release, renewable energy sources are moving to the centre of attention. To date, renewable energy sources, i.e. energy from natural resources such as sunlight, wind, rain, tides and geothermal heat, cover about 13% of the global energy demand. Taking into consideration the criteria of sustainability and for achieving the targets of the Kyoto Protocol, it is indispensible to further expand the use of renewable energies. While most of the energy is consumed by a minority of the world's population, the energy demand of developing and emerging countries is increasing rapidly. However, while the world's energy demand is increasing, resources of fossil fuels are decreasing. It has been estimated that - even if consumption remains the same - oil reserves will be exhausted in 40-50 years, natural gas around 70 years and coal around 200 years. This estimation already includes the exploitation of oil and gas reserves which are currently not economical. Thus, energy prices will increase significantly, likely along with social and political conflicts, destruction of the environment and global warming. Beside the importance of fossil oil as energy source, it is also an important and essential resource for other industries such as chemistry and pharmaceutical industry (~10% of the fossil oil). In the future, a new basis for most of the products is required.

However, currently only fossil and nuclear energy is available in storable form which allows for transportation and always meets a temporarily fluctuating energy demand. In contrast, most renewable energy sources are intermittent, not transportable and principally dependent on the meteorological and geographical conditions. This is especially important for places such as for example northern Europe and America which have a high energy demand, but are - beside general daytime variations - confined to a seasonally fluctuating supply of renewable energies, such as solar or wind power. To fully replace fossil and nuclear power with energy from renewable and environmentally friendly sources in the future, energy from these sources will have to be converted into a storable and transportable form.

Most present approaches rely on renewable energy sources which very often suffer from a low energy recovery rate, fluctuating availability and limited efficiency. Another technical obstacle is a missing infrastructure: bioethanol for example is not suitable for many standard vehicle engines. Further, although the majority of energy consumers agree that fossil energy forms should be replaced by renewable energy sources, discussions regularly arise when agricultural area is required for biofuel production from biomass monoculture, such as for corn or sugarcane cultivation, or a vast area is selected for wind farm construction or hydroelectric dam projects, not to mention nuclear power plants which are sometimes also considered to be an alternative to fossil-fuel power sources.

To date, various methods are available to convert energy from one energy form into another, such as from chemical energy into mechanical energy. However, the conversion rates are generally very low and for example in case of fossil fuel, only around 33% of the energy released reaches the end-user, the remaining around 67% of the energy is lost during conversion as heat or radiance, or in form of chemical substances which are produced during the conversion steps and which remain unused and are considered as waste.

One attempt in this direction is described in US 2010/0269498, wherein a system and method for the capture of the waste heat from generators is described. This "cogeneration" approach uses hydrogen as energy source. However, hydrogen has characteristics which limit its broad applicability, such as its explosiveness and tendency to leak from containers due to its small size. Moreover, hydrogen gas is less energy dense by volume when compared to other combustible fuels unless compressed or liquefied which further increases the risk of explosion and leakage and again requires energy input. Also DE 10 2007 037 672 describes a method that uses renewable energy for the production of hydrogen which may later be used for the hydration of carbon dioxide by Fischer-Tropsch synthesis. And another obstacle of using hydrogen as energy carrier is the missing infrastructure, transportation and storage means.

Hence, another energy carrier is needed which may be produced in a cheap, reliable and low-energy-consuming way and is transportable, storable, easy to handle and for which already an infrastructure exists.

Methane ($CH_4$) would have the characteristics of an ideal energy carrier: it is storable and a well-developed infrastructure is already available. However, burning methane from fossil-fuel sources increases the $CO_2$ release and will deplete in the foreseeable future. Thus, it would be highly desirable to provide a closed $CO_2$ cycle by fixing $CO_2$ in a bioprocess, i.e. to use $CO_2$ from a renewable source, yielding a product which can be readily used. Such a product is methane as the main constituent of natural gas. Its content in natural gas varies between 70% and 99%. Methane can be directly produced from carbon dioxide and hydrogen either chemically or biologically. The general reaction equation is $CO_2 + 4H_2 \rightarrow CH_4 + 2H_2O$. Methane burns with oxygen and solely produces water and carbon dioxide ($CO_2$). Natural gas thus exhibits the cleanest burning properties when compared to other fossil fuels like oil or coal and produces less $CO_2$ per energy unit released.

The chemical process is called Sabatier reaction or Sabatier process. This reaction requires hydrogen and carbon dioxide, elevated temperatures and pressure, and a transition metal catalyst. More effectively, nickel or ruthenium on aluminum oxide is used as catalyst. This process has a good turnover rate, although disadvantages are, for example, a low selectivity, e.g. resulting in side reactions at lower temperatures, the susceptibility of the catalyst to impurities and the high energy demand for heat and pressure all of which reduce the overall process economy.

A much better alternative would be the biological conversion of $CO_2$ and $H_2$ into methane by methanogenic microorganisms, such as for example methanogenic archaea. The biological pathway is also known as methanogenesis. These cells are capable of methanogenesis which is a form of anaerobic respiration. Using those methanogenic microorganisms, the reaction $CO_2+4H_2\rightarrow CH_4+2H_2O$ takes place at relatively moderate temperatures, the reaction system is robust with a good impurity tolerance and has a high selectivity even at normal pressure. To provide a method of converting hydrogen and carbon dioxide into methane by methanogenic microorganisms in a reaction vessel, comprising contacting the methanogenic microorganisms with a gas feed comprising hydrogen and carbon dioxide, is an object of the invention. Thereby, methane as an ideal energy carrier is provided. Another objective of the invention is the fixation of carbon dioxide which is released through industrial and agricultural processes and from industrial plants, i.e. waste carbon dioxide.

Usually, biogas has a methane content of between 50% and maximal 70%, the rest consists mainly of carbon dioxide, water vapour and hydrogen sulphide. Biogas already has an application in combined heat power plants which generate both heat and electricity, although it may also be upgraded in a complex and expensive process to be used as natural gas. Thus, hydrogen sulphide gas, water vapour and carbon dioxide have to be separated from biogas to increase the methane content. It is therefore desirable and an objective of the invention to provide a process which directly produces methane in a concentration that is suitable for direct use as natural gas, i.e. suitable for the existing natural gas infrastructure and end-user applications.

Moreover, the method of the present invention is suitable for upgrading low-content methane from biogas processes as it is not inhibited by the methane which is mixed with the educt gases hydrogen and carbon dioxide. It is a further objective of the invention that the process is easy to handle, environmentally friendly, cost-effective and robust.

One example of biological methane production is given in US 4,883,753, wherein a method for methane production by *Methanococcus thermolitotrophicus* and *Methanobacterium thermoautotrophicus* in a 1 L fermenter under continuous conditions is described. The fermenter is supplied with a gas feed of hydrogen and carbon dioxide with the volumetric ratio of hydrogen to carbon dioxide of 80%:20%.

WO 2008/094282 describes a method of converting $CO_2$ gas from biomass fermentation by methanogenic archaea to methane to improve the energy efficiency of ethanol fuel from corn starch in a 1.3 litre fermenter.

EP 1 574 581 relates to a method for preparing methane gas from a $CO_2$-containing starting material.

US 2009/0130734 describes a method of converting $CO_2$ gas produced during industrial processes comprising contacting methanogenic archaea with the $CO_2$ gas under suitable conditions to produce methane.

WO 2012/110256 provides a method of converting hydrogen and carbon dioxide into methane by methanogenic microorganisms in a reaction vessel, comprising contacting the methanogenic microorganisms with in-gas comprising hydrogen and carbon dioxide.

Rittmann et al. (Biomass and Bioenergy, 2012, 36:293-301) describe the quantitative analysis of media dilution rate effects on *Methanothermobacter marburgensis* grown in continuous culture on $H_2$ and $CO_2$.

Yano et al. (Journal of Fermentation Technology, 1986, 64(5):383-387) relates to the selection of a sulfur source for the methane production by *Methanobacterium thermoautotrophicum.*

Nishimura et al. (Journal of Fermentation and Bioengineering, 1991, 72(4):280-284) relates to the growth of thermophilic methanogen KN-15 on $H_2$-$CO_2$ under batch and continuous conditions.

However, there is still a need for an economic method of producing methane which is optimised with respect to the volume of produced methane per volume of culture per time. The inventors have identified the volumetric productivity to be the key parameter for an economically feasible methane production. The volumetric productivity is calculated from equation (1):

$$\text{Volumetric productivity [mmol/L/h]}$$
$$=$$
$$(\text{biomass concentration [g/L]}) \textbf{ x } (\text{specific methane production rate [mmol/g/h]}). \quad (1)$$

As can be learned from the equation developed by the inventor, the volumetric productivity depends on the biomass concentration of the methanogenic microorganisms in the reaction vessel of the invention and on the specific methane production rate. The "specific methane production rate" [mmol/g/h] denotes the amount of methane [mmol] produced per gram of methanogenic microorganism (biomass) [g] per hour [h]. Thus, on the one hand, the cell concentration should be as high as possible in the method and in the reaction vessel of the invention due to the fact that the cells, i.e. the biomass of methanogenic microorganisms, principally function as a catalyst for converting hydrogen and carbon dioxide into methane. On the other hand, the specific methane production rate reflects the productivity per gram biomass of methanogenic microorganism, i.e. the catalytic productivity of the biomass inside the reaction vessel.

The volumetric productivity considers the size of the reactor vessel and therefore directly the investment and energy costs for a production plant. Hence, it is desired to optimise the methane yield with respect of space and time used in the process in order to keep investment costs low and optimally use energy and resource input. The desired methane concentration in the product gas is dependent on the end-application of the produced methane. This may demand energy intensive downstream clean-up operations for uses as vehicle fuel or in natural gas grid. Depending on the kind of use and legal requirements, methane concentrations in the product gas from 88% to 98% are required. Therefore high methane concentrations in the product gas are desired in order to minimise the costs for gas clean-up, or even render clean-up procedures unnecessary at all.

The inventors found out that the volumetric productivity of a method for producing methane may be increased when the method comprises contacting methanogenic microorganisms in a reaction vessel with hydrogen and carbon dioxide, wherein hydrogen and carbon dioxide are introduced into the reaction vessel at a gas feed rate of at least 1.2 vvm in total (total volume of hydrogen and carbon dioxide introduced per volume reactor/reaction medium comprising the methanogenic microorganisms per minute, i.e. per period of time), preferably at least 1.4 vvm, and wherein inside the reaction vessel an absolute pressure of at least 5.0 bar (4.0 barg) is applied. It was surprisingly identified that particularly a total hydrogen and carbon dioxide gas feed rate of at least 1.2 vvm, preferably at least 1.4 vvm, significantly improved the volumetric productivity. One Mol of an ideal gas has a volume of about 22.414 L at 0 °C (= 273,15 K) and 1013.25 hPa (normal atmospheric pressure at sea level). Based on this, 1 L gas contains about 0.04461 mol (= 44.61 mmol) gas molecules. Thus, if 1 L gas is introduced into a reaction vessel comprising 1 L of reaction medium with methanogenic microorganisms per hour, this means that about 44.61 mmol gas comprising hydrogen and carbon dioxide is introduced regardless of the exact composition of the respective gas source. Thereby, a person skilled in the art may easily calculate the amount of gas molecules to be introduced at higher temperatures, higher or lower pressure or higher or lower vvm, or higher or lower of reaction medium. This is also of relevance if real gases are used which contain, apart from main components carbon dioxide and hydrogen, byproducts such as nitrogen, water vapour, carbon monoxide (e.g. in combustion gases) or are methane enriched gases such as biogas (mixture of carbon dioxide and methane). A description of real gases and means and methods for gas composition determination are given below.

Without intention to be bound by one particular theory, the inventors hold the view that since the conversion of hydrogen and carbon dioxide to methane by methanogenic microorganisms takes place in an aqueous medium phase in a continuously stirred tank reactor (CSTR) and both substrates are gasses (i.e. hydrogen and carbon dioxide), it may be assumed, that the gas/liquid mass transfer plays a major role as the rate limiting step if the supply of liquid media components is sufficient. The gas/liquid transfer rate (GTR) itself is dependent on the volumetric mass transfer coefficient ($k_{La}$) and the concentration gradient between gaseous and liquid phase which acts as the driving force for diffusion. The $k_{La}$ is dependent on the stirrer speed and the gas feed rate, i.e. the gas feed rate of hydrogen and carbon dioxide in total which is introduced into the reaction vessel, as well as the concentration gradient of the partial pressure of a gas in the gas phase which can be varied either by the varying the fraction or ratio of that gas, e.g. hydrogen to carbon dioxide volumetric or partial pressure ratio, in the total gas feed or by the pressure inside the reactor. The inventors identified the total gas feed rate of hydrogen and carbon dioxide to be the predominant influencing factor for increasing the volumetric productivity by improved educt gas supply to the methanogenic microorganisms for biomass production and for the conversion of the educt gases hydrogen and carbon dioxide to methane. In addition, an increased pressure inside the reaction vessel, a specific partial pressure ratio of hydrogen gas to carbon dioxide gas and other factors also influence the volumetric productivity and the methane content in the off-gas. In principle, an increase of the total gas feed rate of hydrogen and carbon dioxide and an increase of the absolute pressure inside the reaction vessel to above normal atmospheric pressure increases the volumetric productivity and the percentage amount of methane in the gas which is released from the reaction vessel, i.e. in the off-gas.

Further, although different approaches exist which aim at the utilization of renewable energy sources, these methods - nearly without exception - suffer from low energy recovery rates on the one hand and high production cost on the other hand. Therefore, these applications are far away from being economic. And as long as these methods and systems are not economic, they are not widely applicable or suitable for world-wide energy supply and of course not competitive to fossil fuels or nuclear power. Hence, there is also still a high need for a system and method which provide energy from a renewable or non-renewable energy source in a storable and transportable form, such as methane, that is also inexpensive, has high energy conversion rates and is environmentally friendly, i.e. at least neutral in carbon dioxide release and low in the release of other environmentally harmful or polluting substances.

Despite the production of methane being a central aspect of the invention, the invention also considers the supply of the methanogenesis educts hydrogen and carbon dioxide and also the further processing of the produced off-gas all way to end-user applications. Hence, it is also an object of the invention to provide a method which produces methane using optimally the energy from renewable energy sources, waste products of other industries (e.g. carbon dioxide being released to the atmosphere and thus adding to global warming) and effectively recycles the byproducts in the off-gas except methane, which are e.g. water vapour, $N_2$ and the two in-gases hydrogen and carbon dioxide. Moreover, a system for efficiently producing methane is described.

The inventors recognized that *inter alia* an improved connection and exploitation of educts, intermediates and products for economically using renewable energy sources in order to replace fossil fuels and nuclear energy as main energy sources is needed. Only this would guarantee an optimal use of natural resources and thus of energy. In such a method none of the components would remain unused or be considered as waste. Moreover, especially substances which are considered waste in other industries should be used as valuable educt components in energy production.

Moreover, the method of the invention consumes carbon dioxide and thus reduces the amount of greenhouse gases in the atmosphere. The method of the invention may use carbon dioxide and hydrogen from any source and thus provides a method for reducing the amount of carbon dioxide released to the atmosphere. Thereby, the amount of carbon dioxide in the atmosphere which is suspected by many researches to cause global warming. Another aspect of the invention is the conversion of carbon dioxide contained in waste gas from any industrial process, such as coal gasification, biomass gasification, liquid fuel production by biomass fermentation, ethanol production from biomass and others into methane. Thus, the invention has a double positive effect on the environment as it uses renewable energy in a preferred embodiment and also helps to reduce the amounts of waste and the release of greenhouse gases or other substances in the atmosphere.

For this purpose, the energy from a renewable or non-renewable energy source is to be converted in a sequence of conversion steps into methane as end-product as methane is storable and easy to transport with a well-developed infrastructure being already available. In an example, the method of the invention uses the energy of a renewable or non-renewable energy source, preferably of a renewable energy source for all method applications where energy is needed. Thereby, the energy of a non-renewable source may be converted into methane in periods of low energy consumption or low net utilisation. The energy of the renewable or non-renewable energy source may in particular be used for the electrolysis of water or brine to produce hydrogen on the one hand and oxygen on the other hand. The hydrogen obtained by the electrolysis may then used for a conversion together with carbon dioxide into methane. Thus, the method and system presented herein would already be applicable in common transportation and heating systems, whereas other approaches aiming at solving the problem of energy supply in the future, require the development of new engines, infrastructure or heating systems in the first place and are therefore not applicable immediately.

The inventors have further surprisingly found out that despite the hydrogen and carbon dioxide gas feed rate to be at least 1.2 or 1.4 vvm in total, and the pressure inside the reaction vessel to be at least 5.0 bar (4.0 barg) to support the gas/liquid transfer of hydrogen and carbon dioxide to get in contact with the methanogenic microorganisms inside the reaction medium, and optionally also that a specific ratio of the partial pressure of $H_2$ to the partial pressure of $CO_2$ is the most appropriate way to control the actual fermentation conditions and gas ratio inside the reaction vessel and thus also the provision of educts to the methanogenic microorganisms. The "partial pressure" of an ideal gas in a gas mixture is equal to the pressure it would exert if the same volume is occupied alone at the same temperature. This is because ideal gas molecules are so far apart that they do not interfere with each other. Real gases come very close to this ideal and the gas phase above the liquid phase inside the reaction vessel has to be regarded as real gas phase in a thermodynamic definition. In the prior art, if any, simply the volumetric inflow ratio of both gases has been considered, however, not the real gas supply situation inside the reaction vessel. Thus, no information on the actual gas ratio inside the reaction vessel is provided in the literature. The actual gas ratio inside of the reaction vessel is more important than the simple gas feed ratio since it reflects the real situation as to how the methanogenic microorganism operates. The partial pressures are linked via Henry's law to the solubility of the gaseous components in the liquid phase, giving the dissolved gas concentrations the cell actually experiences during the biological reaction. The partial pressure of a gas dissolved in a liquid is the partial pressure of that gas which would be generated in a gas phase in equilibrium with the liquid at the same temperature. Gases dissolve, diffuse, and react according to their partial pressures, and not necessarily according to their concentrations in a gas mixture. By controlling partial pressure ratio rather than volumetric amounts, the part of methane production by conversion of hydrogen and carbon dioxide can significantly be improved.

Hence, the method of the invention optionally uses at least partially or even completely electric energy which is obtained from a renewable or non-renewable energy source for the electrolysis of water or brine to obtain hydrogen in the first place and optionally also oxygen. Optionally, the hydrogen being used for the production of methane by methanogenic microorganism is at least partially or even completely produced by the electrolysis of water and/or brine. Optionally, the electrolysis of water and/or brine uses at least partially or completely electric energy generated using a renewable and/or non-renewable energy source, preferably from a renewable energy source. Optionally, the carbon dioxide used in the method of producing methane is at least partially or completely provided by oxygen enriched combustion or gasification using oxygen being at least partially or completely provided by electrolysis of water and/or brine. Oxygen enriched combustion and gasification will be explained in detail below. "Partially" in the sense of the invention denotes that at least 1%, 2%, 3%, 4%, 5%, 7.5%, 10%, 20%, 30%, 40%, 50%, 60%, 70%, 75%, 80%, 85%, 90%, 95%, 96%, 97%, 98% or 99% are used of the particular source. Examples of method components being partially or completely (= 100%) used in the method are hydrogen and oxygen produced by the electrolysis of water and/or brine, electric energy generated from a renewable and/or non-renewable energy source or carbon dioxide provided or produced by oxygen enriched combustion or gasification using oxygen.

The carbon dioxide which is used for the methanogenesis may virtually be derived from any source, for example from an industrial source or an industrial process where carbon dioxide is - for example - considered as waste or by-product which is released to the atmosphere, or the carbon dioxide may be produced in oxygen enriched combustion, wherein at least the oxygen obtained by electrolysis of water is used for producing carbon dioxide. Otherwise a release of carbon dioxide would lead to a net increase of the amount of carbon dioxide and thus of greenhouse gases in the atmosphere which probably contributes to global warming. This effect may at least be partially prevented by applying the system and method of the invention which re-uses such "waste" carbon dioxide.

To overcome the above mentioned drawbacks the present invention provides a method for producing methane comprising contacting methanogenic microorganisms in a reaction vessel with hydrogen and carbon dioxide, wherein the gas feed rate of hydrogen and carbon dioxide into the reaction vessel is at least 1.2 vvm or 1.4 vvm in total and wherein inside the reaction vessel an absolute pressure of at least 5.0 bar (4.0 barg) is applied. Preferably, the gas feed rate of hydrogen and carbon dioxide into the reaction vessel is at least 1.2 to 25 vvm in total or 1.4 to 25 vvm in total.

Optionally, the hydrogen used in the method is at least partially provided by electrolysis of water and/or brine. Optionally, the electrolysis of water and/or brine uses at least partially electric energy generated using a renewable and/or non-renewable energy source, wherein preferably the electric energy is generated using a renewable energy source.

Optionally, the renewable energy source comprises solar energy, wind power, wave power, tidal power, water or hydro power, geothermal energy, biomass and biofuel combustion, or combinations thereof.

In another optional embodiment, the carbon dioxide used in the method is at least partially provided by oxygen enriched combustion or gasification using oxygen being at least partially provided by electrolysis of water and/or brine.

In another optional embodiment, the methanogenic microorganisms are methanogenic archaea, optionally the methanogenic archaea is selected from the group consisting of *Methanosarcinia barkeri, Methanobacterium thermoautotrophicus, Methanothermobacter thermoautotrophicus, Methanococcus maripaludis, Methanothermobacter marburgensis, Methanocaldococcus jannaschii,* and mixtures thereof, preferably, the methanogenic archaea is *Methanothermobacter marburgensis,* more preferably, the methanogenic archaea is *Methanothermobacter marburgensis* strain DSM 2133.

In a preferred embodiment of the invention, the gas feed rate of hydrogen and carbon dioxide into the reaction vessel is in the range of 1.2 vvm to 25 vvm in total or 1.4 to 25 vvm in total. Preferably, the gas feed rate is in the range of 1.2 to 2.0 vvm in total.

Further, inside the reaction vessel an absolute pressure above normal atmospheric pressure of at least 5.0 bar (4.0 barg) is applied.

Also disclosed is a the gas feed rate of hydrogen and carbon dioxide into the reaction vessel of at least 1.2 vvm in total, optionally in the range of 1.2 vvm to 3.0 vvm in total, and an absolute pressure of at least 1.5 bar, optionally an absolute pressure in the range of 1.5 bar to 3 bar, to be applied inside the reaction vessel.

Optionally, the method further comprises at least one methane production phase, wherein in the at least one methane production phase the ratio of the partial pressure of hydrogen to the partial pressure of carbon dioxide inside the reaction vessel is maintained at 5:1 or higher (parts hydrogen : parts carbon dioxide). Optionally, the method further comprises at least one cell growth phase, wherein in the at least one cell growth phase the ratio of the partial pressure of hydrogen to the partial pressure of carbon dioxide inside the reaction vessel is maintained at a ratio different from the ratio in the at least one methane production phase.

In an optional embodiment, the volumetric ratio of hydrogen to carbon dioxide in the feed gas which is introduced into the reaction vessel is 4: 1.

In an optional embodiment, the method of the invention comprises one or more of the following steps:

a) generating electric energy using a renewable and/or non-renewable energy source, preferably generating electric energy using a renewable energy source; and/or
b) using partially or completely said electric energy for the electrolysis of water and/or brine, thereby producing hydrogen and/or oxygen; and/or
c) using partially or completely said hydrogen and carbon dioxide, and optionally partially or completely said electric energy, for producing methane by methanogenic microorganisms in a reaction vessel, comprising contacting the methanogenic microorganisms partially or completely with said hydrogen and carbon dioxide.

In a preferred embodiment, the method further comprises step d), wherein partially or completely the above mentioned oxygen is used for producing partially or completely the carbon dioxide usable in step c) by oxygen enriched combustion or gasification. Thus, the carbon dioxide which is usable in step c) for producing methane by methanogenic microorganisms in a reaction vessel is partially or completely obtained by oxygen enriched combustion or gasification.

Alternatively or in addition, the carbon dioxide used in the method is at least partially from or partially derived from an industrial process, is off-gas of the invention, real gas or a mixture thereof. Optionally, the industrial process comprises biogas or biomass fermentation, anaerobic digestion, the electrolysis of water or brine, methanogenesis by methanogenic archaea, oxygen enriched combustion gasification, and/or combinations thereof. Optionally, the industrial process is selected from the group consisting of biogas or biomass fermentation, anaerobic digestion, the electrolysis of water or brine, methanogenesis by methanogenic microorganisms, such as methanogenic archaea, oxygen enriched combustion, gasification, and/or combinations thereof. Preferably, the used electric energy and/or the energy for the industrial process is generated using a renewable energy source or renewable energy device.

In any of the above embodiments of the method of the invention, the renewable energy source may comprise solar energy, wind power, wave power, tidal power, water or hydro power, geothermal energy, biomass and biofuel combustion, or combinations thereof.

The hydrogen which is used for producing methane by methanogenic microorganisms may also at least be partially or completely from and/or is derived from an industrial process, is off-gas, real gas or a mixture thereof. Optionally, the industrial process comprises biogas or biomass fermentation, anaerobic digestion, the electrolysis of water or brine, methanogenesis by methanogenic archaea, oxygen enriched combustion or gasification, and/or combinations thereof. Optionally, the industrial process is selected from the group consisting of biogas or biomass fermentation, anaerobic digestion, the electrolysis of water or brine, methanogenesis by methanogenic archaea, oxygen enriched combustion or gasification, and/or combinations thereof. In another preferred embodiment of the method of the invention, the energy for the industrial process or the electrolysis of water or brine to obtain hydrogen and/or oxygen is derived from a renewable energy source, such as solar energy, wind power, wave power, tidal power, water or hydro power, geothermal energy, biomass and biofuel combustion, or combinations thereof.

In one alternative embodiment of the invention, the method further comprises at least one methane production phase, wherein in the at least one methane production phase the ratio of the partial pressure of hydrogen to the partial pressure of carbon dioxide inside the reaction vessel is maintained at 5:1 or higher (parts hydrogen : parts carbon dioxide), preferably the ratio of the partial pressure of hydrogen to the partial pressure of carbon dioxide is maintained in the range from 7:1 to 30:1 (parts hydrogen : parts carbon dioxide), more preferably in the range from 9:1 to 25:1 (parts hydrogen : parts carbon dioxide), even more preferably in the range from 10:1 to 18:1 (parts hydrogen : parts carbon dioxide), most preferably, the ratio of the partial pressure of hydrogen to the partial pressure of carbon dioxide inside the reaction vessel in the at least one methane production phase is maintained in the range from 12:1 to 17:1 or adjusted to 15±1:1 or 18±1:1 (parts hydrogen : parts carbon dioxide).

Optionally, in the at least one methane production phase, the oxidation reduction potential inside the reaction vessel is maintained below -350 mV, preferably the oxidation reduction potential is maintained in the range from -400 mV to -700 mV, more preferably, the oxidation reduction potential inside the reaction vessel is maintained in the range from -500 mV to -700 mV, most preferably, maintained at a value of -550±50 mV.

In an optional embodiment of the method of the invention, the method further comprises at least one cell growth phase, wherein in the at least one cell growth phase the ratio of the partial pressure of hydrogen to the partial pressure of carbon dioxide inside the reaction vessel is maintained at a ratio different from the ratio in the at least one methane production phase, preferably, the ratio of the partial pressure of hydrogen to the partial pressure of carbon dioxide is lower than in the at least one methane production phase, more preferably, the ratio of the partial pressure of hydrogen to the partial pressure of carbon dioxide is maintained at 4:1 or lower (parts hydrogen : parts carbon dioxide), even more preferably, in the at least one cell growth phase the ratio of the partial pressure of hydrogen to the partial pressure of carbon dioxide is maintained in the range from 0.5:1 to 4:1 or from 0.75:1 to 3.5:1 (parts hydrogen : parts carbon dioxide), also even more preferably, maintained in the range from 1:1 to 2.5:1 (parts hydrogen : parts carbon dioxide), most preferably, the ratio of the partial pressure of hydrogen to the partial pressure of carbon dioxide inside the reaction vessel in the at least one cell growth phase is maintained at 1.5±0.5:1 or 2±1:1 (parts hydrogen : parts carbon dioxide). Further ratios for hydrogen and carbon dioxide for both phases, the methane production phase and the cell growth phase, are given below.

In any of the above mentioned embodiments of the method of the invention, the method further comprises at least one cell growth phase, optionally, the oxidation reduction potential in the at least one cell growth phase is maintained below -300 mV, preferably, the oxidation reduction potential in the at least one cell growth phase is maintained below -350 mV or in the range from -300 mV to -500 mV, even more preferably, the oxidation reduction potential inside the reaction vessel is maintained in the range from -300 mV to -400 mV, most preferably, the oxidation reduction potential in the at least one cell growth phase inside the reaction vessel is maintained at a value of -350±25 mV.

In any above mentioned embodiment, the oxidation reduction potential may be adjusted using a reducing agent, such as sodium sulphide or hydrogen sulphide, or by adjusting the total hydrogen or oxygen gas feed into the reaction vessel.

In one exemplary embodiment, the method of the invention comprises at least one cell growth phase and at least one methane production phase, preferably, the method comprises at least one cell growth phase, wherein the ratio of the partial pressure of hydrogen to the partial pressure of carbon dioxide is maintained at 4:1 or lower (parts hydrogen : parts carbon dioxide), and at least one methane production phase, wherein the ratio of the partial pressure of hydrogen to the partial pressure of carbon dioxide is maintained at 5:1 or higher (parts hydrogen : parts carbon dioxide).

In another optional embodiment, the method of the invention comprises or consists of the following steps in the order given

a) at least one cell growth phase, wherein the ratio of the partial pressure of hydrogen to the partial pressure of carbon dioxide is maintained at a ratio of 4:1 or lower (parts hydrogen : parts carbon dioxide), and

b) at least one methane production phase, wherein the ratio of the partial pressure of hydrogen to the partial pressure of carbon dioxide is maintained at 5:1 or higher (parts hydrogen : parts carbon dioxide).

In any of the above mentioned embodiments of the method of the invention, the pH value inside the reaction vessel in the method is maintained in the range from 5.0 to 8.0, preferably in the range from 5.5 to 7.8, more preferably in the range from 6.4 to 7.6, even more preferably in the range from 6.7 to 7.2, and most preferably the pH inside the reaction vessel is maintained at 7.0 ± 0.5.

In any embodiment of the method of the invention, wherein at least one cell growth phase is comprised in the method, the methanogenic microorganisms are maintained in the at least one cell growth phase until the cell growth of the methanogenic microorganisms in the reaction vessel stagnates or reaches at least a concentration of 3 g biomass per litre, of at least 5 g biomass per litre, of at least 6 g biomass per litre, of at least 7 g biomass per litre, of at least 9 g biomass per litre, of at least 10 g biomass per litre, of at least 12 g biomass per litre, or of at least 15 g biomass per litre (determined as dry weight).

Further described is a culture of methanogenic microorganisms having a biomass concentration of 3 g biomass per litre, of at least 5 g biomass per litre, of at least 6 g biomass per litre, of at least 7 g biomass per litre, of at least 9 g biomass per litre, of at least 10 g biomass per litre, of at least 12 g biomass per litre, or of at least 15 g biomass per litre. Aforementioned biomass concentrations are particularly useful in the reaction medium inside the reaction vessel for the method of producing methane according to the invention. Optionally, cell retention may be applied in order to further increase the biomass in the reaction medium.

In another preferred embodiment of the method of the invention, the methanogenic microorganisms are switched from at least one cell growth phase to at least one methane production phase by adjusting or maintaining the ratio of the partial pressure of hydrogen to the partial pressure of carbon dioxide inside the reaction vessel to or at 5:1 or higher (parts hydrogen : parts carbon dioxide) or from at least one methane production phase to at least one cell growth phase by adjusting or maintaining the ratio of the partial pressure of hydrogen to the partial pressure of carbon dioxide inside the reaction vessel to or at a ratio different from the ratio in the at least one methane production phase, optionally, for switching the methanogenic microorganisms from at least one cell growth phase to at least one methane production phase the oxidation reduction potential is adjusted to or maintained at a value below -350 mV and/or for switching the methanogenic microorganisms from at least one methane production phase to at least one cell growth phase the oxidation reduction potential is adjusted to or maintained at a value below -300 mV, optionally, using a reducing agent, such as sodium sulphide or hydrogen sulphide, by adjusting the oxygen gas feed into the reaction vessel, the hydrogen gas feed into the reaction vessel and/or pH value as will be explained in detail below.

Also disclosed is a system for producing methane comprising at least one device for generating electric energy from a renewable and/or non-renewable energy source, at least one device for producing hydrogen and/or oxygen by the electrolysis of water and/or brine, and at least one bioreactor comprising a reaction vessel suitable for growing, fermenting and/or culturing methanogenic microorganisms and at least one device for providing, measuring and controlling a gas feed of hydrogen and carbon dioxide into the reaction vessel. Optionally, the bioreactor comprises at least one device for the gas feed of carbon dioxide and a separate device for the gas feed of hydrogen.

In the system, the bioreactor may further comprise at least one device for measuring the head pressure and the off-gas concentration, and at least one device for adjusting or maintaining the partial pressure ratio of hydrogen to carbon dioxide inside the reaction vessel.

The system optionally further comprises at least one device for oxygen enriched combustion or gasification for producing carbon dioxide, preferably the device for oxygen enriched combustion or gasification uses the oxygen produced by the electrolysis of water, said carbon dioxide being transferred to the bioreactor.

Optionally, the system further comprises at least one device for recovery, purification, enriching, storing, recycling and/or further processing of off-gas, hydrogen, water, oxygen, chlorine, carbon dioxide, $H_2S$, sodium sulphide, methanogenic microorganisms, used medium and medium components, methane and other substances of the process from the reaction vessel and bioreactor.

In an optional embodiment, the renewable energy source comprises solar energy, wind power, wave power, tidal power, water/hydro power, geothermal energy, biomass and/or biofuel combustion.

The methanogenic microorganisms for which the reaction vessel is to be suitable for growing, fermenting and/or culturing may be methanogenic archaea, optionally the methanogenic archaea may be selected from the group consisting of *Methanosarcinia barkeri, Methanobacterium thermoautotrophicus, Methanothermobacter thermoautotrophicus, Methanococcus maripaludis, Methanothermobacter marburgensis, Methanocaldococcus jannaschii,* and mixtures thereof, preferably, the methanogenic archaea is *Methanothermobacter marburgensis,* more preferably, the methanogenic archaea is *Methanothermobacter marburgensis* strain DSM 2133.

Optionally, the at least one bioreactor of the system further comprises a device for providing, controlling and/or measuring an absolute pressure inside the reaction vessel above normal atmospheric pressure, of at least 1 bar, at least 1.2 bar, at least 1.5 bar, at least 2.0 bar, at least 2.25 bar, at least 2.5 bar, at least 3.0 bar, and at least 3.5 bar, or in the range of 1.5 bar to 1000.0 bar.

The system is thus also suitable for storing energy in the form of methane and allows for performing and/or conducting the method of the invention.

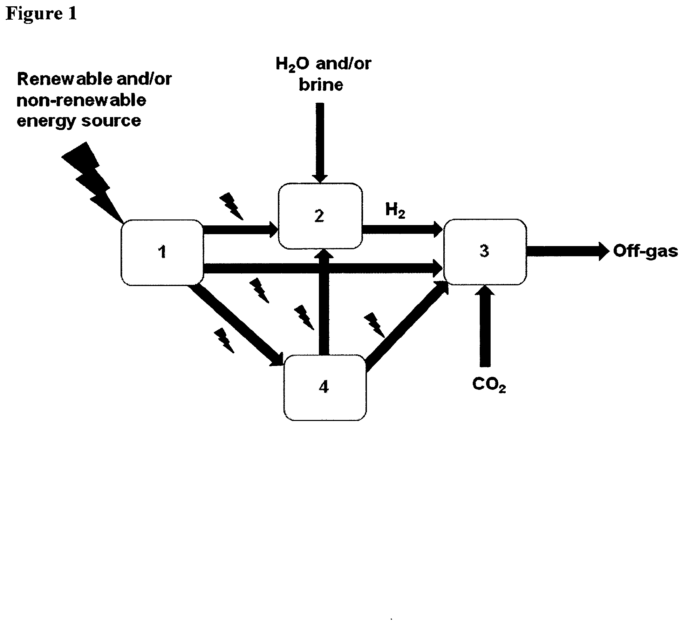

A general set-up of the system is illustrated in Figure 1 and comprises at least one device for generating electric energy from a renewable and/or non-renewable energy source (1), at least one device for producing hydrogen and/or oxygen by the electrolysis of water and/or brine (2), preferably said device for producing hydrogen and/or oxygen by the electrolysis of water and/or brine is an electrolyser, and at least one bioreactor (3) comprising a reaction vessel suitable for growing, fermenting and/or culturing methanogenic microorganisms for converting hydrogen and carbon dioxide into methane and at least one device for providing a gas feed of hydrogen and carbon dioxide into the reaction vessel (Figure 1, further comprising optional component (4) which will be explained below). In Figure 1, hydrogen is provided by the electrolysis of water and/or brine and carbon dioxide is provided from a variable external source. The drawing depicted in Figure 1 shows two separate feed tubes of hydrogen and carbon dioxide, however, in an alternative embodiment, the two gases may also be mixed prior to feed into the reaction vessel comprised in the bioreactor. Preferably, any bioreactor further comprises at least one device for measuring the head pressure and the off-gas concentration and at least one device for adjusting or maintaining the partial pressure ratio of hydrogen to carbon dioxide inside the reaction vessel and/or a device for providing, controlling and/or measuring an absolute pressure inside the reaction vessel of at least 1.5 bar.

In any embodiment of the system it should be noted that more than one device for generating electric energy from a renewable and/or non-renewable energy source (1), more than one device for producing hydrogen and/or oxygen by the electrolysis of water and/or brine (2), preferably electrolyser, and/or more than one bioreactor (3), comprising one or more reaction vessel suitable for growing, fermenting and/or culturing methanogenic microorganisms for converting hydrogen and carbon dioxide into methane and optionally comprising more than one device for providing a gas feed of hydrogen and carbon dioxide into the reaction vessel, may be present in the system. "More than one" denotes two, three, four, five, and so on. Of course, the particular number of the devices for a certain purpose, i.e. for generating electric energy, for producing hydrogen and/or oxygen or the bioreactor may be selected individually. Further, in case that more than one device for generating electric energy from a renewable and/or non-renewable energy source is used in the system of the invention, of course, devices for generating electric energy from different renewable and/or non-renewable energy sources may be used. In case that more than one bioreactor is present in the system of the invention, it is also applicable that one or more bioreactor is used for the cell growth phase and one or more other bioreactor is used for the methane production phase. Even different ratios of the partial pressure of hydrogen to carbon dioxide may be applied in the reaction vessels of bioreactors, which are used for the same phase of the invention, or a different pressure inside the reaction vessels may be applied.

In case of the electrolysis of water, wherein oxygen and hydrogen are produced, both hydrogen and oxygen are separated by means known to a person skilled in the art and discussed in detail below as only the hydrogen should be introduced into the bioreactor, whereas the oxygen is optionally used in gasification or oxygen enriched combustion processed as also discussed in detail below. Optionally the renewable energy source comprises solar energy, wind power, wave power, tidal power, water/hydro power, geothermal energy, biomass and biofuel combustion. Arrows indicate the transfer of one or more compounds (e.g. electric energy, liquid, gaseous and/or solid compounds) between the devices/bioreactor(s) or else of the system. The electric energy from a renewable and/or non-renewable energy source, may be transferred via general power supply lines or cables or else to the at least one device for producing hydrogen and/or oxygen by the electrolysis of water and/or brine (2), preferably said device for producing hydrogen and/or oxygen by the electrolysis of water and/or brine is an electrolyser, and/or to the at least one bioreactor (3) or be stored in an electric energy storage device (4), such as a battery or accumulator or else. In general and applicable to all embodiments of the invention, the electric energy storage device (4), such as a battery or accumulator or else may be any common device suitable for storing electric energy, such as a common battery or common accumulator to be used according to the manufacturer's instructions. From the electric energy storage device (4) the electric energy may be transferred via general power supply lines or cables or else to the at least one device for producing hydrogen and/or oxygen by the electrolysis of water and/or brine (2), preferably said device for producing hydrogen and/or oxygen by the electrolysis of water and/or brine being an electrolyser, and/or to the at least one bioreactor (3). Water and/or brine are provided via general transportation or tubes, pipes or else but also via water tanks transported by transportation systems such as trains, cars, trucks or else. Water in general, and in particular water from rain or water which was purified in order to be suitable for the use in electrolysis may be stored in water tanks, cisterns, artificial lakes, reservoirs or else.

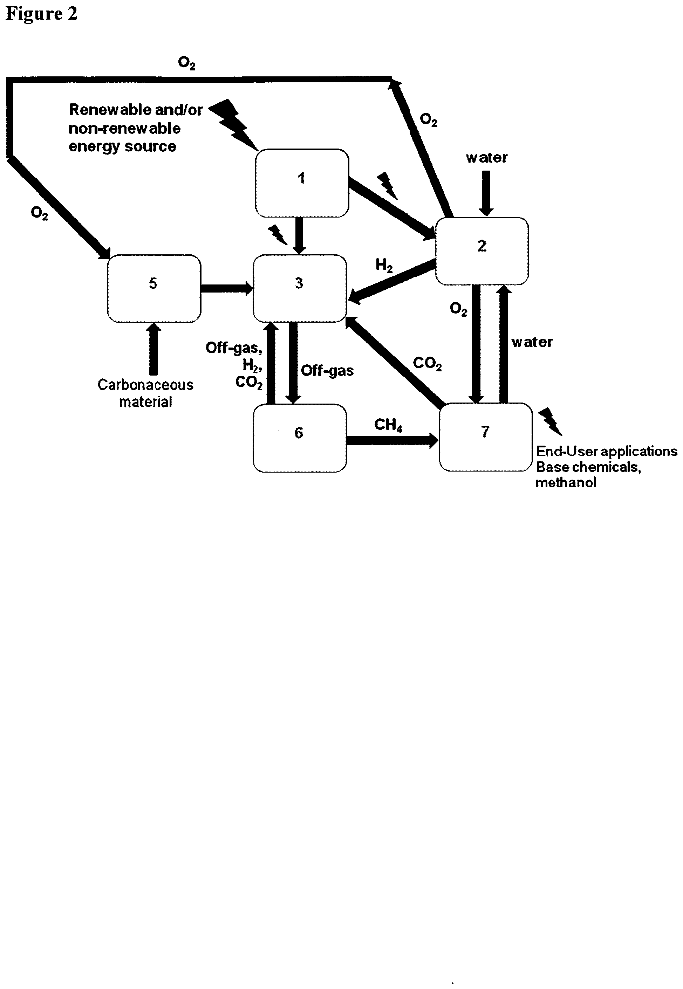

The hydrogen obtained by electrolysis is partially or completely transferred to the bioreactor (3), where it is converted together with carbon dioxide into methane by methanogenic microorganisms. Hydrogen, carbon dioxide and also other gaseous substances, such as oxygen, may in general be transferred via standard gas transport means, such as tubes, pipes or else. They may also be delivered, for example, in gas cylinders or bottles which allow for storage of carbon dioxide and/or hydrogen before introduction into the bioreactor. Gas cylinders and/or bottles allow not also for storage of carbon dioxide prior to introduction into the bioreactor, such as for example of hydrogen after electrolysis in case of excess production or else, but also for individual transportation of carbon dioxide and/or hydrogen to bioreactors (3) which are - for example - not connected to any gas transport means, such as tubes, pipes or else. The carbon dioxide may be of any source as will be further discussed in detail below, optionally the carbon dioxide used herein is air or carbon dioxide waste gas from an industrial process. The off-gas is removed from the bioreactor (3) via the standard means of the bioreactor (3), such as tubes, pipes or else. These standard means are familiar to a person skilled in biotechnology and can also be taken from the bioreactor manufacturer's instructions. The off-gas comprises methane, water vapour, hydrogen and carbon dioxide but also other gases which are present inside the reaction vessel as described below. The off-gas may be further purified or enriched (by at least partially removing components except methane) for increasing the methane content by methods well known to a person skilled in the art and further discussed in detail below. The off-gas or the methane-enriched off-gas may also be stored in gas cylinders, bottles or else or it may be recycling and/or further processed in order to also use gaseous components other than methane, such as carbon dioxide and/or hydrogen which have not been converted into methane by the methanogenic microorganisms. The hydrogen and/or carbon dioxide comprising off-gas may also be fed back into the bioreactor by standard means and devices or it is transferred into another bioreactor. Or the off-gas, methane or methane-enriched off-gas may be used directly and/or after transportation in gas cylinders, bottles or else or by standard gas transport means, such as tubes, pipes or else, as fuel or energy carrier in end-user applications, for heating devices, vehicles, or be converted into other forms of energy, such as thermal, mechanical, electric energy or else. Methane burns with oxygen, such as comprised in air or produced by electrolysis of water, to carbon dioxide, which may be re-used for methane production in the bioreactor by the methanogenic microorganisms, and water, which may be re-used for electrolysis for hydrogen and oxygen production. The overall process and an exemplary set-up of the optional system of the invention are illustrated in Figure 2. Thereby, none of the substances or components remains unused. Another end-user application of methane is the conversion into methanol via a three step reaction as follows: (i) Steam reforming: $CH_4 + H_2O \leftrightarrow CO + 3H_2$ (requires Ni catalyst and 800 °C), (ii) water shift reaction: $CO + H_2O \leftrightarrow CO_2 + H_2$ (requires Ni catalyst and 800 °C) and (iii) synthesis: $2H_2 + CO \leftrightarrow CH_3OH$. The methanol may further be converted to gasoline by the Mobil process. Methanol may also be used as educt for the production of base chemicals (e.g. olefin via "methanol to olefin process" and access to solid polymers like polypropylene). All these methods are known to a person skilled in the art. Information on the so-called "methanol to olefin process" may for example be obtained from UOP Corporate Offices, Des Plaines, IL, USA) or from Norsk Hydro ASA, Oslo, Norway.

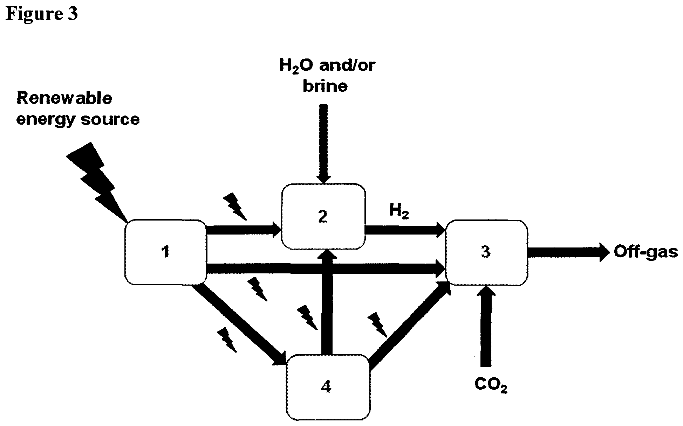

The system may comprise at least one device for generating electric energy from a renewable energy source, optionally the renewable energy source comprises solar energy, wind power, wave power, tidal power, water/hydro power, geothermal energy, biomass and biofuel combustion, at least one device for producing hydrogen and/or oxygen by the electrolysis of water and/or brine, and at least one bioreactor comprising a reaction vessel suitable for growing, fermenting and/or culturing methanogenic microorganisms for converting hydrogen and carbon dioxide into methane. The general set-up of a system is illustrated in Figure 3. All explanations regarding the general set-up of the system as in Figure 1 also apply with respect to the exemplary system in Figures 2, 3 and 4.

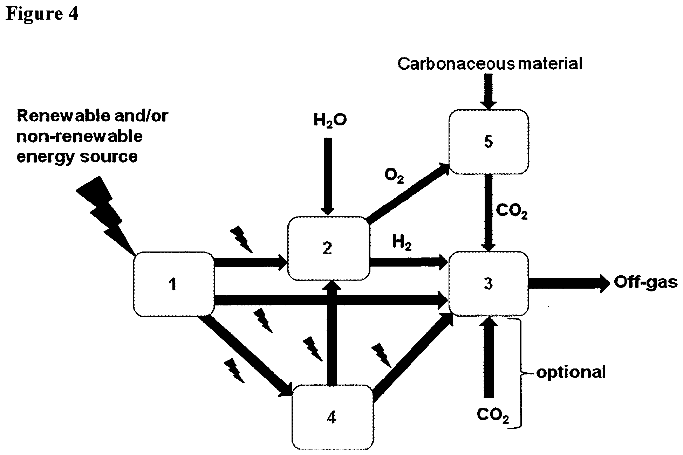

The system may further comprise at least one device for oxygen enriched combustion or gasification (5) for producing carbon dioxide, preferably, the device for oxygen enriched combustion or gasification (5) uses partially or completely the oxygen produced by the electrolysis of water in device (2) for producing carbon dioxide, said carbon dioxide being transferred to the bioreactor (3) via a device for providing, measuring and/or controlling a gas feed of hydrogen and carbon dioxide into the reaction vessel, thereby being transferred to the methanogenic microorganisms inside the reaction vessel comprised in the bioreactor (3). An exemplary scheme comprising at least one device for oxygen enriched combustion (5) or gasification (5) for producing carbon dioxide is illustrated in Figure 4. All explanations regarding the general set-up of the system as in Figure 1, 2 or 3 also apply with respect to the system as in Figure 4. As already described above, hydrogen, carbon dioxide and also other gaseous substances, such as oxygen, may in general be transferred via standard gas transport means, such as tubes, pipes or else, such as the at least one device for providing, measuring and/or controlling a gas feed of hydrogen and carbon dioxide into the reaction vessel. They may also be delivered, for example, in gas cylinders or bottles which allow for storage of the substance before introduction into the bioreactor in case of hydrogen and carbon dioxide or delivered to combustion or gasification in case of the oxygen derived from the electrolysis of water. Gas cylinders and/or bottles not only allow for the storage of carbon dioxide prior to introduction into the bioreactor (3), such as for example of hydrogen after electrolysis in case of excess production or else, but also for the individual transportation of carbon dioxide and/or hydrogen to bioreactors (3) which are - for example - not connected to any gas transport means, such as tubes, pipes or else. Optionally, additional carbon dioxide from any other carbon dioxide source mentioned herein may be introduced into the bioreactor (3) and thereby to the methanogenic microorganisms comprised in the reaction vessel comprised in the bioreactor. This may be applicable in case of low carbon dioxide production by the combustion or gasification device (5). For combustion/oxygen enriched combustion or gasification, carbonaceous material, e.g. petroleum, wood, coal, living and/or dead biomass, is required and may be delivered via standard transportation as known to a person skilled in the art.

The system may further comprise one or more devices for purifying carbon dioxide of any source and also mixtures thereof from contaminants prior to its feed into the reaction vessel of the bioreactor of the invention. For example, such a carbon dioxide purifying device may be present between the device for oxygen enriched combustion or gasification and the bioreactor. Such devices and methods are known to a person skilled in the art, such as a carbon dioxide scrubber, contacting the gas with an absorbing medium which selectively absorbs carbon dioxide, such as amine, monoethanolamine solutions or quicklime absorption, or which selectively absorbs gases other than carbon dioxide from the real gas, activated charcoal or activated coal, by the regenerative carbon dioxide removal system, polymer membrane gas separators, molecular sieves, others and combinations thereof.

In addition, the system may further comprise at least one device for recovery, purification, enriching, storing, recycling and/or further processing of off-gas, hydrogen, water, oxygen, chlorine, electric energy, carbon dioxide, $H_2S$, sodium sulphite, methanogenic microorganisms, used medium and medium components, methane and other substances of the process. These devices for recovery, purification, enriching, storing, recycling and/or further processing are connected to the respective device or to the bioreactor where the above listed substance occurs or can easily obtained. They may be removed from the system using devices and/or means which are suitable for this purpose depending on the nature of the particular substance, such as cables or lines for electric energy or pipes, tubes and else for liquid and/or gaseous substances. "Substances of the process" or "compounds of the process" denote all liquid, gaseous and/or solid substances and also solutions, cells, medium, medium components and suspensions comprising methanogenic microorganisms of the present invention which are introduced into the system and/or produced by the methanogenic microorganisms of the invention or by any other chemical or biological reaction which may occur inside the system of the invention, in particular inside the reaction vessel and/or the device for electrolysis of water or brine. Substances of the process are also substances which occur only as intermediates of the method. Hydrogen, water, oxygen, chlorine, carbon dioxide, $H_2S$, off-gas, sodium sulphite, methanogenic microorganisms, used medium and medium components, methane, electric energy and others are exemplary substances of the process. It is of particular importance in the method and also the system of the invention that all educts, products and intermediates are optimally used. Therefore, especially the off-gas is further processed, methane purified and other components recycled. How especially carbon dioxide, oxygen and hydrogen are recovered from the off-gas and re-used in the method of the invention is described in detail below and in the claims.

Optionally, the bioreactor comprises

a) at least one reaction vessel for fermenting, growing and/or culturing methanogenic microorganisms,
b) at least one device for introducing and removing gaseous substances into the at least one reaction vessel, e.g. at least one device for providing, measuring and/or controlling a gas feed of hydrogen and carbon dioxide into the reaction vessel
c) at least one device for introducing or removing a liquid substance or a cell suspension into or from the bioreactor or reaction vessel,
d) devices for measuring and/or adjusting at least the oxidation reduction potential, temperature, pressure, head pressure, off-gas concentration and content, the partial pressure ratio of hydrogen and carbon dioxide and/or pH value,
e) at least one device for removing off-gas from the at least one reaction vessel,
f) at least one stirring device inside the at least one reaction vessel, and/or
g) at least one device for gas liquid mass transfer, such as an agitator.

The bioreactor may further comprises at least one device for providing, controlling and/or measuring an absolute pressure inside the reaction vessel of above normal atmospheric pressure or of at least 1 bar, 1.2 bar, 1.4 bar, 1.5 bar, 1.6 bar, 1.7 bar, 1.8 bar, or at least 1.9 bar, preferably at least 2.0 bar, 2.1 bar, 2.2 bar, 2.25 bar, 2.3 bar, 2.4 bar, 2.5 bar, 2.6 bar, 2.7 bar, 2.8 bar, 2.9 bar, 3.0 bar, more preferably of at least 3.2 bar, 3.4 bar, 3.6 bar, 3.8 bar, 4.0 bar, 4.25 bar, 4.5 bar, 4.75 bar, or 5 bar.

Said device for providing, controlling and/or measuring an absolute pressure inside the reaction vessel is also suitable for providing and maintaining absolute pressures in the range of 1.0 bar to 1200 bar, 1.2 bar to 1000 bar, 1.3 bar to 500 bar, preferably in the range of 1.5 bar to 1000 bar. In particular, the range of 1.5 bar to 5 bar has been calculated by the inventors to be optimal for the production of methane and would be well tolerable by the methanogenic microorganisms of the present invention.

Of course, this implies that the bioreactor and in particular the reaction vessel which is used in the method in the invention and also in the system is suitable for such high pressures. Examples of suitable reaction vessels, bioreactors and pressure resistant materials are well known to a person skilled in the art and can for Example be obtained from Sartorius (Göttingen, Germany), such as Biostat® Cplus, Büchi (Essen, Germany) or other suppliers of bioreactors.

The method of the invention may optionally comprise at least one methane production phase and further optionally at least one cell growth phase. In the at least one methane production phase of the invention, the partial pressure ratio of hydrogen to carbon dioxide ($H_2/CO_2$ ratio) in the reaction vessel is maintained at 5:1 or higher (parts hydrogen :

parts carbon dioxide), preferably, maintained in the range from 7:1 to 30:1 (parts hydrogen : parts carbon dioxide), more preferably in the range from 9:1 to 25:1 (parts hydrogen : parts carbon dioxide), even more preferably in the range from 10:1 to 18:1 (parts hydrogen : parts carbon dioxide), most preferably, the ratio of the partial pressure of hydrogen to the partial pressure of carbon dioxide inside the reaction vessel is maintained in the range from 12:1 to 17:1 or adjusted to about 15 ±1:1 or 18±1:1 (parts hydrogen : parts carbon dioxide) in the at least one methane production phase, whereas in the optional at least one cell growth phase, the partial pressure ratio of hydrogen to carbon dioxide is different from the ratio in the at least one methane production phase, preferably, the partial pressure ratio of hydrogen to carbon dioxide is maintained at or adjusted to a ratio lower than in the ratio in the at least one methane production phase, particularly preferably, the partial pressure ratio is maintained at or adjusted to 4:1 or lower (parts hydrogen : parts carbon dioxide), more preferably, the partial pressure ratio is maintained at or adjusted to a ratio in the range from 0.5:1 to 4:1 or from 0.75:1 to 3.5:1 (parts hydrogen : parts carbon dioxide), even more preferably, the partial pressure ratio is maintained at or adjusted to a ratio in the range from 1:1 to 2.5:1 (parts hydrogen : parts carbon dioxide), most preferably, the partial pressure ratio is maintained at or adjusted to a ratio of 1.5±0.5:1 or 2±1:1 (parts hydrogen : parts carbon dioxide) in the at least one cell growth phase.

The partial pressure of a gas reflects the specific, i.e. partial, pressure of said gas in a gas mixture, here in the gas phase inside the reaction vessel. In the reaction vessel, essentially a liquid phase and a gaseous phase are present. The gaseous phase comprises the gases which are fed into the reactor or are produced by the methanogenic microorganisms. The liquid phase inside the reaction vessel comprises the methanogenic microorganisms of the invention which are typically suspended in a standard medium. "Head pressure" denotes the total pressure inside the reaction vessel. The measurement and adjusting or maintaining of the ratio of the partial pressures of at least carbon dioxide and hydrogen allows for a specific transport of the gases to the methanogenic microorganisms inside the reaction vessel. Different sources of carbon dioxide and hydrogen with different content of said gases may be used, mixed and exchanged according to the online measurement. Thereby, it is not even necessary to determine the exact composition of the gas source which is used, e.g. in case of biogas. The pressure ratio of both gases inside the reaction vessel may be adjusted to or maintained at a specific value or in a range according to the desired carbon flux, i.e. towards cell growth in the cell growth phase or towards methane production in the methane production phase.

The partial pressures of both gases, hydrogen and carbon dioxide, which are the methanogenesis educts for the conversion to methane by the methanogenic microorganisms of the method of the invention, provide a reliable information on how much of the two educt substances is actually provided to the cells. At a known temperature, the partial pressures of the gases also allow the calculation of the dissolved gas concentrations which are actually available to the cells since partial pressures are linked via Henry's law to the solubility of the gaseous components in the liquid phase. The partial pressures of the gases are determined by a combination of sensors which measure the head pressure and the off-gas concentration, and thus may be adjusted or maintained in real time to a specific ratio or in a specific ratio range. For the measurement of the off-gas concentrations standard gas analyzer systems (such as provided by BlueSens gas sensor GmbH, Herten, Germany) can be used. For measurement of the head pressure standard pressure sensors such as provided by Keller (Winterthur, Switzerland) are used either directly inside the reaction vessel or for off-gas measurement inside the device for removing the off-gas from the reaction vessel. The partial pressures are derived in real time in a process management system (Lucullus; Biospectra AG, Schlieren, Switzerland) according to the equation $p_i = c_i/p_{total}/100$, wherein i specifies the respective component, $c_i$ is the gas concentration of component i in volume % and $p_{total}$ is the total head pressure in bar absolute.

Surprisingly, the inventors found out that the methanogenic microorganisms seem to prefer a different partial pressure of carbon dioxide and hydrogen for optimal cell growth, i.e. biomass concentration increase, than for optimal methane production. A determined ratio of the partial pressure of hydrogen to the partial pressure of carbon dioxide is beneficial for the ability of the biomass i.e. of the methanogenic microorganisms to produce methane. On the contrary, the methanogenic microorganisms prefer a different ratio of the partial pressure of hydrogen to the partial pressure of carbon dioxide than for methane production for cell growth, i.e. for biomass concentration increase. Thus, it was surprisingly found out by the inventors that the partial pressure ratio of hydrogen to carbon dioxide in the reaction vessel has a high impact on the performance of the cells, i.e. either mainly cell growth is promoted or mainly methane is produced.

Thus, optionally the method of the invention comprises at least one methane production phase and optionally at least one cell growth phase. Principally, the two phases are characterized by a different ratio of the partial pressure of hydrogen to the partial pressure of carbon dioxide in both phases. It is even more surprising that a ratio of the partial pressure of hydrogen to the partial pressure of carbon dioxide inside the reaction vessel which is maintained at or adjusted to 5:1 or higher (parts hydrogen : parts carbon dioxide) promotes methane production of the methanogenic microorganisms, i.e. the carbon flux from carbon dioxide is directed to methane. Preferably, the ratio of the partial pressure of hydrogen to the partial pressure of carbon dioxide in the reaction vessel in the at least one methane production phase is maintained in the range from 7:1 to 30:1 (parts hydrogen : parts carbon dioxide), more preferably, in the range from 9:1 to 25:1 (parts hydrogen : parts carbon dioxide), even more preferably, in the range from 10:1 to 18:1 (parts hydrogen : parts carbon dioxide), most preferably, the ratio of the partial pressure of hydrogen to the partial pressure of carbon dioxide inside the reaction vessel is maintained in the range from 12:1 to 17:1 or adjusted to 15±1:1 or 18±1:1 (parts hydrogen : parts carbon dioxide). "Or higher" with respect to a partial pressure ratio denotes that the ratio is increasing so that more parts hydrogen in comparison to parts carbon dioxide are present in the gas phase inside the reaction vessel, in particular more than 5 parts hydrogen are facing 1 part carbon dioxide. Examples of such "higher ratios" are also given above and elsewhere in the description.

On the contrary, a different ratio of the partial pressure of hydrogen to the partial pressure of carbon dioxide inside the reaction vessel has surprisingly been identified as further promoting cell growth, i.e. biomass concentration increase, hence, wherein the carbon flux from carbon dioxide is directed to biomass production, of the methanogenic microorganisms, thus, in the at least one cell growth phase, preferably the partial pressure ratio of hydrogen to carbon dioxide is maintained at or adjusted to a ratio lower than the ratio in the at least one methane production phase, particularly preferably, the partial pressure ratio is maintained at 4:1 or lower (parts hydrogen : parts carbon dioxide) in the at least one cell growth phase, more preferably, the partial pressure ratio is maintained in the range from 0.5:1 to 4:1 or from 0.75:1 to 3.5:1 (parts hydrogen : parts carbon dioxide), even more preferably, the partial pressure ratio is maintained in the range from 1:1 to 2.5:1 (parts hydrogen : parts carbon dioxide), most preferably, the partial pressure ratio is maintained at or adjusted to a ratio of 1.5±0.5:1 or 2±1:1 (parts hydrogen : parts carbon dioxide) in the at least one cell growth phase. "Or lower" with respect to a partial pressure ratio denotes that the ratio is decreasing so that less parts hydrogen in comparison to parts carbon dioxide are present in the gas phase inside the reaction vessel, in particular less than 4 parts hydrogen are facing 1 part carbon dioxide. Examples of such "lower ratios" are given also given above and elsewhere in the description.

In an optional embodiment of the method of the invention, the volumetric ratio of hydrogen to carbon dioxide in the gas feed introduced into the reaction vessel is 4:1. In this embodiment, no control of the partial pressure ratio of hydrogen to carbon dioxide is performed and thus no specific adjustment is possible, however, if the gas feed rate is applied as described above, and optionally also the pressure inside the reaction vessel is increased above normal atmospheric pressure as also described above, the simple volumetric ratio control is sufficient to obtain a good volumetric productivity and a good percentage amount of methane in the off-gas. However, the control of the partial pressure of hydrogen to methane is preferred. In case of a hydrogen and carbon dioxide gas feed based on the volumetric ratio of both educt gases, it is beneficial to determine the purity of the educt gases in order to have a volumetric ratio of hydrogen to carbon dioxide which is not falsified by contaminant gases.

"Phase" or "fermentation phase" in the sense of the invention describes a situation, i.e. a condition, state or fermentation condition of the methanogenic microorganisms in the reaction vessel of the invention which is characterized by specific fermentation conditions which are applied to the methanogenic microorganism, e.g. the ratio of the partial pressures of hydrogen and carbon dioxide or the oxidation reduction potential is maintained at or in a range or adjusted to as mentioned above. Any "cell growth phase" and in particular the "at least one cell growth phase" is a phase of the invention, wherein the ratio of the partial pressure of hydrogen to the partial pressure of carbon dioxide inside the reaction vessel is maintained at or adjusted to a ratio different to the ratio in a methane production phase and thereby mainly characterized by an increase of the biomass concentration by cell division of the methanogenic microorganisms of the invention, i.e. by cell growth of the methanogenic microorganisms of the invention. Any "methane production phase" and in particular the "at least one methane production phase" is a phase of the invention, wherein the ratio of the partial pressure of hydrogen to the partial pressure of carbon dioxide inside the reaction vessel is maintained at or adjusted to 5:1 or higher (parts hydrogen : parts carbon dioxide) and thereby mainly characterized by methane production. However, during any cell growth phase, the cells may also or may not produce methane and during any methane production phase, the biomass concentration may increase, remain the same or decline, preferably, the biomass concentration increases in the methane production phase and in particular in the at least one methane production phase.

The method of the invention may further optionally comprise an intermediate phase, wherein the methanogenic microorganisms in the reaction vessel are switched from a methane production phase, in particular from the at least one methane production phase, to a cell growth phase, in particular to the at least one cell growth phase, or from a cell growth phase, in particular from the at least one cell growth phase, to a methane production phase, in particular to the at least one methane production phase.

While the methanogenic microorganisms are in an intermediate phase, they may produce methane or not or the cells may divide, grow or not.

The methanogenic microorganisms of the invention may be switched from one phase to another phase, e.g. from a methane production phase, in particular from the at least one methane production phase, to a cell growth phase, in particular to the at least one cell growth phase, or from a cell growth phase, in particular from the at least one cell growth phase, to a methane production phase, in particular to the at least one methane production phase, by maintaining or adjusting the ratio of the partial pressure of hydrogen to the partial pressure of carbon dioxide at or to values as disclosed above. Optionally, also the oxidation reduction potential is maintained or adjusted using a reducing agent. Any substance which reduces the oxidation reduction potential inside the reaction vessel without being harmful to the cells or having an inhibitory effect on cell growth or methane production may be used as reducing agent. Examples for such reducing agents are sodium sulphide or hydrogen sulphide. Alternatively, the oxidation reduction potential may be maintained or adjusted by the oxygen gas inflow, hydrogen inflow and/or pH shift. The oxidation reduction potential is measured in real time, for example by a redox probe from Mettler-Toledo GmbH (Greifensee, Switzerland) or any other suitable means, and therefore a specific adjustment or maintaining is possible. The reducing agent may be pulsed into the reaction vessel or fed in a continuous mode.

"Oxidation reduction potential (ORP)" or simply "reduction potential" is a measure of the tendency of a chemical species to acquire electrons and thereby be reduced. ORP is measured in volts (V), millivolts (mV), or $E_h$ (1 $E_h$ = 1 mV). Each species has its own intrinsic oxidation reduction potential; the more positive the potential, the greater the species' affinity for electrons and tendency to be reduced and *vice versa.* An aqueous solution, such as the medium of the cell culture of the invention, with a lower (more negative) oxidation reduction potential has for example a tendency to donate electrons to a newly introduced species (i.e. to be oxidized by reducing a newly introduced species). Thus, the transfer of hydrogen ions between chemical species determines the pH of an aqueous solution, the transfer of electrons between chemical species determines the oxidation reduction potential of an aqueous solution, here the oxidation reduction potential inside the reaction vessel. The oxidation reduction potential inside the reaction vessel in the at least one methane production phase may be maintained below -350 mV, preferably, in the range from -400 mV to -700 mV, more preferably, the oxidation reduction potential inside the reaction vessel is maintained in the range from -500 mV to -700 mV, most preferably, maintained at or adjusted to a value of -550±50 mV. In the at least one cell growth phase, the oxidation reduction potential inside the reaction vessel is maintained below -300 mV, preferably, in the range from -300 mV to -500 mV, even more preferably, the oxidation reduction potential inside the reaction vessel is maintained at or adjusted to a value of -350±25 mV. Thus, for promoting the switching of the methanogenic microorganisms from the at least one methane production phase to the at least one cell growth phase, the oxidation reduction potential inside the reaction vessel should optionally be maintained below -300 mV, preferably, in the range from -300 mV to -500 mV, even more preferably, the oxidation reduction potential inside the reaction vessel is maintained at or adjusted to a value of -350±25 mV. For promoting the switching of the methanogenic microorganisms from the at least one cell growth phase to the at least one methane production phase, the oxidation reduction potential inside the reaction vessel should optionally be maintained below -350 mV, preferably, in the range from -400 mV to -700 mV, more preferably, the oxidation reduction potential inside the reaction vessel is maintained in the range from -500 mV to -700 mV, most preferably, maintained at or adjusted to a value of -550±50 mV.

Surprisingly, a specific oxidation reduction potential is required to start cell growth after inoculation of a medium in the reaction vessel in step c) of the method with the methanogenic microorganisms of the invention. Here, the oxidation reduction potential should be adjusted to a value of -350±25 mV. This value may be achieved by different means, for example by adding 500 mM $Na_2SxH_2O$ to the inoculated medium. Inoculation procedures will be explained in detail below.

The optional methane production phase and the optional cell growth phase, in particular the at least one methane production phase and the at least one cell growth phase, are characterized by the different ratios of the partial pressures of hydrogen and carbon dioxide as described in detail above. The oxidation reduction potential in both phases is beneficially maintained or maintained at or adjusted to the values or in the ranges as also described in detail above; however, the ratio of the partial pressures is the main characteristic and determines the phase of the methanogenic microorganisms.

The phases of the methanogenic microorganisms, the growth phase, the intermediate phase and the methane production phase may be separated i.e. do not have to follow timely immediately after each other as the methanogenic microorganisms may be stored at lower temperatures depending on the specific cell strain used. Also, the phases do not have to occur in the same reaction vessel but the cells may be grown in one reaction vessel and be transferred to another reaction vessel for methane production. For this purpose, either two or more bioreactors may be used or the bioreactor may comprise at least two reaction vessels, at least one for cell growth and at least one for methane production. All fermentation conditions, in particular the ratio of the partial pressures of hydrogen and carbon dioxide or the oxidation reduction potential inside the reaction vessel comply according to the phase of the methanogenic microorganisms.

In a preferred set-up of the method of the invention, first, a suitable medium, e.g. standard medium, is placed in the reaction vessel and is inoculated with a suitable amount of the methanogenic microorganisms. In case the methanogenic microorganism is a methanogenic archaea, the suitable amount of cells is provided in a cell suspension which is anaerobically transferred to the reaction vessel. Anaerobic procedures and techniques can be taken from the literature, such as Sowers, Schreier *et al.,* 1995. A suitable amount of the methanogenic microorganisms of the invention is for example 0.5 to 10% of a living cell suspension of the methanogenic microorganism respective to the target volume. However, in principle a single cell is enough to inoculate the medium and thus, the amount may vary in a broad range. The skilled person knows that microorganisms and also methanogenic microorganisms grow exponentially, thus, the more cells are used for inoculation, the earlier the cells reach a certain biomass concentration. Further, inoculation procedures are known to the skilled person and may be individually selected without having a negative effect on the success of the invention (Schoenheit, Moll *et al.,* 1980).

Optionally, the at least one cell growth phase may follow. During the at least one cell growth phase, the ratio of the partial pressures of hydrogen and carbon dioxide is maintained at or adjusted to a ratio different compared to the ratio in the at least one methane production phase, preferably, the ratio of the partial pressure of hydrogen to the partial pressure of carbon dioxide in the at least one cell growth phase is maintained at or adjusted to a ratio of the partial pressure of hydrogen to the partial pressure of carbon dioxide which is lower than the ratio of the partial pressure of hydrogen to the partial pressure of carbon dioxide in the at least one methane production phase, particularly preferably, the partial pressure ratio in the at least one cell growth phase is maintained at or adjusted to 4:1 or lower (parts hydrogen : parts carbon dioxide), more preferably, the partial pressure ratio is maintained in the range from 0.5:1 to 4:1 or from 0.75:1 to 3.5:1 (parts hydrogen : parts carbon dioxide), even more preferably, in the range from 1:1 to 2.5:1 (parts hydrogen : parts carbon dioxide), most preferably, the partial pressure ratio is maintained at or adjusted to a ratio of $1.5\pm0.5:1$ or $2\pm1:1$ (parts hydrogen : parts carbon dioxide) in the at least one cell growth phase.

In general, the length of the optional at least one cell growth phase depends on the cell growth rate of the methanogenic microorganisms in the cell culture; however, the time period of any cell growth phase and in particular of the at least one cell growth phase may be chosen by the experimenter performing the method of the invention. In principle, the least one cell growth phase is not necessary for the success of methane production of the method of the invention as the cells may also grow and divide under the fermentation conditions of the at least one methane production phase, in particular characterized by a specific $H_2/CO_2$ partial pressure ratio. Surprisingly, it has been found out by the inventors that at least one cell growth phase is additionally beneficial for a high biomass concentration in the methane production phase which results in a higher methane yield of step c) and the overall method. This is possibly due to the surprising discovery that the partial pressure ratio which is applied in the cell growth phase promotes the carbon flux from carbon dioxide to biomass prior to enter the methane production phase, wherein the carbon flux is mainly directed from carbon dioxide to methane. Another advantage of an additional cell growth phase is that a catalytically acceptable biomass concentration of 3 g/L is reached after a shorter period of fermentation time and hence, the productivity of the overall methane production process is optimized, preferably the biomass concentration for methane production is at least 5 g/L, or at least 8 g/L, more preferably at least 10 g/L, even more preferably at least 12 g/L and most preferably at least 15 g/L. Hence, the carbon flux may be directed according to the needs of the experimenter and according to the procedural requirements.

Surprisingly, it has further been found out that it is additionally beneficial for the volumetric productivity of the overall methane production of the method of the invention to maintain the methanogenic microorganisms in the at least one cell growth phase until the cell growth of the methanogenic microorganisms in the reaction vessel stagnates (optionally, at around 3 g/L or higher as described above), i.e. the biomass concentration does not increase any further, or the biomass concentration reaches a value of at least 3 g biomass per litre or higher. The biomass concentration may be determined as described elsewhere, for example gravimetrically (Schill, van Gulik *et al.,* 1996). A person skilled in the art, of course, knows other methods to determine the biomass concentration which might be used as well for this purpose.

**In** principle, at any time during fermentation, the methanogenic microorganisms may be switched from a cell growth phase to a methane production phase or an intermediate phase or from a methane production phase to a cell growth phase or an intermediate phase by adjusting the fermentation conditions if regarded beneficial by the experimenter for example to even more increase the biomass concentration. Thus, also during stable methane production it may be desirable to temporarily switch the methanogenic microorganisms to cell growth phase, for example for cell growth after medium exchange or oxygen contamination. Hence, the method of the present invention may also comprise two or more methane production phases, optionally one, two or more cell growth phases and optionally one, two or more intermediate phases.

After an optional cell growth phase, the fermentation conditions of the methane production phase may optionally be applied, in particular the ratio of the partial pressures of hydrogen to carbon dioxide inside the reaction vessel is maintained at or adjusted to the ratio or in the range of the methane production phase. Thereby, the methanogenic microorganisms are switched to the methane production phase, optionally the methanogenic microorganisms pass an intermediate phase. The microorganisms of the invention are maintained in this phase over a period of time which can range from hours to weeks to months and years depending on fresh medium supply, the cell species and the specific needs of the experimenter. The biomass concentration in the at least one methane production phase may increase to a value of at least 6 g biomass per litre, preferably at least 7 g biomass per litre, more preferably at least 8 g biomass per litre, even more preferably at least 9 g biomass per litre and most preferably of at least 10 g, 12 g or 15 g biomass per litre, wherein optionally no additional methanogenic microorganism is added after inoculation or cell retention is applied during fermentation, but the biomass concentration is reached by cell division of the originally inoculated cells which are in general 0.5 to 10% of the inoculation volume from an earlier harvest of 3 g/L stored in a pressurized bottle at +4 °C. However, a person skilled in the art knows that the number of cells which are used for inoculation has only little influence on the biomass yield that may be achieved during fermentation. It was absolutely unexpected that the experimental set-up provided by the method of the invention leads to such high biomass concentrations of the methanogenic microorganisms, in particular of the methanogenic archaea. Thus, the use of the methanogenic microorganisms of the invention inside a reaction vessel having a biomass concentration between 6 g to 15 g biomass per litre under continuous fermentation conditions such as described herein is also provided herein.

The skilled person is well aware of the fact that in any phase of the invention, the fermentation conditions have to be adjusted to the specific needs of the employed microorganism species or cell strain; however, the fermentation conditions as described herein apply to all species and strains of the invention for cell growth and methane production.

"Biomass" or "cell culture" or "cells" of the invention equally denote to the same collective of methanogenic microorganisms of one or more species which is used in the method of the invention for converting hydrogen and carbon dioxide into methane. The methanogenic microorganisms, i.e. cells of the biomass or cell culture in the reaction vessel of the invention, principally function as a catalyst for the biological conversion of carbon dioxide and hydrogen into methane. They are placed inside the reaction vessel of the invention by inoculation of the medium inside the reaction vessel or by inoculation of the medium prior to the transfer of the medium into the reaction vessel of the invention. Thus, during fermentation the term "methanogenic microorganisms" also denotes to methanogenic microorganisms in a suspension with medium as inside a reaction vessel, the cell culture of methanogenic microorganisms typically comprises medium. The methanogenic microorganisms may be removed with or without medium from the reaction vessel either completely or partially during fermentation and in any phase for various purposes, such as exchange of cells or medium and/or for analytics, such as cell weight determination or cell vitality examination, or harvest for future inoculum. Methane is obtained from the gas leaving the liquid phase, i.e. the cell-medium suspension.

Biomass obtained from the reaction vessel may be used as source for the recovery of metabolites and as basis for new culture medium. "Healthy biomass" denotes a good vitality status of a living biomass, i.e. of the living methanogenic microorganisms. A "microorganism" is an organism that is unicellular or lives in a colony of cellular organisms. Microorganisms are very diverse; they include bacteria, fungi, archaea, microscopic plants (such as green algae), and animals such as plankton and the planarian. Some microbiologists also include viruses, but others consider these as non-living. "Methanogenic" in the sense of the invention denotes the capability of converting carbon dioxide and hydrogen into methane, i.e. being able to perform the reaction pathway of $CO_2+4H_2 \rightarrow CH_4+2H_2O$ which is called methanogenesis.

A "methanogenic microorganism" is any microorganism which is capable of producing methane from other substances, i.e. capable of methanogenesis, preferably from carbon dioxide and hydrogen. Hence, the methanogenic microorganisms of the invention are capable of producing methane from hydrogen and carbon dioxide. The methanogenic microorganism of the invention may be grown in a reaction vessel, which is for example comprised in a bioreactor, under controlled fermentation or cell culture conditions in a controlled environment. In principle, any such methanogenic microorganism can be used for the method of the invention. "Environment" or "controlled environment" refers to the surrounding of the methanogenic microorganisms inside the fermenter or reaction vessel comprising both, the liquid, characterized for example by the medium composition, feed and exchange rate, and the gaseous phase, characterized for example by the gas feed and gas feed composition, and the applied fermentation conditions.

The methanogenic microorganism in the cell culture of the invention is obtainable from public collections of organisms, such as the American Type Culture Collection, the Deutsche Stammsammlung für Mikroorganismen und Zellkulturen (DSMZ, Braunschweig, Germany), CBS (Utrecht, Netherlands) and the Oregon Collection of Methanogens, or they can be isolated from a variety of environmental sources. Such environmental sources include anaerobic soils and sands, bogs, swamps, marshes, estuaries, dense algal mats, both terrestrial and marine mud and sediments, deep ocean and deep well sites, sewage and organic waste sites and treatment facilities, animal intestinal tracts, volcano areas and faeces. Suitable cell cultures may be pure i.e. contain only cells of a single species, or may be mixed cultures i.e. contain cells of more than one species. Preferably, a pure cell culture of methanogenic microorganisms is used for the method of the invention.

A methanogenic microorganism by nature being capable of producing methane is especially suitable for any embodiment of the method of the invention. Preferably, the methanogenic microorganism of the invention is a methanogenic archaea, comprising all methanogenic members of the Archaeal domain, such as for example *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arbor iphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barken, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus* and *Methanopyrus kandleri.* Preferred are methanogenic archaea which belong to the species *Methanosarcinia barkeri, Methanothermobacter marburgensis,* such as for example DSM 2133 from Deutsche Stammsammlung für Mikroorganismen und Zellkulturen (DSMZ, Braunschweig, Germany), *Methanobacterium thermoautotrophicus, Methanocaldococcus jannaschii, Methanothermobacter thermoautotrophicus, Methanococcus maripaludis,* or mixtures thereof, more preferably, the methanogenic archaea and thus the preferred methanogenic microorganism is of the species *Methanothermobacter marburgensis,* most preferably, the methanogenic microorganism or methanogenic archaea is *Methanothermobacter marburgensis* strain DSM 2133. "Archaea" are single-celled microorganisms. Archaea have no cell nucleus or any other membrane-bound organelles within their cells. In the past they were viewed as an unusual group of bacteria and named archaeabacteria, however, to date archaea are considered as having an independent evolutionary history since they show many differences in their biochemistry in comparison to other forms of life. Thus, they are now classified as a separate domain (Archaeal domain) in the three-domain system. In this system the phylogenetically distinct branches of evolutionary descent are the archaea, bacteria and eukarya.

In principle, the methanogenic microorganism of the invention, i.e. the biomass, is used in cell culture, i.e. the methanogenic microorganism is used as suspension with medium. The medium of the invention comprises at least a source of nitrogen, assimilable salts, a buffering agent and trace elements. Sulphur is provided to the cells by the reducing agent or may be provided extra, thus, the reducing agent and the sulphur-providing substance may or may not be the same. Sulphur may be provided to the cells by the provision of biogas. The medium is generally designed to keep the cells vital, healthy and productive without being expensive and thus beneficial for the entire process economy of the method of the invention. Prior to inoculation of the medium with the methanogenic microorganism of the invention, the medium should be degassed for being anaerobic as is required for optimal methane production by the methanogenic microorganism which is used in the particular experiment, this applies in particular if the methanogenic microorganism is a methanogenic archaea. The skilled person knows techniques to prepare an oxygen free medium, for example by flushing the medium with a gas mixture of 80% hydrogen and 20% carbon dioxide (v/v = volume per volume) or with nitrogen for 5 min per litre medium. Standard medium compositions may be taken from the literature and be adapted (Schoenheit, Moll *et al.,* 1980; Schill, van Gulik *et al.,* 1996; Liu, Schill *et al.,* 1999). An example for a standard medium has the following composition ($L^{-1}$): 2,1 g $NH_4Cl$; 6,8 g $KH_2PO_4$; 3,4 g $Na_2CO_3$; 0,09 g Titriplex I; 0,04 g $MgCl_2$x $6H_2O$; 0,01 g $FeCl_2$x$4H_2O$; 0,2 mg $CoCl_2$x$6H_2O$; 1,2 mg $NiCl_2$x$6H_2O$; 0,2 mg $NaMoO_4$x$2H_2O$, the pH will be discussed below and can be adjusted by titrating 1 M $(NH_4)_2CO_3$, NaOH or $NH_4OH$. Said exemplary standard medium may be used for any embodiment of the method of the invention. In some of the Experiments described in the Experimental section below higher concentrated medium, in particular with respect to trace elements has been used. This is a direct consequence of the higher biomass concentration which may be achieved using the high total gas feed rates of hydrogen and carbon dioxide in the present invention. It may be required that also the buffer components may be higher concentrated, however, the skilled person knows very well which measures are to be taken in order to keep the pH value in the desired range or at the desired value.

The medium may be refreshed in a constant or step-wise mode during fermentation under continuous conditions. The medium feed rate or medium in-feed rate of medium or medium components is generally adjusted between 0.001 $h^{-1}$ and 0.1 $h^{-1}$.

The medium out-feed rate, i.e. the rate with which medium or a liquid leaves the reaction vessel, generally corresponds to the in-feed rate plus water which is produced by the methanogenic microorganisms during methanogenesis. Said water can be re-used for various purposes, such as medium preparation or else.

The skilled person is aware that the medium has to be adjusted to the specific needs of the microorganism species, i.e. cell strain. In general, the fermentation conditions, i.e. medium composition, and other parameters, such as $H_2/CO_2$ partial pressure ratio, pH, temperature, stirring speed, pressure, oxidation reduction potential or medium or medium component (i.e. consumables) feed rate, i.e. fresh medium supply rate, have to be adjusted according to the specific needs of the microorganism strain selected and the procedural requirements depending for example on the phase of the methanogenic microorganisms in the reaction vessel. Herein, information is provided which particular fermentation condition is applicable to all methanogenic microorganisms, such as the standard medium, stirring speed, gas feed rate, oxidation reduction potential, pressure, or the partial pressure ratios of hydrogen and carbon dioxide, and which should be adapted to the specific needs of a cell species, such as the temperature and pH value.

"Consumable" comprises all substances, components and/or materials in any form, i.e. solid, gaseous or liquid, which might be required by the methanogenic microorganisms in the reaction vessel of the invention. Also included are substances which are fed into the reactor to maintain a certain fermentation condition, such as a certain medium composition, a specific oxidation reduction potential or partial pressure ratio. The supply of consumables can be adjusted by the experimenter throughout the method of the invention if deemed appropriate by the experimenter. "Procedural requirements" comprise all situations which may arise during the method of the invention, such as the requirement to switch to another phase or in response to oxygen contamination, feeding real gas, or else. The method of the invention is designed for the conversion of hydrogen and carbon dioxide into methane by methanogenic microorganisms in a reaction vessel; however, it might be necessary for a high volumetric productivity to switch from the optional methane production phase to an intermediate or cell growth phase, wherein excess liquid, parts of the gas phase or the biomass are partially or completely exchanged. Such phases may be used for example to increase the cell density of the cell culture inside the reactor or to introduce fresh medium or medium components. At no time, the overall feasibility of the invention is impeded.

"Off-gas", "exhaust gas", "outgas" or "output gas" denote the gaseous outcome of the reaction vessel of the invention and thus of the method which is typically a gas mixture leaving the reaction vessel. The qualitative and quantitative content of the off-gas depends on various factors, such as the phase of the methanogenic microorganisms in the reaction vessel, the total gas feed rate into the reaction vessel, and the composition of the gas feed into the reaction vessel. The off-gas may comprise water vapour, the gases which are introduced into the reaction vessel, such as for example hydrogen, carbon dioxide, hydrogen sulphide or else, and gases which are produced by the methanogenic microorganisms such as methane. The off-gas mixture may further contain contaminants which may be present in the gases which are introduced into the reaction vessel or originate from the cell culture, for example oxygen, compounds from biogas generation, nitrogen gas and others. For Example, e.g. during a methane production phase, the off-gas mixture mainly comprises methane, preferably the methane content is at least 40%, 45%, 50%, or 60%, more preferably the methane content is at least 70%, more preferably 80%, 84%, 86%, or 88%, even more preferably the methane content in the off-gas is at least 90%, most preferably the methane content is at least 92%, 94% or 96% wherein the higher the content the more preferred. During cell growth phase, the methane content in the off-gas is typically between 50% and 80%. The methane from the off-gas may be separated by standard means, e.g. by membranes such as obtainable from Du Pont (Wilmington, DE, USA) or Gore (Newark, DE, USA). It may further be used in end-user applications as energy carrier for energy applications to produce heat and/or electric energy or else or as raw material in various chemical and biological conversions.

"In-gas" denotes the total gas or gas mixture which is fed into the reaction vessel and thus provided to the methanogenic microorganism. The term is used to describe the total gas introduced into the reaction vessel or bioreactor, however, that does in no way mean that all potentially introduced gases are introduced in a mixture or single gas flow. The gases which may be comprised in the in-gas may thus be introduced as a mixture, separately via different devices or tubes or timely one after another via the same device r tube or via different devices or tubes. The in-gas or in-gas feed may comprise the gases which are required for the production of methane, i.e. hydrogen and carbon dioxide, and other gases which may be required for other purposes such as for example to adjust the oxidation reduction potential in the reaction vessel by addition of hydrogen sulphide or are required for other biological processes of the microorganisms or gases which are introduced as contaminants. This is especially the case if real gases are used as gas sources for the method of the invention. "Feed" in general means the introduction, flow or transfer of a gas, liquid, suspension or any other substance into the reaction vessel or into the bioreactor of the invention and also the removal or withdrawal of a gas, liquid, suspension or any other substance from the reaction vessel or from the bioreactor to the outside. "In-gas feed" denotes the feed or flow or introduction of the in-gas, such as hydrogen or carbon dioxide, into the reaction vessel of the invention. "Hydrogen in-gas feed" or "hydrogen gas feed" denotes the feed or flow of hydrogen comprising in-gas or of hydrogen gas into the reaction vessel or bioreactor. "Carbon dioxide in-gas feed" or "carbon dioxide gas feed" denotes the feed or flow of carbon dioxide comprising in-gas or carbon dioxide gas into the reaction vessel or bioreactor.

"Feed rate" in general denotes the amount of a gas, liquid, suspension or any other substance into the reaction vessel or into the bioreactor of the invention or the removal or withdrawal of a gas, liquid, suspension or any other substance from the reaction vessel or from the bioreactor to the outside within a certain period of time. "In-gas feed rate" or "gas feed rate" denotes the volume of an in-gas or gas which is introduced into the reaction vessel, e.g. hydrogen or carbon dioxide, per volume of reaction medium within a certain period of time. Thus, the in-gas feed rate or gas feed rate reflects the amount of gas provided to the methanogenic microorganisms within a certain period of time. In the course of the method of the invention, the in-gas feed rate or feed rate is given as volume gas per volume of reaction vessel medium per time period (e.g. vvm, volume per volume per minute), i.e. per minute, hour or day, preferably per minute. The skilled person will appreciate that the volume of gas introduced into the reaction vessel is given in relation to the volume of reaction medium. The "reactor medium", "reaction medium" or "reaction vessel medium" or similar is the liquid medium inside the reaction vessel of the bioreactor, i.e. the cell suspension, which comprises the methanogenic microorganisms and the medium. This is particularly useful as the amount of gas introduced into the reaction vessel is thus put in relation to the volume of liquid wherein the two educt gases hydrogen and carbon dioxide must dissolve in order to get in contact with the methanogenic microorganism. The volume of the reaction medium is determined gravimetrically, by differential pressure, radar or any other method known to the skilled person. Exemplary suppliers are e.g. MettlerToledo (Greifensee/Switzerland) for balances and e.g. Lewa (Leonberg, Germany) for pumps.

The gas feed rate of hydrogen and carbon dioxide into the reaction vessel plays a major role in the method of the invention. The gas feed rate of both gases is given in total, which denotes the sum gas feed rate of both hydrogen and carbon dioxide together. However, depending on the bioreactor, reaction vessel or system set-up it may be more suitable to give the gas feed rates of both gases, i.e. of hydrogen and carbon dioxide, separately. Hence, in any embodiment of the present invention, hydrogen and carbon dioxide may be fed into the reaction vessel separately or as a mixture. In case of a separate feed into the reaction vessel, nevertheless the total gas feed rates, i.e. the gas feed rate of hydrogen plus the gas feed rate of carbon dioxide should be considered to determine the total gas feed rate.

As an example, in case that in the method of the invention, the gas feed rate of hydrogen and carbon dioxide into the reaction vessel is at least 1.2 vvm or 1.4 vvm in total, this means that the sum of the gas feed rate of hydrogen and of the gas feed rate of carbon dioxide is 1.2 vvm or 1.4 vvm, respectively. Since the volume of reaction medium and the period of time remains the same, the volume of hydrogen gas and the volume of carbon dioxide are to be summed up as total volume of gas (volume of hydrogen plus volume of carbon dioxide) which is introduced into the reaction vessel per volume of reaction medium inside the reaction vessel and comprising the methanogenic microorganisms per period of time, e.g. per minute.

The method for producing methane according to the present invention comprises contacting methanogenic microorganisms in a reaction vessel with hydrogen and carbon dioxide, wherein the gas feed rate of hydrogen and carbon dioxide, i.e. the sum gas feed rate of hydrogen and carbon dioxide, i.e. the gas feed rate of hydrogen plus the gas feed rate of carbon dioxide, into the reaction vessel is at least 1.2 vvm in total, 1.3 vvm in total, 1.4 vvm in total, more preferably is at least 1.5 vvm in total, 1.6 vvm in total, 1.7 vvm in total, 1.8 vvm in total, 1.9 vvm in total, most preferably is at least 2.0 vvm in total or is at least 2.1, 2.3, 2.5, 2.7, 2.8, 3.0, 3.5, 4.0, 4.5, 5.0, 5.5, 6.0, 7.0, 7.5, 8.0, 9.0, 10.0, 12.5, 15.0, 17.5, 20.0, 30.0, 40.0, 50.0, 75.0 or 100.0 vvm in total or higher, or the gas feed rate of hydrogen and carbon dioxide, i.e. the sum gas feed rate of hydrogen and carbon dioxide, i.e. the gas feed rate of hydrogen plus the gas feed rate of carbon dioxide, into the reaction vessel is in the range of 1.2 vvm to 100.0 vvm in total, 1.2 vvm to 25.0 vvm in total, more preferably is in the range of 1.5 vvm to 15.0 vvm in total, even more preferably is in the range of 1.7 vvm to 10.0 vvm in total, most preferably is in the range of 2.0 vvm to 8.0 vvm in total.

As a general rule, the higher the gas feed rate of hydrogen and carbon dioxide in total the higher is the volumetric productivity achieved in the method and the higher is the percentage amount of methane in the off-gas and the higher the pressure inside the reaction vessel the higher is the volumetric productivity achieved in the method and the higher is the percentage amount of methane in the off-gas.

During culturing of the methanogenic microorganisms, e.g. in an early growth phase or due to unforeseeable events, it may be necessary to reduce the gas feed rates of hydrogen and carbon dioxide appropriately for a short period of time. Hence, in certain situations the total gas feed may vary between 0.05 vvm and 200 vvm without significantly influencing the outcome of the method in producing methane.

"Real gas" denotes that a gas is not absolutely pure, i.e. is gas mixture. In case of hydrogen and carbon dioxide, "real gas" denotes that beside carbon dioxide or hydrogen also other gases are comprised which are denoted as contaminants. On the contrary, an "ideal gas" is absolutely pure according to general industrial standards. A typical example for a real gas is "biogas" or also the off-gas of the method of the invention. "Biogas" typically refers to a gas produced by the biological breakdown of organic matter, e.g. biomass, in the absence of oxygen, such as for example biomass fermentation. Biogas originates from biogenic material and is a type of biofuel like bioethanol. Biogas is produced by anaerobic digestion or fermentation of biodegradable materials, i.e. of biomass, such as manure, sewage, municipal waste, green waste, plant material and energy crops. Thus, "biomass fermentation" denotes the anaerobic digestion or fermentation of biodegradable materials, i.e. of biomass. This type of biogas comprises primarily methane and carbon dioxide and is preferred as carbon dioxide real gas source of the invention. Other gas species generated by use of biomass is wood gas, which is created by gasification of wood or other biomass. This type of gas consists mainly of nitrogen, hydrogen, and carbon monoxide, with trace amounts of methane.

In general, an "industrial process" is any process which involves chemical or mechanical steps to aid in the manufacture of an item or items, usually carried out on a larger scale by man and not by nature. For the present invention, relevant industrial processes produce carbon dioxide and/or hydrogen or other gases and substances which might be required for the method of the invention either as main product or as side product. If said substances are a side product, they are sometimes referred to as waste products (e.g. "industrial waste gas"). Industrial processes which produce or release hydrogen or carbon dioxide are also denoted as "industrial sources" for both gases. Depending on the purity of the released gas, the gas is referred to as being an "ideal gas" having a very high or absolute purity/quality or being a "real gas" having a lower or not absolute purity, meaning that one or more other gases may be present in various amounts, however, the gas species not being harmful to the cells of the invention or being present in the real gas in an amount not being harmful to the cells.

The carbon dioxide, which is used for the method of the invention and is converted into methane by the methanogenic microorganism, may be pure or of high quality/purity, i.e. "ideal gas", or be a "real gas", characterized in that it comprises beside carbon dioxide also other gases which are denoted as contaminants. In general, the carbon dioxide may be of any source. These carbon dioxide sources include but are not limited to ideal gas sources delivering ideal gas or real gas sources delivering real gas. Ideal gases may be obtained by various purchasers which are known to the skilled person (e.g. Air Liquide, Paris, France). Sources of real gas are natural sources, such as the atmosphere, fixed carbon dioxide in living or dead biomass or industrial processes, also denoted as "industrial sources", comprising the combustion or oxygen combustion or oxygen enriched combustion of carbonaceous material, such as biomass, waste, biofuel, fossil fuels, or else, the burning of vegetable matter, biogas, bioethanol, biomass fermentation or anaerobic digestion to produce liquid fuels and coal, biomass gasification processes, general gasification, combustion from engines, such as cars, any other carbon dioxide releasing process, carbon dioxide contained in the off-gas of the method of the invention or combinations thereof. Preferably, carbon dioxide real gas is used for the method of the invention, i.e. carbon dioxide from a real gas source, more preferably the carbon dioxide real gas is from an industrial process, such as industrial waste gas, biogas or biomass fermentation, off-gas of the method of the invention itself, from other methanogenesis performed by methanogenic archaea, oxygen combustion or oxygen enriched combustion or mixtures thereof. In particular, using the off-gas of the method itself and using carbon dioxide which was produced by oxygen enriched combustion using the oxygen produced by the electrolysis of water improves educt utilization, economy and cost-effectiveness of the overall method. Real gas of carbon dioxide or hydrogen is often cheaper and also makes a contribution to environmental protection due to optimal raw material usage. And carbon dioxide, which would otherwise be released to the atmosphere as waste gases and potentially would contribute to global warming, is recycled and used as new energy source raw material. "Combustion", e.g. "oxygen enriched combustion" or "oxygen combustion", is a process wherein carbonaceous material, e.g. petroleum, coal, living and/or dead biomass reacts with an oxidizing element, such as oxygen, into carbon dioxide or also carbon monoxide, by thermal conversion. The oxygen which is required for combustion may be derived from the electrolysis of water (see below). Devices for combustion, oxygen enriched combustion or oxygen combustion may be obtained from Mitsubishi Heavy Industries, Tokyo, Japan. Biomass for combustion and/or gasification may also be the methanogenic microorganisms of the invention which are dried as it might be advisable to completely empty the bioreactor, remove the methanogenic microorganisms from the reaction vessel and start methanogenesis with a completely new batch of cells.

Carbon dioxide of any source and also mixtures thereof may be purified from contaminants prior to its feed into the reaction vessel of the bioreactor of the invention by various methods which are known to the person skilled in the art, such as using a carbon dioxide scrubber, contacting the gas with an absorbing medium which selectively absorbs carbon dioxide, such as amine, monoethanolamine solutions or quicklime absorption, or which selectively absorbs gases other than carbon dioxide from the real gas, activated charcoal or activated coal, by the regenerative carbon dioxide removal system, polymer membrane gas separators, molecular sieves, others and combinations thereof. All aforementioned methods are also useful for recovery, purification, enriching, storing, recycling and/or further processing of carbon dioxide which is contained in the off-gas, and thus was not converted into methane by the methanogenic microorganisms, of the method of the invention. After carbon dioxide is separated from other components of the off-gas it may be fed back by tubes or other devices and means which are known to the skilled person into the reaction vessel as educt gas to be converted into methane, i.e. will be introduced into the reaction vessel as part of the in-gas, and is thus recycled. In this way, the use of carbon dioxide is optimized.

In general, carbon dioxide of any natural, industrial, technical or artificial process, which releases carbon dioxide, may be used as well as carbon dioxide from gasification. "Gasification" is a process which converts carbonaceous materials, e.g. petroleum, coal, living and/or dead biomass, biofuel or else into carbon dioxide, hydrogen and carbon monoxide, by thermal conversion and a controlled amount of oxygen and/or steam into a resulting gas mixture called synthesis gas or syngas. In equilibrium, the concentrations of carbon monoxide, water vapour, carbon dioxide and hydrogen balance. In principle, a limited amount of oxygen or air is introduced into the reactor (= device) to allow some of the organic material to be "burned" to produce carbon monoxide and energy, which drives a second reaction that converts further organic material to hydrogen and additional carbon dioxide. Further reactions occur when the formed carbon monoxide and residual water from the organic material react to form methane and excess carbon dioxide. Catalysts can be used to improve the reaction rates. The oxygen which is required for gasification may be derived from the electrolysis of water (see below). Preferably, the carbon dioxide comprising real gas is purified according to any of the techniques mentioned above or known in the art, to increase the carbon dioxide content to around 80%.

Gasification reactors or devices can be obtained from Condens Heat Recovery Oy (Hämeenlinna, Finland), Babcock & Wilcox Vølund A/S (Esbjerg, Denmark) or Siemens AG (Munich, Germany).

Carbon dioxide, i.e. carbon dioxide gas, of any source, e.g. ideal or real, may be used in the method. In general, carbon dioxide of different ideal and real sources may be mixed in any ratio and the mixture may be used. It is also appropriate to mix the gas sources or change the gas sources during fermentation or during performing the method. This is in particular possible when the partial pressures of carbon dioxide and hydrogen are determined in real time during fermentation and are adjusted or maintained at a specific value or in a specific range according to the desired carbon flux, i.e. towards cell growth in the cell growth phase or towards methane production in the methane production phase. In any way, it is not even necessary to determine the exact composition of the real gas or determine the exact hydrogen content in the real gas which is another very important advantage of the method of the invention over the methods known in the art.

The hydrogen, i.e. hydrogen gas, which is used for the method of the invention and may be converted into methane by the methanogenic microorganisms, may be pure or of high quality/purity, i.e. "ideal gas", or "real gas" characterized in that it comprises beside hydrogen also other gases which are denoted as contaminants. In general, the hydrogen may be of any source. Ideal hydrogen gases may be obtained by various purchasers which are known to the skilled person (e.g. Air Liquide, Paris, France). Hydrogen sources include but are not limited to ideal gas sources delivering ideal gas, or real gas sources delivering real gas, such as natural sources, industrial processes (industrial "waste gas") comprising the electrolysis of water and/or brine, propane dehydrogenation, hydrogen from oil refining, such as from cracking process, biomass fermentation, be made via methane steam reforming or hydrogen may be off-gas released from the method or reaction vessel of the invention or from other methanogenesis performed by methanogenic archaea. Hydrogen is recovered from the off-gas of the method using a separation device such as a membrane which allows the comparatively small hydrogen molecules to pass through the pores of the membrane and allows the comparatively large molecules of the other gas components to be rejected by the membrane. Such separation devices and membranes may be obtained for example from Du Pont (Wilmington, DE, USA) or Gore (Newark, DE, USA). The hydrogen recovered from the off-gas is then fed back into the reaction vessel via tubes or means which are known to the skilled person to be used as hydrogen source for the conversion into methane by the methanogenic microorganisms. The use of hydrogen of the off-gas of the invention improves educt utilization and process economy of the overall method. In general, hydrogen of different sources may be mixed in any ratio. As it also applies for carbon dioxide, it is also appropriate to mix the hydrogen gas sources or change the hydrogen gas sources during fermentation or during performing the method. Thus, the hydrogen content in the gas sources may vary greatly without affecting the feasibility of the invention, preferably, the hydrogen comprising real gas comprises at least 80% of hydrogen, more preferably at least 85%, even more preferably at least 90%, most preferably at least 95%. If it is necessary to purify the hydrogen comprising real gas prior to use in the method, this may be done according to any of the techniques mentioned above or known in the art. Also for hydrogen real gas, it is not necessary to determine the exact composition of the real gas or determine the exact hydrogen content in the real gas which is also a very important advantage of the method of the invention over the methods known in the art if the partial pressure ratio is controlled. "Electrolysis" in the sense of the invention means a method or process which uses an electric current to induce an otherwise non-spontaneous chemical reaction. In the process, an electric current passes through a substance, thereby causing a chemical change of said substance, usually the gaining or losing of electrons. Electrolysis requires an electrolytic cell, such as an electrolyser, e.g. Hofmann voltameter, consisting of separated positive and negative electrodes (anode and cathode, respectively) immersed in an electrolyte solution containing ions or in a molten ionic compound. Reduction occurs at the cathode, where electrons are added that combine with positively charged cations in the solution. Oxidation occurs at the anode, where negatively charged anions give up electrons. Standard or high-pressure electrolyser can be obtained from various manufacturers such as from Hydrogen Technologies (Notodden/Porsgrunn, Norway), Proton Energy Systems (Wallingford, CT, USA), Heliocentris Energy Solutions AG (Berlin, Germany), Claind (Lenno, Italy), Hydrogenics GmbH (Gladbeck, Germany), Sylatech Analysentechnik GmbH (Walzbachtal, Germany), h-tec Wasserstoff-Energie-Systeme GmbH (Luebeck, Germany), zebotec GmbH (Konstanz, Germany), $H_2$ Logic (Herning, Denmark), QuinTech (Goeppingen, Germany), and electrolysis may be performed according to the manufacturer's instructions.

In the electrolysis of water, water is used as the substance through which the electronic current passes. The electronic current leads to the decomposition of the water into oxygen ($O_2$) and hydrogen ($H_2$). The overall reaction equation is: $2\,H_2O(liquid) \rightarrow 2\,H_2(gaseous) + O_2(gaseous)$. Hydrogen can also be produced by the electrolysis of brine (i.e. a water sodium chloride mixture). This electrolysis is commonly used for the production of chlorine, wherein hydrogen is produced as a side product. Herein, the passed through current leads to the oxidation of chloride ions to chlorine. The overall reaction equation is: $2\,NaCl + 2\,H_2O \rightarrow Cl_2 + H_2 + 2\,NaOH$. The water and/or brine for the electrolysis may be obtained by any source, such as tap water, from rivers, lakes, sea water, rain or waste water from industrial processes (here the explanations of industrial waste gas apply accordingly). In case that the water and/or brine does not have the necessary purity for the electrolysis, the water and/or brine may be purified by distillation, filtration and/or centrifugation and other means which are well known to a person skilled in the art and can for example be taken from "Water Treatment, Principles and Design", 2. edition, 2005, John Wiley & Sons.

The energy for any industrial process mentioned herein which provides hydrogen, oxygen, carbon dioxide or any other precursor of the method, in particular for the electrolysis of water or brine to provide hydrogen and/or oxygen which may then be used for gasification or combustion processes to provide carbon dioxide, or for the bioreactor which requires energy, e.g. for temperature and pressure control as well as feed pumps, stirrer movement and other applications, not even mentioned the remaining laboratory equipment, may principally be provided by any energy source, such as renewable or non-renewable energy sources, such as electricity from combustion of fossil fuels and related energy carriers or nuclear energy. Preferably, the energy for the industrial processes is from a renewable energy source, in particular the energy for the hydrogen-producing electrolysis, for gasification or combustion is from a renewable energy source. "Renewable energy" is energy which comes from natural resources or sources and is renewable, i.e. naturally replenished. Renewably energy comprises wind power, solar power, geothermal power, water/hydro power, wave power, tidal power, biofuels, biomass and combinations thereof. More preferably, the energy source is selected from the group consisting of solar, wind, water and geothermal power. A "device for generating electric energy from a renewable and/or non-renewable energy source" are the reactors or devices, i.e. respective power plants, which convert the energy of the energy source, such as thermal, kinetic, chemical and/or mechanical energy into electric energy. Said power plants may be large-scale power plants or household-scale devices or else.

Solar powered electrical generation relies on concentrated solar heat (e.g. heat engines) and solar power (e.g. photovoltaics in solar panels). A device for generating electric energy from solar power may for example be selected from photovoltaics, concentrated solar power system or else and may be obtained for example from Bosch Solar Energy AG (Erfurt, Germany), sonnen_systeme Projektgesellschaft mbH (Alheim-Heinebach; Germany), First Solar (Tempe, USA), Suntech (Wuxi, China), Sharp (Tokyo, Japan), Q-Cells (Bitterfeld-Wolfen, Germany) and Stirling Energy Systems, Inc. (Scottsdale, AZ, USA). Solar hot water system solar heat engines may be used for heating the bioreactor of the system of the invention.

Biomass is also regarded as renewable energy source and is biological material from living or recently living organisms, such as wood, waste, (hydrogen) gas, and alcohol fuels. Living or recently living organisms comprise plants and trees, such as miscanthus, switchgrass, hemp, corn, poplar, willow, sorghum, sugarcane, eucalyptus or palms, but also forest residues, e.g. dead trees, branches and tree stumps, yard clippings, wood chips, garbage, plant or animal matter used for production of fibres or chemicals, and biodegradable waste that can be burnt as fuel. Excluded are such organic materials as fossil fuels which have been transformed by geological processes into substances such as coal or petroleum. Biofuels, e.g. bioethanol, biobutanol (biogasoline), biogas, syngas, bioethers, biodiesel or solid biofuels as wood pellets, cube or pucks, are a wide range of fuels which are in some way derived from biomass. The energy for the production of biomass or biofuel is thus derived from the sun and converted by photosynthesis into the biological material. Devices for generating electric energy from biomass or biofuel comprise biomass power plants, and can be obtained for example from SBBiogas GmbH (Marktbreit, Germany), Evonik New Energies GmbH (Saarbruecken, Germany) or Envi Con & Plant Engineering GmbH (Nuremberg, Germany).

A device for generating electric energy from wind power may for example be selected from wind turbine, wind mill, wind pump or drainage. Suppliers of said devices are for example for small applications: Superwind (Bruehl, Germany), Braun Windturbinen (Nauroth, Germany), Urban Green Energy (New York, USA) and Helix Wind (Poway, USA). Exemplary suppliers of wind turbines for larger applications are Vestas (Randers, Denmark), GE Wind Energy (Fairfield, USA), Sinovel (Beijing, China), Enercon (Aurich, Germany), Goldwind (Urumqui, China), Gamesa (Zamudio, Spain) and Siemens Wind Power (Brande, Denmark).

Geothermal electricity is electricity generated from geothermal energy. A device for generating electric energy from geothermal power may for example be selected from dry steam power plant, flash steam power plant, hybrid plant and binary cycle power plant and may be obtained for example from Ansaldo Energia (Genoa, Italy) or ECONAR (Maple Grove, MN, USA).

In general, the production of hydro electricity uses the gravitational force of falling or flowing water. Conventional hydroelectric power stations use the potential energy of dammed water for driving a water turbine and generator. The energy amount obtained in this way depends on the water volume and the difference in height between the source and the water's outflow. Pumped-storage hydroelectric power stations produce electric energy to supply high peak demands by moving water between reservoirs of different levels. Here, water is released back into the lower reservoir through a turbine which is connected to a generator or preferably may be connected to the bioreactor or device for producing hydrogen and/or oxygen by the electrolysis of water and/or brine. Also, run-of-the-river hydroelectric stations, small, micro or pico hydro stations are available. A device for generating electric energy from water/hydro power may for example be obtained from AC-Tec G.m.b.H (Kaltern, Italy) or EnBW Energie Baden-Württemberg AG (Karlsruhe, Germany).

Wave power denotes the energy of ocean surface waves. A device for generating electric energy from wave power may for example be selected from point absorber or buoy, surfacing following or attenuator oriented parallel to the direction of wave propagation, terminator, submerged pressure differential, oriented perpendicular to the direction of wave propagation, oscillating water column, or an overtopping facility. The devices comprise a hydraulic ram, elastomeric hose pump, pump-to-shore, hydroelectric turbine, air turbine or a linear electrical generator, optionally also a parabolic for increasing the wave energy at the point of capture. Such devices may be located close to shoreline, near shore or offshore. Suppliers are for example Ocean Navitas Ltd. (Marton, UK), AWS Ocean Energy Ltd. (Inverness, UK), BioPower Systems (Mascot, Australia), Columbia Power Technologies (Corvallis, OR, USA), Ocean Power Technologies (Pennington, NJ, USA) or Pelamis Wave Power (Edinburgh, UK).

A device for generating electric energy from tidal power which uses the daily rise and fall of water due to tides may for example be selected from tidal power stations, undershot waterwheels, dynamic tidal power plants, tidal barrage, tidal lagoons and turbine technology, such as axial turbines or crossflow turbines. Suppliers are for example BioPower Systems (Mascot, Australia), Neptune Renewable Energy Ltd. (North Ferriby, UK) or Clean Current Power Systems Incorporated (Vancouver, Canada).

Carbon dioxide and hydrogen are both required for methane production. Both gases are generally fed into the reaction vessel of the invention at a certain gas feed rate as described above. Optionally, both gases may be introduced into the reaction vessel according to the desired ratio of their partial pressures or according to a certain volumetric ratio. They may be pure or contaminated with other gases tolerated by the system, i.e. not irreversibly harmful to the methanogenic microorganisms. A contamination with other gases may e.g. occur when industrial waste gases of carbon dioxide from the atmosphere are used. Positively, this also reduces the release of greenhouse gases or removes carbon dioxide from the atmosphere. Inside the reaction vessel, the gases are partially dissolved in the medium or reaction medium of the invention which comprises the methanogenic microorganisms. The dissolved part of the gases is then taken up by the microorganisms of the invention and converted into methane, subsequently. The gases may be fed separately into the reaction vessel or be mixed outside the reaction vessel in a feed rate which is at least 1 vvm in total, optionally in a ratio which results in a certain partial pressure ratio or certain volumetric ratio and then fed in a mixture. Hence, the in-gas or gas feed of hydrogen and carbon dioxide always denotes the total amount of hydrogen and carbon dioxide provided to the methanogenic microorganisms. For the purpose of the invention it is suitable to either mix the two gases hydrogen and carbon dioxide prior to the introduction into the reaction vessel or to introduce both gases separately via two different gas feed lines to the reaction vessel or to introduce the two gases timely one after another, as long as the gas feed rate is maintained, and optionally the volumetric ratio of hydrogen to carbon dioxide or the ratio of the partial pressures of hydrogen to carbon dioxide is maintained as described herein. The mix-ratio may be adjusted in real time according to the determined partial pressure ratio or volumetric ratio of hydrogen and carbon dioxide inside the reaction vessel as desired by the skilled person and as described above.

Typical "contaminants" of the gases which are fed into the reaction vessel of the invention are process dependent and may be oxygen, chlorine, carbon monoxide, nitrogen and others. In response to oxygen contamination of the reaction vessel, the oxidation reduction potential has to be stabilized using sulphide or else, adjusting the hydrogen gas feed into the reaction vessel and the pH value, wherein the concrete action may be chosen by the person skilled in the art.

A "fermentation condition" or "phase condition" is a parameter of the method of the condition which may be adjusted by the skilled person who performs the method of the invention. In general, "fermentation conditions" in the sense of the invention are all standard fermentation conditions which are known to a person skilled in the art of microorganism fermentation (Bailey, Ollis (1986) Biochemical Engineering Funda-mentals, McGraw-Hill Science, New York, USA). Examples of fermentation conditions are the partial pressures of hydrogen and carbon dioxide and thus the ratio of the partial pressures of hydrogen and carbon dioxide, the oxidation reduction potential, the pH value, biomass concentration, temperature, pressure, stirring speed, medium composition, gas feed rates, medium exchange rates and others. The skilled person knows that in a reaction vessel containing a suspension of living organisms such as the methanogenic microorganisms of the invention the fermentation conditions, which are required for optimal cell performance regarding cell growth or methane production, may fluctuate and vary and will have to be adjusted accordingly by the experimenter. Since all relevant fermentation conditions may be measured or determined either in real time or by simple means, the skilled person knows how to the fermentation conditions can be adjusted to successfully perform the method of the invention.

The optional cell growth phase and the optional methane production phase are characterized by a different ratio of the partial pressure of hydrogen to the partial pressure of carbon dioxide inside the reaction vessel. Optionally, also the oxidation reduction potential is different in both phases, values are given above. The $H_2/CO_2$ partial pressure ratios and the optional different oxidation reduction potential of both phases apply to all methanogenic microorganisms of the invention, preferably to the methanogenic archaea, described herein. The optimal fermentation temperature and pH depend on the cell species and microorganism strain used and information thereon will be given in detail below. All other fermentation conditions apply to both phases and to all species of methanogenic microorganisms, i.e. have no significant effect on cell growth or methane production of the methanogenic microorganisms. The $H_2/CO_2$ partial pressure ratio may be adjusted or maintained according to the real time measurement of the reaction vessel.

It is possible to feed both gases hydrogen and carbon dioxide separately in the amount which is required to adjust or maintain a specific ratio inside the reaction vessel or to mix both gases prior to the feed into the reaction vessel in a ratio which results in the desired ratio of the partial pressures or volumetric ratio of carbon dioxide and hydrogen inside the reaction vessel. The ratio of the partial pressures of hydrogen to the partial pressure of carbon dioxide may be determined in real time as described above and the volumetric ratio may be determined via the mass flow. Also, the gas feed rate ([vvm]) is controlled via typical mass flow controllers which are well known to a person skilled in the art. Typical mass flow controllers are e.g. supplied by Bronkhorst (Kamen, Germany), Wagner (Offenbach, Germany), Brooks Instruments (Dresden, Germany) or others.

"Tolerated" means that something, which is not directly having a beneficial and/or positive effect on the process of electrolysis, generating electric energy or the methanogenic microorganism, the reaction vessel, the experimental set-up of the method of the invention as a whole, the composition of the gases fed into the reaction vessel or off-gas and/or the methane production rate of the invention, nevertheless does not lead to methane production inefficiency or overall process inefficiency of the method of the invention or prevents the success of the method of the invention or the use of the methanogenic microorganism.

The temperature inside the reaction vessel depends on the methanogenic microorganism which is used for the method of the invention. In general, for all methanogenic archaea the temperature should be in the range from 40 °C to 100 °C. Preferably, for mesophilic microorganisms, such as *Methanosarcinia barkeri* or *Methanococcus maripaludis,* the temperature of the biomass in the reaction vessel is in the range of about 30 °C to about 40 °C, more preferably in the range of about 35 °C to about 37 °C. Preferably, for thermophiles such as *Methanothermobacter marburgensis, Methanobacterium thermoautotrophicus* or *Methanothermobacter thermoautotrophicus* the temperature inside the bioreactor was about 60 °C to 67 °C, preferably about 65±1 °C, or about 85 °C to 90 °C for organisms such as *Methanocaldococcus jannaschii.* Surprisingly, the inventors found out that *Methanothermobacter marburgensis* also grows and produces methane very well at lower temperatures of about 45±1 °C to about 55±1 °C. Hence, the method of the invention may also be performed at 45±1 °C, 47±1 °C, 49±1 °C, 50±1 °C, 52±1 °C, 54±1 °C, 55±1 °C, 57±1 °C, 59±1 °C, 60±1 °C, 62±1 °C, 64±1 °C, 67±1 °C, 69±1 °C or 70±1 °C or at a temperature in the range of 45±1 °C to 67±1 °C, 50±1 °C to 65±1 °C inside the reaction vessel.

The stirring speed is adjusted to a value which is optimal for a high gas liquid mass transfer in the volume of the respective reaction vessel, in a typical fermentation the stirring speed is at least 20 rpm or 50 rpm, preferably, at least 500 rpm, more preferably at least 1000 rpm, even more preferably at least 1500 rpm, most preferred, the stirring speed is at least 1500 rpm or the maximum which can be applied by the stirrer used in the reaction vessel of the bioreactor. Thus, the stirring speed in particular of large-volume reaction vessels, such as 15,000 L, may be significantly less than 1500 rpm, for example in the range between 20 rpm to 100 rpm, e.g. 65 rpm.

The pH of the cell culture inside the reaction vessel should be broadly in the neutral range. Preferably, for all methanogenic microorganisms of the invention the pH value of the invention is in the range of 5.0 to 8.0, more preferably, the pH value is in the range of 5.5 to 7.8, even more preferably, the pH value is in the range of 6.4 to 7.6, in particular preferably, the pH value is in the range of 6.7 to 7.2, most preferably, the pH value is about $6.9 \pm 0.5$ or $7.0 \pm 0.5$.

In general, methanogenesis is an anaerobic process and inhibited by oxygen. However, certain amounts of oxygen may be tolerated by the system. If the oxygen content in a real gas is too high, for example a common oxygen scrubber may be used to remove oxygen from the in-gases, i.e. from the hydrogen and carbon dioxide feed. After an accidental inflow of high amounts of oxygen, it may also be possible to remove the oxygen by fast feed or high gas feed rate of hydrogen and carbon dioxide into the reaction vessel and oxygen clean-up of off-gas which is removed from the reaction vessel at the same time. Also here, a common oxygen scrubber may be used. Experiments, for example in WO 2008/094282, have shown that even 100% oxygen feed for more than 10 hours only stopped methane production for a that time and that the system soon recovered and started to again produce methane. In no way, the system will be permanently damaged. Thus, the system is very robust to oxygen contamination and oxygen is tolerated to a certain extend. It is, however, preferred to have a very low oxygen contamination inside reaction vessel, preferably below 2%.

Inside the reaction vessel and during the method of the invention, the pressure is as defined in the claims and may be adjusted to a value of at least 5 bar. Also disclosed is an absolute pressure inside the reaction vessel in the range of 1.0 bar to 1200 bar, 1.2 bar to 1000 bar, 1.3 bar to 500 bar, preferably in the range of 1.5 bar to 1000 bar. In particular, 5 bar has been calculated by the inventors to be optimal for the production of methane and would be well tolerable by the methanogenic microorganisms of the present invention. Since methanogenic microorganisms, such as methanogenic archaea, are known which tolerate very well extremely high pressures the pressure values mentioned before and in the following may be applied in the method of the invention for producing methane. Methanogenic archaea which are extremely tolerable to high pressures are naturally derived from a high pressure environment, such as from deep sea.

"Absolute pressure" in the sense of the invention denotes the total pressure which is inside the reaction vessel including the normal atmospheric pressure and the additional pressure applied by the method operator via a device such as the device according to the invention for providing, controlling and/or measuring an absolute pressure inside the reaction vessel of at least 1 bar or higher as described above. A person skilled in the art knows very well that the normal atmospheric pressure may vary with time and also depending on the location where a method is performed. Atmospheric pressure is defined as the force per unit area exerted onto a surface by the weight of air above that surface in the atmosphere. In most circumstances atmospheric pressure is closely approximated by the hydrostatic pressure caused by the mass of air above the measurement point. As elevation increases, there is less overlying atmospheric mass, so that pressure decreases with increasing elevation. The average sea-level pressure is 1.01325 bar (= 1013.25 hPa ) and the atmospheric pressure may generally vary within $\pm$ 50 mbar. Based on this, "normal atmospheric pressure" in the sense of the invention is generally a pressure of $1 \pm 0.05$ bar, however, depending on the location of operation "an absolute pressure above normal atmospheric pressure" denotes any additional increase of the pressure inside the reaction vessel compared to the outside of the reaction vessel. A person skilled in the art knows very well how to determine or find out the pressure which applies to his location. The pressure inside the reaction vessel may also be given in the unit "barg" which denotes the pressure inside the reaction vessel independent from the atmospheric pressure. In this case, the local atmospheric pressure value has to be added to the pressure given as "barg". As a general rule, the absolute pressure may be calculated by adding the pressure value given as "barg" to $1 \pm 0.05$ bar. Of course, the absolute pressure may also be measured directly. Exemplary suppliers of pressure controllers are e.g. LABOM Mess- und Regeltechnik GmbH (Hude, Germany), KROHNE Messtechnik GmbH (Duisburg, Germany) or others.

In an optional embodiment of the present invention, the gas feed rate of hydrogen and carbon dioxide into the reaction vessel is at least 2.0 vvm, at least 2.5 vvm, at least 3.0 vvm, at least 4.0 vvm, at least 5.0 vvm, at least 7 vvm, at least 10.0 vvm, or at least 20.0 vvm in total and inside the reaction vessel an absolute pressure of at least 5.0 bar (4.0 barg) is applied.

In another optional embodiment of the present invention, the gas feed rate of hydrogen and carbon dioxide, i.e. the sum gas feed rate of hydrogen and carbon dioxide, i.e. the gas feed rate of hydrogen plus the gas feed rate of carbon dioxide, into the reaction vessel is in the range of 1.2 vvm to 100.0 vvm, in total 1.2 vvm to 25.0 vvm in total, more preferably is in the range of 1.5 vvm to 15.0 vvm in total, even more preferably is in the range of 1.7 vvm to 10.0 vvm in total, most preferably is in the range of 2.0 vvm to 8.0 vvm in total, and the pressure is adjusted to a value of at least 5 bar.

During the whole method, it is appropriate to adapt the fermentation conditions, such as the stirring speed, the temperature, the supply rate of any liquid, gaseous or solid compound, the pressure and/or pH value in order to change the fermentation conditions inside the bioreactor comprising the reaction vessel for various purposes including but not limited to the increase of methane production and/or of cell growth. Classically, two fermentation modes are applicable in the methods described herein (in the literature also denoted as phases although herein not to be mixed up with the "phases of the invention" characterized by a specific $H_2/CO_2$ partial pressure ratio): the batch mode (= batch cultivation mode or batch fermentation mode) and the continuous mode (=continuous cultivation mode or continuous fermentation mode). In the batch mode, typically no mass (solid, liquid, gaseous) transfer into the reaction vessel or into the bioreactor is allowed. Thus, during batch mode the system comprising the bioreactor and the methanogenic cells in the reaction vessel is closed. The only exception is that hydrogen and carbon dioxide may still be fed into the reaction vessel and be provided to the methanogenic microorganisms inside the reaction vessel or not. If this is the case, the fermentation mode may also be regarded as semi-batch mode. On the contrary, the continuous cultivation mode is characterized by a continuous supply with fresh medium or medium components, gases or other substances which are continuously required by the cells in the reaction vessel, such as hydrogen, carbon dioxide or medium. In general, the reaction volume, i.e. the culture volume, inside the reaction vessel is kept constant, so that also gaseous and/or liquid substances have to be removed from the reaction vessel depending on the total mass in-flow. Thus, during the continuous mode, the system comprising the bioreactor and the methanogenic cells in the reaction vessel can be regarded as being open and components are exchanged.

Hence, these two fermentation modes describe a setting of the bioreactor comprised in the system of the invention. Batch mode conditions are very often applied during the initial phase when cells are dividing and biomass is increasing exponentially, whereas continuous cultivation conditions are often applied when cell growth stagnates or is constant. In this mode, the cells are usually kept over a longer period of time for producing the desired product, here methane.

During the optional methane production phase or optional cell growth of the method characterized by the partial pressure ratios of hydrogen and carbon dioxide as described above, the fermentation mode may be batch mode or continuous mode. If the cells are only kept in the methane production phase according to the partial pressure ratio of hydrogen and carbon dioxide, batch and continuous conditions may be applied as regarded beneficial by the experimenter, however, the inventor found out that best results may be achieved when the mode is switched from batch to continuous when cell growth stagnates. If at least one cell growth phase and at least one methane production phase are applied, it was found out that it is especially beneficial if the batch mode conditions are applied during cell growth phase and the continuous cultivation mode is applied during methane production phase. The switch between both phases and mode is then performed as described above when cell growth stagnates or reaches at least a concentration of 3 g biomass per litre.

The bioreactor (= fermenter or reactor) comprises all components and devices which are necessary to perform the method of the invention, i.e. to produce methane of hydrogen and carbon dioxide. For this purpose, the bioreactor comprises at least a reaction vessel suitable for fermenting, growing and culturing the methanogenic microorganisms of the invention, devices for the supply of liquid, solid and gaseous substances and for analytics as already described above. "Suitable for fermenting, growing and culturing the methanogenic microorganisms" in the sense of the invention denotes that the reaction vessel allows to maintain a surrounding or condition which promotes cell growth, fermentation and culturing of the cells. This comprises, for example, maintaining the culture at a pre-determined temperature, stirring the cells and providing substances which are essential for the metabolism of the cells, such as gases, medium or medium components. In principle, the reaction vessel may be any container wherein methanogenic microorganism may be cultured, grown and fermented. In general, the reaction vessel is made of glass or steel depending on the pressure which is applied to the culture of methanogenic microorganisms. The reaction vessel may be connected to all necessary devices and measuring probes for adjusting the fermentation conditions of the invention to grow the methanogenic microorganisms up to a desired biomass concentration and/or to switch the phase of the methanogenic microorganisms or to keep the cells in one of the phases over a period of time which may be selected from the experimenter. The period of time may be understood as minutes or hours or days or even longer. The method of the invention also allows keeping the methanogenic microorganisms in a non-methane-producing state without further cell growth.

The bioreactor may be derived from a standard bioreactor suitable for culturing, fermenting, growing and/or processing of a biomass comprising the methanogenic microorganisms of the invention. Suppliers of standard bioreactors are, for example, Applikon Biotechnology B.V. (Schiedam, The Netherlands), Infors (Bottmingen, Switzerland), Bioengineering (Wald, Switzerland) and Sartorius Stedim Biotech GmbH (Göttingen, Germany).

The bioreactor, i.e. reactor comprises at least one vessel or container suitable for culturing, fermenting and/or processing of the methanogenic microorganisms, i.e. the reaction vessel of the invention. The bioreactor may also comprise two or more reaction vessels, one or more for cell growth and also one or more for methane production. Even different gas feed rates of hydrogen and carbon dioxide, different pressures inside the reaction vessels, different methanogenic microorganisms and different ratios of the partial pressure of hydrogen to carbon dioxide may be applied in the reaction vessels which are used for the invention. These reaction vessels may be linked, for example via tubes, pipes or else, to transport the cell culture comprising the suspension of methanogenic microorganisms from one reaction vessel to the other or back if needed. Said one or more reaction vessels may also not be directly linked to each other, in this case, the methanogenic microorganism may be transferred from one reaction vessel to the other via another container. The bioreactor may be equipped with various means and devices for adjusting and monitoring the fermentation conditions in the bioreactor and in the reaction vessel or in the reaction vessels. With reference to the bioreactor, "suitable for the method" denotes that the bioreactor comprises the components and devices which are necessary to culture the microorganisms of the invention in a way that they are able to grow and produce methane and what else is necessary to perform the method of the invention.

Optionally, the bioreactor comprises a device for a feed of medium, e.g. a continuous feed of medium, and/or reducing agent and/or sulphur source.

An ideal stirred tank bioreactor may be used for the method of the invention. This bioreactor comprises a reaction vessel with head plate and multiple inlet and outlet ports. The probes for temperature, pH and oxidation reduction potential measurement are fitted to the ports. The bioreactor is further equipped with an agitator with impeller blades (Rushton) on at least three levels. Medium and liquid reducing agent is fed via a pump, controlled gravimetrically or with a flow meter or is needed to control the oxidation reduction potential. Acid or base is added by titration to control pH as desired. "Gas liquid mass transfer" denotes the transfer of mass in general, i.e. molecules and atoms, between a liquid and a gaseous phase, wherein phase is to be understood in its thermodynamic meaning and not to be mixed up with a fermentation "phase of the invention". Of particular importance for the method of the invention is the transfer of hydrogen and carbon dioxide from the gaseous phase to the liquid phase comprising the biomass suspension and the methanogenic microorganism inside the reaction vessel, and the transfer of methane from the liquid phase to the gaseous phase inside the reaction vessel to leave the reaction vessel. The gas liquid mass transfer indirectly influences the mass transfer from the liquid to the solid phase, such as the biomass.

The bioreactor and also the reaction vessel comprised in the bioreactor may be connected to various sources of hydrogen and carbon dioxide, nitrogen, real gases and others through the at least one device for introducing of gaseous substances and further to automated systems for feeding liquid, gaseous and solid substances and materials into the bioreactor via peristaltic pumps and others. Also devices and means may be attached to the bioreactor which measure the composition of the educt gases for the method and which further improve the quality of the gas to be fed into the reaction vessel, in case of a real gas application, or the off-gas. The bioreactor also comprises a device for removing and measuring the off-gas from the reaction vessel. The off-gas composition, in particular the amounts of hydrogen, carbon dioxide and methane, may be measured for example by an individually applied gas analyser system, such as supplied by BlueSens gas sensor GmbH (Herten, Germany). The off-gas produced by the method of the invention may optionally be further processed by standard means, such that hydrogen and carbon dioxide, which were not converted into methane, are separated from methane and or from each other. Hydrogen and carbon dioxide which are comprised in the off-gas are preferably recycled in that they are fed back into the reaction vessel of the invention. Hydrogen may be separated from the off-gas of the invention using a separation device such as a membrane which allows the small hydrogen molecules to pass through the pores of the membrane and does not allow the passage of larger molecules or by classical condensation using a cold finger or else. Aforementioned methods are of course also applicable to increase the hydrogen content in the used hydrogen educt gas or real gas. Carbon dioxide may be separated from the off-gas of the invention for example with an absorbing medium which selectively absorbs carbon dioxide or which selectively absorbs gases other than carbon dioxide from the off-gas or by any method described above. Both hydrogen and carbon dioxide which have been recovered from the off-gas may be used as educts in the method of the invention. After or before or while hydrogen and carbon dioxide are removed from the off-gas also other gases such as water vapour and other gases may be removed to increase the percentage of methane in the off-gas for methane or natural gas based applications. The higher the percentage of methane, and thus the purity, in the off-gas, the more preferred. Most preferred is a percentage of methane in the off-gas which closely resembles natural gas, i.e. is between 40% and 90%, preferably between 60% and at least 90%.

The bioreactor may further comprise devices which allow the sterile and anaerobic entry and exit of substances required for the method of the invention. "Substances required for the method of the invention" are for example the biomass comprising the methanogenic microorganisms, the medium or medium components, water, gases, such as hydrogen, carbon dioxide, methane, hydrogen sulphide, nitrogen or mixtures thereof, and compositions which might contain a mixture of all these substances.

The bioreactor is further equipped with discharge lines, pumps and compressors. The bioreactor at least comprises all devices which are necessary to feed the gases which are required for the method of the invention, i.e. at least hydrogen and carbon dioxide, liquid substances, such as the cell culture suspension and fresh medium or medium components, devices for removing the off-gas of the invention, excess liquid and gaseous parts from inside the bioreactor, and all other components which are necessary to control the fermentation parameters of the invention.

Preferably, the bioreactor and reaction vessel is suitable for culturing, fermenting and/or processing of a biomass comprising microorganisms under high pressure, e.g. a pressure higher than the normal atmospheric pressure. For this purpose, the bioreactor comprises a reaction vessel or housing made of a material which is suitable for high pressure and fulfils the requirements of occupational safety and follows work safety rules. The material of the housing may be selected from the group comprising steel, such as stainless steel according to European standard EN 10088, for example 1.43XX, 1.44XX, 1.45XX, or else.

A reaction vessel of the bioreactor for culturing methanogenic microorganisms may have different volumes, wherein larger volumes are preferred for the industrial production of methane. The industrial production of methane also comprises the methane production in decentralized household applications, wherein the volume of the reaction vessel comprised in the bioreactor may be in the smaller and middle range, and largely depends on the particular methane amount which is needed and the available space. In any embodiment of the invention, the volume of the reaction vessel is at least 1 L, preferably at least 5 L, 10 L, 25 L, more preferably at least 50 L, 100 L, 500 L, 1000 L, even more preferably the volume of the reaction vessel is at least 5000 L, 10,000 L, 20,000 L, 50,000 L, most preferably the volume of the reaction vessel is at least 100,000 L. In another preferred embodiment, more than one reaction vessel is comprised in the bioreactor of the invention or more than one bioreactor comprising one or more reaction vessels are combined, for example attached serially or in parallel. In a serial arrangement, the volume of the reaction vessel is typically smaller, i.e. in the range of 0.5 L to 20 L, preferably in the range of 1 L to 15 L, more preferably in the range of 1 L to 10 L and most preferably in the range of 1 L to 5 L, however, if regarded beneficial, the volume of the reaction vessel may also be larger, as indicated above, for example at least 1 L, preferably at least 5 L, 10 L, 25 L, more preferably at least 50 L, 100 L, 500 L, 1000 L, even more preferably the volume of the reaction vessel is at least 5000 L, 10,000 L, 20,000 L, 50,000 L, or the volume of the reaction vessel is at least 100,000 L. A serial or parallel arrangement allows using the hydrogen, methane and carbon dioxide comprising off-gas as new educt gases or in-gas for the next following reaction vessel and bioreactor. Optionally, the methane is removed from the off-gas before it is re-used as in-gas. Thereby, the process economy is improved and methanogenesis educts, i.e. hydrogen and carbon dioxide, which have not been converted into methane in the preceding reaction vessel, may again be used. Of course, in each reaction vessel, the fermentation conditions and in particular the $H_2/CO_2$ partial pressure ratio and thus the phase of the methanogenic microorganisms may be applied individually. It is also possible to use different methanogenic species and mixtures thereof in each of the reaction vessels.

In a preferred embodiment of the method of the invention, the bioreactor is designed in a way that the carbon dioxide and the hydrogen released from the reaction vessel in the off-gas of the invention is re-used in the method of the invention. Thus, the hydrogen and carbon dioxide of the off-gas of the invention are recycled.

**BRIEF DESCRIPTION OF THE DRAWINGS**

Figure 1: General set-up of the system. The system comprises at least one device for generating electric energy from a renewable and/or non-renewable energy source (1), at least one device for producing hydrogen and/or oxygen by the electrolysis of water and/or brine (2), preferably an electrolyser, and at least one bioreactor (3) comprising a reaction vessel suitable for growing, fermenting and/or culturing methanogenic microorganisms for converting hydrogen and carbon dioxide into methane and at least one device for providing a gas feed of hydrogen and carbon dioxide into the reaction vessel. Optionally, the renewable energy source comprises solar energy, wind power, wave power, tidal power, water/hydro power, geothermal energy, biomass and biofuel combustion. Especially for initiating the system, it might also be necessary to introduce energy from outside, i.e. from a generator or from the common public power supply system. Arrows indicate the transfer of one or more compounds (e.g. electric energy, liquid, gaseous and/or solid compounds) between the devices/bioreactor(s) or else of the system. The electric energy from a renewable and/or non-renewable energy source may be transferred via general power supply lines or cables or else to the at least one device for producing hydrogen and/or oxygen by the electrolysis of water and/or brine (2), preferably to an electrolyser, and/or to the at least one bioreactor (3) and/or optionally be at least partially stored in an electric energy storage device (4), such as a battery or accumulator or else. In general and applicable to all embodiments of the invention, the electric energy storage device (4) may be any common device suitable for storing electric energy, such as a common battery or common accumulator to be used according to the manufacturer's instructions. From the electric energy storage device (4) the electric energy may be transferred via general power supply lines or cables or else to the at least one device for producing hydrogen and/or oxygen by the electrolysis of water and/or brine (2), preferably to an electrolyser, and/or to the at least one bioreactor (3). Water and/or brine are provided via general transportation or tubes, pipes or else but also via water tanks transported by transportation systems such as trains, cars, trucks or else. Water in general, and in particular water from rain or water which was purified in order to be suitable for the use in electrolysis may be stored in water tanks, cisterns, artificial lakes, reservoirs or else. The hydrogen obtained by electrolysis is transferred to the bioreactor (3), where it is converted together with carbon dioxide into methane by methanogenic microorganisms. Hydrogen and carbon dioxide may be transferred to the bioreactor via standard gas transport means, such as tubes, pipes or else. Both may also be delivered, for example, in gas cylinders or bottles which allow for storage of carbon dioxide and/or hydrogen before introduction into the bioreactor. Gas cylinders, storage cylinders, storage vessels and/or bottles not only allow storage of carbon dioxide or of hydrogen after electrolysis in case of excess production or else prior to introduction into the bioreactor, but also allow individual transportation of carbon dioxide and/or hydrogen to bioreactors which are - for example - not connected to any gas transport means, such as tubes, pipes or else or storage or transportation of the off-gas or methane prior to end-user applications. The off-gas is removed from the bioreactor via the standard means of the bioreactor, such as tubes, pipes or else and may be transported directly to end-user applications via tubes, pipes or else or in gas cylinders, storage cylinders, storage vessels and/or bottles or be further processed as described elsewhere in the description. These standard means are familiar to a person skilled in biotechnology and can also be taken from the bioreactor manufacturer's instructions. The off-gas comprises methane, water vapour, hydrogen and carbon dioxide but also other gases which are present inside the reaction vessel as described below. Preferably, said bioreactor further comprises at least one device for measuring the head pressure and the off-gas concentration, at least one device for adjusting or maintaining the partial pressure ratio of hydrogen to carbon dioxide inside the reaction vessel, and at least a device for providing, controlling and/or measuring an absolute pressure inside the reaction vessel of at least 1.5 bar. Please note that the size or thickness of any symbol, arrow, font or else is not intended to express yields, importance or conversion rates.

Figure 2: Exemplary set-up of the system which illustrates examples for the further use of the off-gas of the invention which is released from the bioreactor/reaction vessel. By standard gas transportation means as described herein (tubes, pipes, gas cylinders/bottles or else) the off-gas in transferred to one or more devices for recovery, purification, enriching, storing, recycling and/or further processing of off-gas (6). These devices (6) may be combined or used individually in any embodiment of the system of the invention described herein and comprise for example devices for increasing the content of methane in the off-gas by removing other substances contained in the off-gas, such as for example hydrogen or carbon dioxide, and/or remove methane from the off-gas. Thereby, methane is purified and also enriched for various end-user applications (7), wherein methane is used as energy carrier for thermal, mechanical, electric energy or else and/or wherein methane is converted into diverse base chemicals, such as methanol, olefin or gasoline. Exemplary devices for all purposes are described in detail above and in the description. Devices for storing off-gas, methane, carbon dioxide or hydrogen may be gas tanks, cylinders, bottles or any other suitable vessel or reservoir. The off-gas, hydrogen and/or carbon dioxide may be fed back into one or more bioreactors (3). In general, methane optionally burns with oxygen, such as comprised in air, ideal or real gas or produced by electrolysis of water, to carbon dioxide, which may be re-used for methane production in the bioreactor by the methanogenic microorganisms, and water, which may be re-used for electrolysis for hydrogen and oxygen production. Optionally, also a device for oxygen enriched combustion or gasification may be present. For all other components or devices of the exemplary embodiment of the system of the invention depicted herein, for example (1), (2), (3) or (5) please refer to the explanation of Figures 1, 3 or 4. Of course, also here optionally an electric energy storage device (4), such as a battery or accumulator or else, may be present. Further, all components or intermediates may be stored in gas tanks, cylinders, bottles or any other suitable vessel or reservoir until further use or for transportation reasons. Please note that the size or thickness of any symbol, arrow, font or else is not intended to express yields, importance or conversion rates. Of course, this exemplary embodiment may comprise the features depicted in Figure 1 or 3 or any other embodiment described herein and in the description. All components shown in the Figure may be individually selected by the person skilled in the art and combined or left away.

Figure 3: Exemplary set-up of the system comprising at least one device for generating electric energy from a renewable energy source (1), at least one device for producing hydrogen and/or oxygen by the electrolysis of water and/or brine (2), preferably an electrolyser, and at least one bioreactor (3) comprising a reaction vessel suitable for growing, fermenting and/or culturing methanogenic microorganisms for converting hydrogen and carbon dioxide into methane and at least one device for providing a gas feed of hydrogen and carbon dioxide into the reaction vessel. Optionally, the renewable energy source comprises solar energy, wind power, wave power, tidal power, water/hydro power, geothermal energy, biomass and biofuel combustion. Especially for initiating the system, it might also be necessary to introduce energy from outside, i.e. from a generator or from the common public power supply system. Arrows indicate the transfer of one or more compounds (e.g. electric energy, liquid, gaseous and/or solid compounds) between the devices or bioreactor(s) or else of the system. The electric energy from the renewable energy source, may be transferred via general power supply lines or cables or else to the at least one device for producing hydrogen and/or oxygen by the electrolysis of water and/or brine (2), preferably to an electrolyser, and/or to the at least one bioreactor (3) and/or optionally be at least partially stored in an electric energy storage device (4), such as a battery or accumulator or else. In general and applicable to all embodiments of the invention, the electric energy storage device (4), such as a battery or accumulator or else may be any common device suitable for storing electric energy, such as a common battery or common accumulator to be used according to the manufacturer's instructions. From the electric energy storage device (4) the electric energy may be transferred via general power supply lines or cables or else to the at least one device for producing hydrogen and/or oxygen by the electrolysis of water and/or brine (2), preferably to an electrolyser, and/or to the at least one bioreactor (3). Water and/or brine are provided via general transportation or tubes, pipes or else but also via water tanks transported by transportation systems such as trains, cars, trucks or else. Water in general, and in particular water from rain or water which was purified in order to be suitable for the use in electrolysis may be stored in water tanks, cisterns, artificial lakes, reservoirs or else. The hydrogen obtained by electrolysis is transferred to the bioreactor (3), where it is converted together with carbon dioxide into methane by methanogenic microorganisms. Hydrogen and carbon dioxide may be transferred to the bioreactor via standard gas transport means, such as tubes, pipes or else. Both may also be delivered, for example, in gas cylinders or bottles which allow for storage of carbon dioxide and/or hydrogen before introduction into the bioreactor. Gas cylinders, storage cylinders, storage vessels and/or bottles not only allow storage of carbon dioxide or of hydrogen after electrolysis in case of excess production or else prior to introduction into the bioreactor, but also allow individual transportation of carbon dioxide and/or hydrogen to bioreactors which are - for example - not connected to any gas transport means, such as tubes, pipes or else or storage or transportation of the off-gas or methane prior to end-user applications. The off-gas is removed from the bioreactor via the standard means of the bioreactor, such as tubes, pipes or else and may be transported directly to end-user applications via tubes, pipes or else or in gas cylinders, storage cylinders, storage vessels and/or bottles or be further processed as described elsewhere in the description. These standard means are familiar to a person skilled in biotechnology and can also be taken from the bioreactor manufacturer's instructions. The off-gas comprises methane, water vapour, hydrogen and carbon dioxide but also other gases which are present inside the reaction vessel as described elsewhere in the description. Preferably, said bioreactor further comprises at least one device for measuring the head pressure and the off-gas concentration, at least one device for adjusting or maintaining the partial pressure ratio of hydrogen to carbon dioxide inside the reaction vessel, and at least adevice for providing, controlling and/or measuring an absolute pressure inside the reaction vessel of at least 1.5 bar. Please note that the size or thickness of any symbol, arrow, font or else is not intended to express yields, importance or conversion rates.

Figure 4: Exemplary set-up of the system further comprising at least one device for oxygen enriched combustion or gasification (5) for producing carbon dioxide. Preferably, the device for oxygen enriched combustion (5) or gasification (5) uses the oxygen produced by the electrolysis of water in device (2) for producing carbon dioxide, said carbon dioxide being transferred to the bioreactor (3), thereby being transferred to the methanogenic microorganisms inside the reaction vessel comprised in the bioreactor. Optionally, additional carbon dioxide from any other carbon dioxide source mentioned herein may be introduced into the bioreactor (3). For combustion or oxygen enriched combustion or gasification, any fuel and/or carbonaceous material, e.g. petroleum, wood, coal, living and/or dead biomass, is required and may be delivered via standard transportation as known to a person skilled in the art. For all other components of the exemplary embodiment of the system depicted herein please refer to the explanation of Figures 1 to 3. Of course, also here optionally an electric energy storage device (4), such as a battery or accumulator or else, may be present. Further, all components or intermediates may be stored in gas tanks, cylinders, bottles or any other suitable vessel or reservoir until further use or for transportation reasons. Please note that the size or thickness of any symbol, arrow, font or else is not

Figure 5: intended to express yields, importance or conversion rates. Volumetric productivity as a function of the partial pressure ratio of hydrogen to carbon dioxide in batch cultivations of *M. marburgensis*.

Figure 6: Biomass yield per mol carbon dioxide as a function of the partial pressure ratio of hydrogen to carbon dioxide in batch cultivations of *M. marburgensis*.

Figure 7: Volumetric productivity as a function of time and switch of a *M. marburgensis* culture from cell growth phase to methane production phase.

Figure 8: Biomass yield per mol carbon dioxide as a function of time and switch of a *M. marburgensis* culture from cell growth phase to methane production phase.

Figure 9: Volumetric productivity as a function of time during an equilibrium state of *M. marburgensis* in methane production phase and in continuous mode.

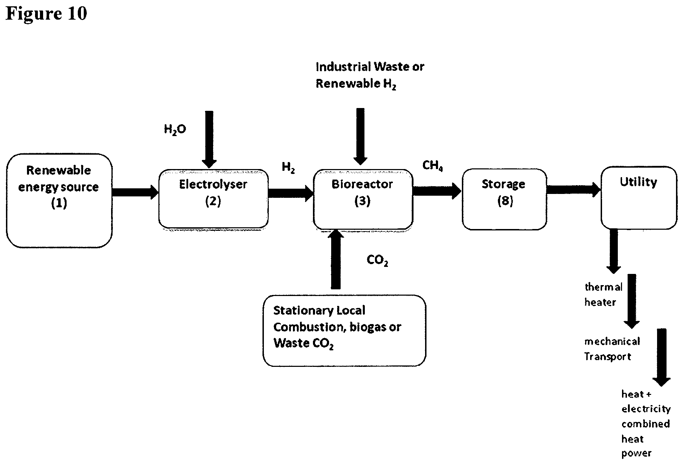

Figure 10: Exemplary set-up of the system which illustrates examples for the further use of the off-gas of the invention which is released from the bioreactor/reaction vessel. The system comprises at least one device for generating electric energy from a renewable energy source (1), at least one device for producing hydrogen and/or oxygen by the electrolysis of water and/or brine (electrolyser, 2), and at least one bioreactor (3) comprising a reaction vessel suitable for growing, fermenting and/or culturing methanogenic microorganisms for converting hydrogen and carbon dioxide into methane and at least one device for providing a gas feed of hydrogen and carbon dioxide into the reaction vessel. Optionally, the renewable energy source comprises solar energy, wind power, wave power, tidal power, water/hydro power, geothermal energy, biomass and biofuel combustion. In this particular embodiment, industrial waste or $H_2$ from a renewable source and stationary local combustion, biogas or waste $CO_2$ are used as $H_2$ and $CO_2$ for running the methane production in the bioreactor (3). Optionally, a gas storage device (8) is used between the bioreactor (3) and the end user application (utility). Examples of end-user applications may be thermal heater, mechanical transport and/or heat + electricity combined heat power. Other aspects and features are as described in Figures 1 to 4, adapted if necessary.

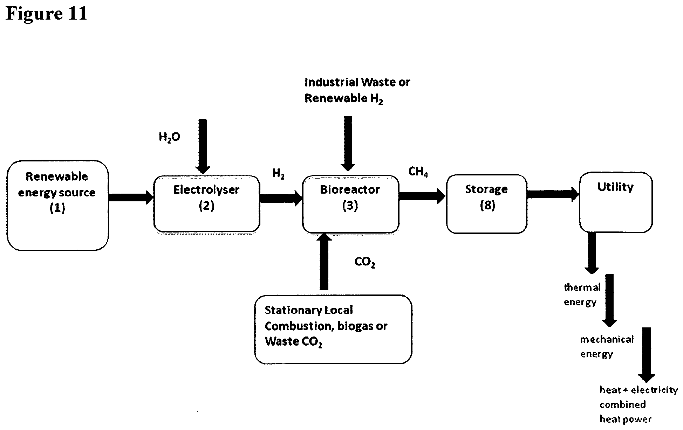

Figure 11: Exemplary set-up of the system which illustrates examples for the further use of the off-gas of the invention which is released from the bioreactor/reaction vessel. The system comprises at least one device for generating electric energy from a renewable energy source (1), at least one device for producing hydrogen and/or oxygen by the electrolysis of water and/or brine (electrolyser, 2), and at least one bioreactor (3) comprising a reaction vessel suitable for growing, fermenting and/or culturing methanogenic microorganisms for converting hydrogen and carbon dioxide into methane and at least one device for providing a gas feed of hydrogen and carbon dioxide into the reaction vessel. Optionally, the renewable energy source comprises solar energy, wind power, wave power, tidal power, water/hydro power, geothermal energy, biomass and biofuel combustion. Here, industrial waste or $H_2$ from a renewable source and stationary local combustion, biogas or waste $CO_2$ are used as $H_2$ and $CO_2$ for running the methane production in the bioreactor (3). Optionally, a gas storage device (8) is used between the bioreactor (3) and the final utility such as thermal energy, mechanical energy and/or heat + electricity combined heat power. Other aspects and features are as described in Figures 1 to 4, adapted if necessary.

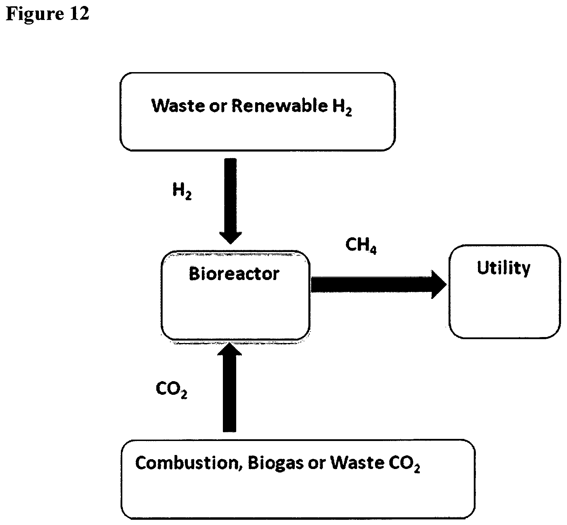

Figure 12: Bioreactor with supplied $H_2$ and $CO_2$ is shown and connection to utility devices.

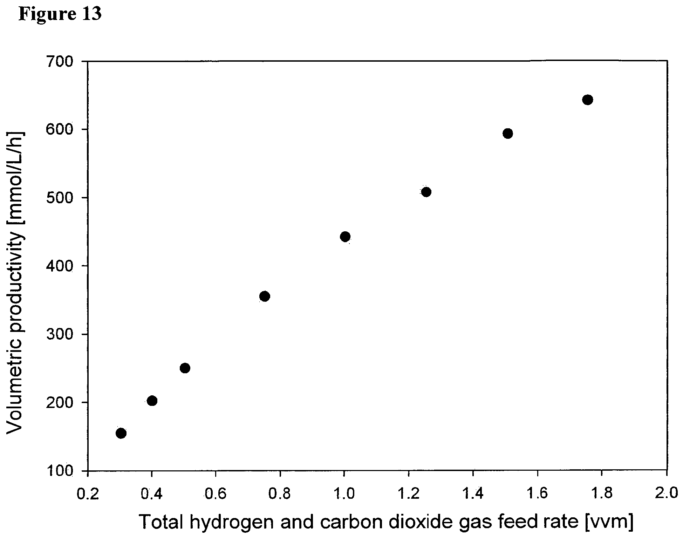

Figure 13: Volumetric productivity as a function of the hydrogen and carbon dioxide gas feed rate in total ([vvm]) based on Experiment Nos. 24 - 39 at constant pressure inside the reaction vessel.

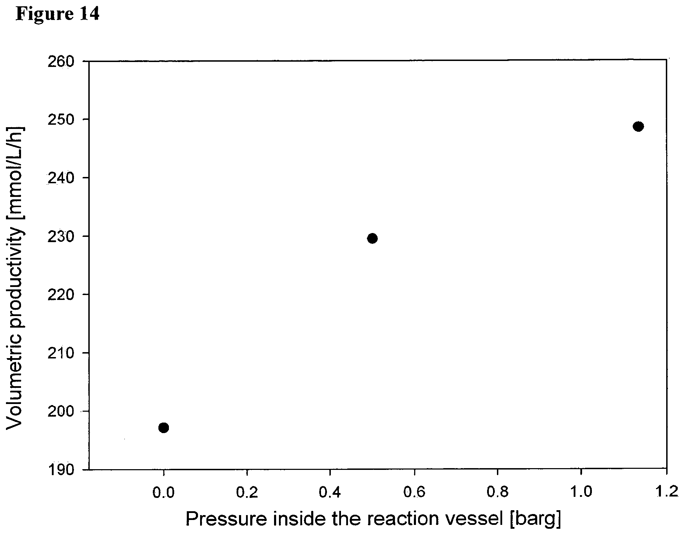

Figure 14: Volumetric productivity as a function of pressure inside the reaction vessel ([barg]) based on Experiment Nos. 25, 26 and 37 at constant hydrogen and carbon dioxide gas feed rate in total.

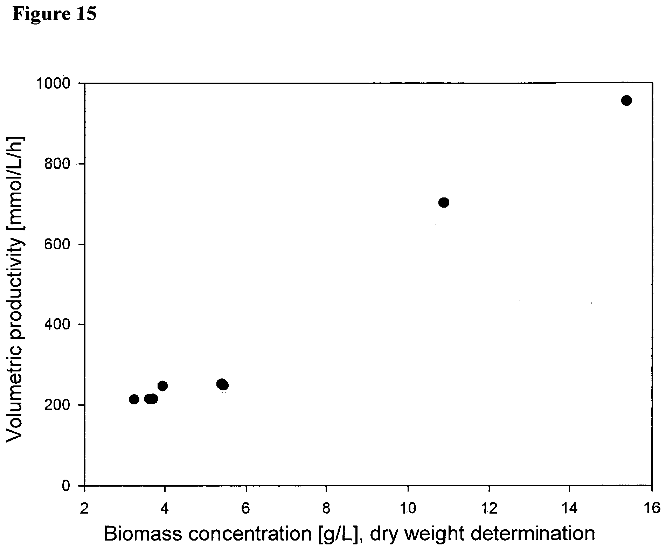

Figure 15: Volumetric productivity as a function of biomass concentration ([g/L]) determined by dry weight.

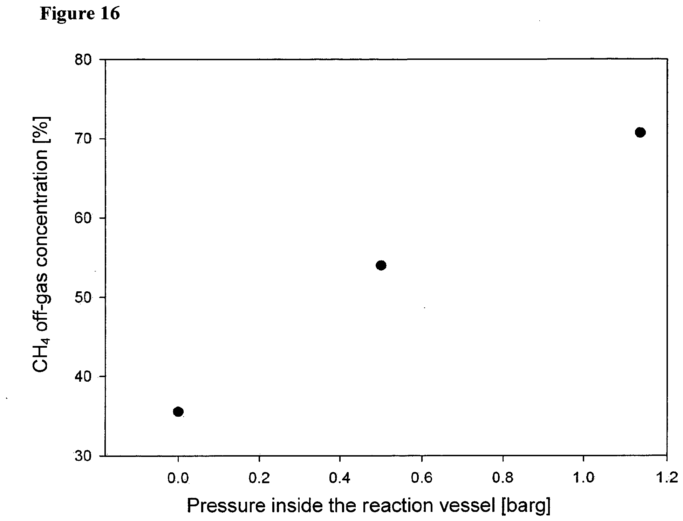

Figure 16: $CH_4$ off-gas concentration [%] as a function of pressure inside the reaction vessel ([barg]) based on Experiment Nos. 25, 26 and 37 at constant hydrogen and carbon dioxide gas feed rate in total.

## EXAMPLES

### 1. Strain and Preculture

*Methanothermobacter marburgensis* strain DSM 2133 was obtained from the Deutsche Stammsammlung für Mikroorganismen und Zellkulturen (DSMZ, Braunschweig, Germany). *M. marburgensis* was cultivated as described below and stored as a living culture at 4 °C with 0.5 bar overpressure of 80%/20% (v/v) $H_2$/$CO_2$ (Air Liquide, Paris, France) in pressure resistant bottles (Schott AG, Mainz, Germany) sealed with natural gum rubber stopper (Staint-Gobain, Charny, France) for maintenance of anaerobic conditions. Gas phase was replaced weekly by evacuating the gas phase from the bottle headspace and replacing with 0.5 bar of 80%/20% (v/v) $H_2$/$CO_2$. Strain maintenance was carried out in an anaerobic chamber flushed with formation gas (95% $N_2$, 5% $H_2$ v/v).

### 2. Cultivation Medium

The standard medium for cultivation comprised the following components ($L^{-1}$): 2.1 g $NH_4Cl$, 6.8 g $KH_2PO_4$, 3.4 g $Na_2CO_3$, 0.09 g Titriplex I, 0.04 g $MgCl_2x6H_2O$, 0.01 g $FeCl_2x4H_2O$, 0.2 mg $CoCl_2x6H_2O$, 1.2 mg $NiCl_2x6H_2O$ and 0.2 mg $NaMoO_4x2H_2O$. Prior to the transfer of the cell medium with or without cells to the reaction vessel comprised in the bioreactor, the complete bioreactor system with all devices and connected tubings etc. was made anaerobic by flushing with 80%/20% (v/v) $H_2$/$CO_2$ for five minutes.

**3. Standard Fermentation with methane production phase but without cell growth phase in 1 L scale**

Cultivation of *M. marburgensis* was performed in standard medium in a 1 L Applikon bioreactor (Applikon Biotechnology B.V., Schiedam, The Netherlands) at 65°C. For inoculation, a 50 mL suspension of a living culture of any strain *M. marburgensis* was anaerobically transferred per litre of medium into the bioreactor using anaerobic procedures as described elsewhere (Sowers, Schreier *et al.,* 1995). Anaerobic 500 mM $Na_2Sx9H_2O$ was used as sulphur source and was either pulsed into the bioreactor by syringe or fed continuously via peristaltic pump at a feed rate of 1.0 mL/h to 3 mL/h in the bioreactor. The pH value was measured via pH probe (Mettler-Toledo GmbH, Greifensee, Switzerland) and kept constant at 6.8 using 1 M $(NH_4)_2CO_3$ as base in order to compensate for acidification of the medium during growth of *M. marburgensis.* Oxidation reduction potential (ORP) was measured via redox probe (Mettler-Toledo GmbH, Greifensee, Switzerland) and kept below -350 mV. The individual gas flow of $H_2$ and $CO_2$ (Air Liquide, Paris, France) was controlled via mass flow controllers (Brooks Instrument, Matfield, USA). The total gassing rate ($H_2$ and $CO_2$) was controlled by mass flow controllers. Off-gas was detected via individually serially applied gas analyzer systems (BlueSens gas sensor GmbH, Herten, Germany) for $H_2$, $CO_2$ and $CH_4$, respectively. Biomass was determined as dry weight using standard drying procedures and gravimetric determination of preweighted glass tubes. Optical density was measured using a spectrophotometer (Hitachi U-1100, Japan) at an absorption of 578 nm. For maintainance of anaerobic conditions of the medium and of the $Na_2Sx9H_2O$ solution, a nitrogen blanket of approx. 0.1 bar was applied to each storage vessel. The in-feed rates of the gases were regulated so that the ratio of the partial pressures of hydrogen to carbon dioxide is maintained at 5:1 or higher. For this purpose, the off-gas concentration of both gases is measured in real time and the partial pressure of each gas is determined in relation to the total pressure inside the reaction vessel, i.e. in relation to the head pressure. For continuous culture mode of *M. marburgensis,* the medium feed supply was controlled gravimetrically via PID controller to maintain a constant feed rate. The volume inside the reaction vessel was held constant via a harvest pump connected to an immersion pipe at a constant reactor volume of 1.0 L. The concentration of methane in the off-gas was between 60% and 90%.

Example 3 was also performed as described above but with the process variations as shown in following Table 1:

Table 1: Process variations. [1]Anaerobic 500 mM $Na_2Sx9H_2O$, feed-rate [mL/h];

| No. | Total gas feed rate [vvm][1] | Partial pressure (H₂/CO₂) | Oxidation reduction potential (mV) | Na₂S*9H₂O feed-rate (mL/h)[1] | Dissolution rate [vvh][2] | Volumetric productivity [mmol/L*h][3] |
|---|---|---|---|---|---|---|
| 1 | 0,41 | 5,31 | -475 | 0,600 | 0,101 | 165 |
| 2 | 0,23 | 19,74 | -485 | 0,433 | 0,080 | 104 |
| 3 | 0,41 | 5,25 | -478 | 0,600 | 0,102 | 165 |
| 4 | 0,38 | 5,44 | -477 | 0,600 | 0,094 | 165 |
| 5 | 0,38 | 5,61 | -476 | 0,567 | 0,094 | 164 |
| 6 | 0,36 | 5,97 | -477 | 0,533 | 0,089 | 159 |
| 7 | 0,30 | 5,78 | -446 | 0,167 | 0,105 | 127 |
| 8 | 0,31 | 6,43 | -452 | 3,733 | 0,108 | 129 |
| 9 | 0,32 | 5,19 | -446 | 1,867 | 0,074 | 131 |
| 10 | 0,32 | 6,50 | -453 | 0,100 | 0,031 | 128 |
| 11 | 0,31 | 9,56 | -460 | 1,200 | 0,056 | 116 |
| 12 | 0,31 | 9,21 | -459 | 1,667 | 0,058 | 116 |
| 13 | 0,31 | 10,74 | -491 | 4,267 | 0,061 | 118 |
| 14 | 0,31 | 9,30 | -495 | 4,333 | 0,062 | 114 |
| 15 | 0,31 | 7,51 | -480 | 3,800 | 0,058 | 113 |
| 16 | 0,31 | 7,61 | -482 | 2,867 | 0,060 | 113 |
| 17 | 0,26 | 9,04 | -414 | 1,000 | 0,112 | 106 |
| 18 | 0,26 | 9,04 | -415 | 0,933 | 0,111 | 106 |
| 19 | 0,26 | 9,07 | -416 | 0,933 | 0,112 | 106 |

(continued)

| No. | Total gas feed rate [vvm][1] | Partial pressure (H$_2$/CO$_2$) | Oxidation reduction potential (mV) | Na$_2$S*9H$_2$O feed-rate (mL/h)[1] | Dissolution rate [vvh][2] | Volumetric productivity [mmol/L*h][3] |
|---|---|---|---|---|---|---|
| 20 | 0,31 | 9,42 | -430 | 1,233 | 0,107 | 109 |
| 21 | 0,31 | 8,48 | -433 | 1,367 | 0,111 | 109 |
| 22 | 0,31 | 5,41 | -422 | 1,100 | 0,203 | 90 |
| 23 | 0,42 | 21,09 | -501 | 0,567 | 0,091 | 154 |

[2]Dissolution rate which denotes the exchanged volume per total volume per day [vvd]; [3]Volumetric productivity [mmol/L/h]. Total gas feed rate is the total gas feed rate of hydrogen and carbon dioxide. Experiments No. 1 to 23 do have a low total gas feed rate, however, they illustrate the effect of partial pressure ratio on volumetric productivity.

The concentration of methane in the off-gas was between 40% and 90%.

## 4. Standard Fermentation with methane production phase but without cell growth phase in 10 L scale

Cultivation of *M. marburgensis* was performed in standard medium in a 10 L bioreactor type Biostat® Cplus (Sartorius Stedim Biotech GmbH, Göttingen, Germany) at 65°C. For inoculation, a 50 mL suspension of a living culture of *M. marburgensis* was anaerobically transferred per litre of medium into the bioreactor using anaerobic procedures as described elsewhere (Sowers, Schreier *et al.,* 1995). Anaerobic 500 mM Na$_2$Sx9H$_2$O was used as sulphur source and was fed continuously via peristaltic pump at a feed rate of 7.0 mL/h to 14.0 mL/h in the bioreactor.

The pH value was measured via pH probe (Mettler-Toledo GmbH, Greifensee, Switzerland) and kept constant at 6.8 using 1 M (NH$_4$)$_2$CO$_3$ as base in order to compensate for acidification of the medium during growth of *M. marburgensis.* Oxidation reduction potential (ORP) was measured via redox probe (Mettler-Toledo GmbH, Greifensee, Switzerland) and kept below -350 mV. The individual gas flow of H$_2$ and CO$_2$ (Air Liquide, Paris, France) was controlled via mass flow controllers (Brooks Instrument, Matfield, USA). The total gas rate (H$_2$ and CO$_2$) was controlled by mass flow controllers. Off-gas was detected via individual serially applied gas analyzer systems (BlueSens gas sensor GmbH, Herten, Germany) for H$_2$, CO$_2$ and CH$_4$, respectively. Biomass was determined as dry weight using standard drying procedures and gravimetric determination of preweighted glass tubes. Optical density was measured using a spectrophotometer (Hitachi U-1100, Japan) at an absorption of 578 nm. For maintainance of anaerobic conditions of the medium and of the Na$_2$Sx9H$_2$O solution, a nitrogen blanket of approx. 0.1 bar was applied to each storage vessel.

The feed rates of the in-gases were regulated so that the ratio of the partial pressures of hydrogen to carbon dioxide is maintained at 5:1 or higher. For this purpose, the off-gas concentration of both gases is measured in real time and the partial pressure of each gas is determined in relation to the total pressure inside the reaction vessel, i.e. in relation to the head pressure.

For continuous culture mode of *M. marburgensis,* the medium feed supply was controlled gravimetrically via PID controller to maintain a constant feed rate.

The volume inside the reaction vessel was held constant via a harvest pump connected to an immersion pipe. Therefore, the reactor weight was kept constant over a feedback regulation on the harvest pump. The concentration of methane in the off-gas was between 60% and 90%.

## 5. Standard Fermentation with methane production phase and with cell growth phase in 1 L scale

Cultivation of *M. marburgensis* was performed in standard medium in a 1 L Applikon bioreactor (Applikon Biotechnology B.V., Schiedam, The Netherlands) at 65°C. For inoculation, a 50 mL suspension of a living culture of any strain, for example of *M. marburgensis,* was anaerobically transferred per litre of medium into the bioreactor using anaerobic procedures as described elsewhere (Sowers, Schreier *et al.,* 1995). Anaerobic 500 mM Na$_2$Sx9H$_2$O was used as sulphur source and was either pulsed into the bioreactor by syringe or fed continuously via peristaltic pump at a feed rate of 1.0 mL/h to 3 mL/h in the bioreactor. The pH value was measured via pH probe (Mettler-Toledo GmbH, Greifensee, Switzerland) and kept constant at 6.8 using 1 M (NH$_4$)$_2$CO$_3$ as base in order to compensate for acidification of the medium during growth of *M. marburgensis.* Oxidation reduction potential (ORP) was measured via redox probe (Mettler-Toledo GmbH, Greifensee, Switzerland). The individual gas flow of H$_2$ and CO$_2$ (Air Liquide, Paris, France) was controlled via mass flow controllers (Brooks Instrument, Matfield, USA). The total gassing rate (H$_2$ and CO$_2$) was controlled by mass flow controllers. The feed rates of the in-gases hydrogen and carbon dioxide were regulated depending on the determined partial pressure ratios, Initially, during the cell growth phase, the partial pressure ratio of hydrogen to carbon dioxide was set to be maintained in the range from 0.5:1 to 4:1 (parts hydrogen : parts carbon dioxide). Within this range from 0.5:1 to 4:1, the partial pressure ratio may fluctuate. In the cell growth phase, the fermentation, and therefore also the settings of the bioreactor, was started in batch cultivation mode with an ORP value below -300 mV. In the methane production phase, the partial pressure ratio of hydrogen to carbon dioxide was set to be maintained at 5:1 or higher. For the methane production phase, the continuous culture mode was used for long term stable operation with an ORP value below -350 mV. For a continuous culture of *M. marburgensis,* the medium feed supply was controlled gravimetrically via PID controller to maintain a constant feed rate. The volume inside the reaction vessel was held constant via a harvest pump connected to an immersion pipe at a constant cell culture volume of 1.0 L. During the whole fermentation procedure, off-gas was detected via individually serially applied gas analyzer systems (BlueSens gas sensor GmbH, Herten, Germany) for $H_2$, $CO_2$ and $CH_4$, respectively. Biomass was determined as dry weight using standard drying procedures and gravimetric determination of preweighted glass tubes. Optical density was measured using a spectrophotometer (Hitachi U-1100, Japan) at an absorption of 578 nm. For maintainance of anaerobic conditions of the medium and of the $Na_2Sx9H_2O$ solution, a nitrogen blanket of approx. 0.1 bar was applied to each storage vessel. The concentration of methane in the off-gas was between 60% and 90%.

## 6. Standard Fermentation with methane production phase and with cell growth phase in 10 L scale

Cultivation of *M. marburgensis* was performed in standard medium in a 10 L bioreactor type Biostat® Cplus (Sartorius Stedim Biotech GmbH, Göttingen, Germany) at 65°C. For inoculation, a 50 mL suspension of a living culture of any strain, for example of *M. marburgensis,* was anaerobically transferred per litre of medium into the bioreactor using anaerobic procedures as described elsewhere (Sowers, Schreier *et al.,* 1995). Anaerobic 500 mM $Na_2Sx9H_2O$ was used as sulphur source and was fed continuously via peristaltic pump at a feed rate of 7.0 mL/h to 14.0 mL/h in the reaction vessel of the bioreactor. The pH value was measured via pH probe (Mettler-Toledo GmbH, Greifensee, Switzerland) and kept constant at 6.8using 1 M $(NH_4)_2CO_3$ as base in order to compensate for acidification of the medium during growth of *M. marburgensis.* Oxidation reduction potential (ORP) was measured via redox probe (Mettler-Toledo GmbH, Greifensee, Switzerland). The individual gas flow of $H_2$ and $CO_2$ (Air Liquide, Paris, France) was controlled via mass flow controllers (Brooks Instrument, Matfield, USA). Off-gas was detected via individually serially applied gas analyzer systems (BlueSens gas sensor GmbH, Herten, Germany) for $H_2$, $CO_2$ and $CH_4$, respectively. The total gassing rate ($H_2$ and $CO_2$) was controlled by mass flow controllers. The feed rates of the in-gases hydrogen and carbon dioxide were regulated depending on the determined partial pressure ratio of hydrogen to carbon dioxide. In the initial cell growth phase, the partial pressure ratio of hydrogen to carbon dioxide was maintained in the range from 0.5:1 to 4:1 (parts hydrogen : parts carbon dioxide). Within this range from 0.5:1 to 4:1, the partial pressure ratio may fluctuate. In the cell growth phase, the fermentation, and therefore also the settings of the bioreactor, was started in batch cultivation mode with an ORP value below -300 mV. In a subsequent methane production phase, the partial pressure ratio of hydrogen to carbon dioxide was set to be maintained at a value of 5:1 or higher (parts hydrogen : parts carbon dioxide) and the fermentation mode was switched to continuous culture mode which was used for long term stable operation. The ORP value was kept below -350 mV. For a continuous culture of *M. marburgensis,* the medium feed supply was controlled gravimetrically via PID controller to maintain a constant feed rate. Biomass was determined as dry weight using standard drying procedures and gravimetric determination of preweighted glass tubes. Optical density was measured using a spectrophotometer (Hitachi U-1100, Japan) at absorption of 578 nm. For maintainance of anaerobic conditions of the medium and of the $Na_2Sx9H_2O$ solution, a nitrogen blanket of approx. 0.1 bar was applied to each storage vessel. The volume inside the reaction vessel was held constant via a harvest pump connected to an immersion pipe. Therefore, the reactor weight was kept constant over a feedback regulation on the harvest pump. The concentration of methane in the off-gas was between 60% and 90%.

## 7. Fermentation conditions depending on the species of the methanogenic microorganism

The above described fermentation conditions are principally the same for all methanogenic microorganisms of the invention, in particular for methanogenic archaea of the species *Methanosarcinia barkeri, Methanothermobacter marburgensis,* such as for example DSM 2133 (DSMZ, Braunschweig, Germany), *Methanobacterium thermoautotrophicus, Methanocaldococcus jannaschii, Methanothermobacter thermoautotrophicus, Methanococcus maripaludis,* or mixtures thereof. Table 2 summarizes fermentation conditions which have to be adjusted according to the species of the methanogenic microorganism used in the particular experiment. Further information on general fermentation conditions can be obtained from DSMZ, Braunschweig, Germany.

Table 2: Species dependent fermentation parameters as they can also be obtained from DSMZ, Braunschweig, Germany.

| Strain species | Range | | Preferred value | |
|---|---|---|---|---|
| | T [°C] | pH | T [°C] | pH |
| *Methanothermobacter marburgensis* | 45 - 70 | 6.7 - 7.0 | 65 | 6.8 - 7.0 |
| *Methanothermobacter thermautotrophicus, Methanobacterium thermoautotrophicus* | 63 - 68 | 6.7 - 8.0 | 65 | 6.8 |
| *Methanosarcina barkeri* | 35 - 37 | 6.5 - 6.8 | 36 | 6.7 |
| *Methanobrevibacter arboriphilicus* | 35 - 38 | 6.7 - 7.0 | 37 | 6.8 |
| *Methanocaldococcus jannaschii* | 80 - 87 | 5.0 - 6.3 | 85 | 6 |

**8. Volumetric productivity of methane as a function of partial pressure ratio of hydrogen to carbon dioxide**

Three 5 L batch cultivations of *M. marburgensis* with different partial pressure ratios of $H_2/CO_2$ were performed. The partial pressure ratio $H_2/CO_2$ was varied for each experiment by adjusting the in-gas feeds according to the partial pressure determined by off-gas concentrations and head pressure measurement.

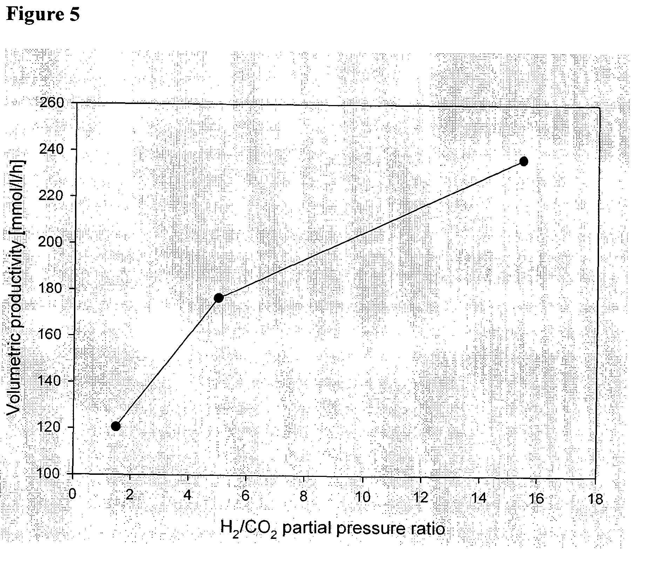

The volumetric productivity of the methanogenic microorganisms at the end of the exponential phase was taken as indicator for the cell's performance depending on the $H_2/CO_2$ partial pressure ratios during fermentation. The results as presented in Figure 5 show, that the higher the partial pressure ratio of $H_2/CO_2$ the higher the volumetric productivity of methane.

**9. Biomass yield per mol carbon dioxide as a function of the partial pressure ratio of hydrogen to carbon dioxide**

Three 5 L batch cultivations of *M. marburgensis* with different partial pressure ratios of $H_2/CO_2$ were performed. The partial pressure ratios $H_2/CO_2$ were varied for each experiment by adjusting the in-gas feeds according to the partial pressure determined by off-gas concentrations and head pressure measurement.

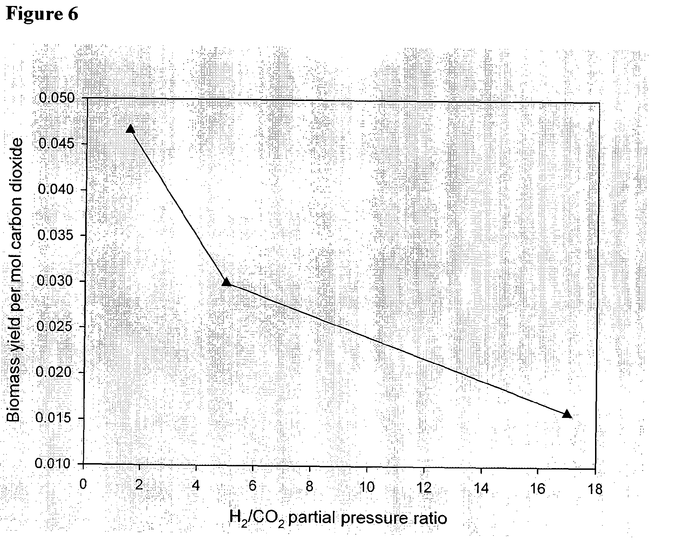

The biomass yield per mol carbon dioxide of the culture of methanogenic microorganisms was determined during the exponential phase. "Exponential phase" or "exponential cell growth phase" in the sense of the invention denotes the phase of exponential cell growth which is not to be mixed up with the cell growth phase of the invention characterized by a specific $H_2/CO_2$ partial pressure ratio. Several experiments with different $H_2/CO_2$ partial pressure ratios were performed. The results presented in Figure 6 show the lower the partial pressure ratio of $H_2/CO_2$ the higher the biomass yield per mol carbon dioxide.

**10. Volumetric productivity of methane as a function of time and switch from cell growth phase to methane production phase**

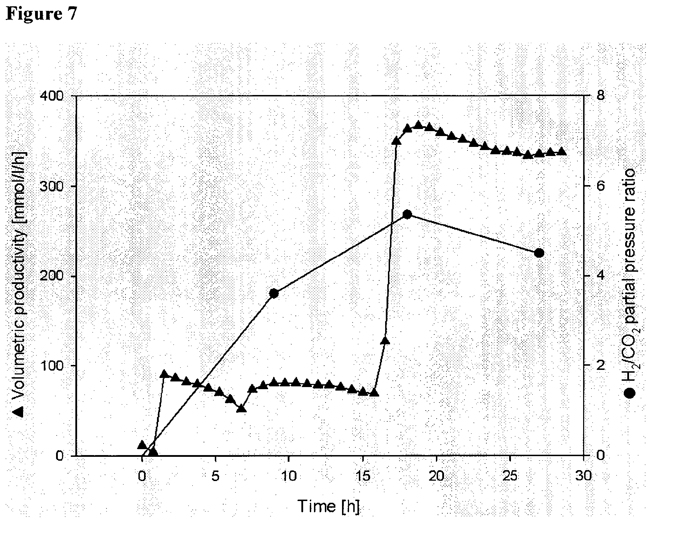

A 3 L batch culture of *M. marburgensis* with an initial partial pressure ratio of $H_2/CO_2$ of 2:1 was performed. After about 15 h, the culture was switched to continuous cultivation mode, with a constant feed rate of a dilution rate (which is the reciprocal of the residence time of the medium in the bioreactor and is derived by the feed rate divided by the working/culture volume of the reaction vessel inside the bioreactor) of 0.05 L medium/h. At the same time, the partial pressure ratio of $H_2/CO_2$ was switched from a ratio of $2\pm1$:1 to a ratio above 5:1 (parts hydrogen : parts carbon dioxide), by adjusting the in-gas feeds of both gases according to the partial pressure determined by off-gas concentrations and head pressure measurement as described above. Figure 7 shows the trajectory of the volumetric productivity over time. Although shortly a high methane production can be observed prior to the shift to higher partial pressure ratios at the end of the cell growth phase, however, advantageous sustained and continuous high methane production can only be achieved by switching to a higher partial pressure ratio, i.e. to a $H_2/CO_2$ partial pressure ratio of 5:1 or higher.

**11. Biomass yield per mol carbon dioxide during switch from cell growth phase to methane production phase**

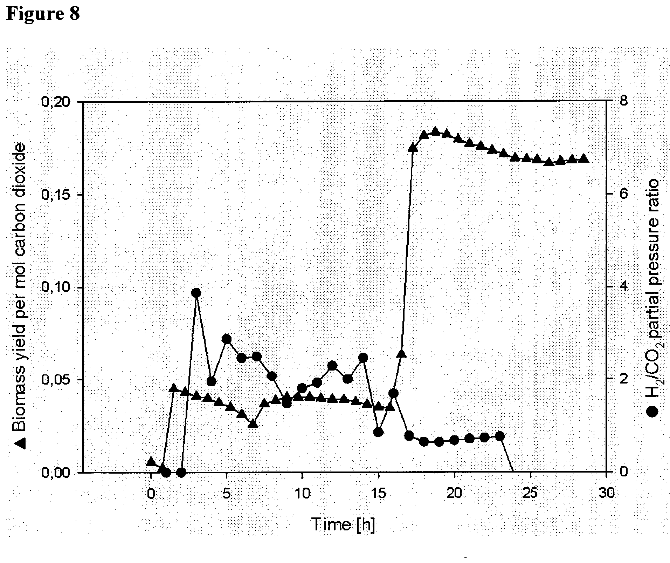

A 3 L batch culture of *M. marburgensis* with an initial partial pressure ratio of $H_2/CO_2$ of $2\pm1$:1 (parts hydrogen : parts carbon dioxide) was fermented in batch cultivation mode. After about 15 h, the culture was switched to continuous cultivation mode, with a constant feed rate of a dilution rate of 0.05 L medium/h. At the same time, the partial pressure ratio of $H_2/CO_2$ was switched from a ratio of $2\pm1$:1 to a ratio above 5:1 (parts hydrogen : parts carbon dioxide), by adjusting the in-gas feeds of both gases according to the partial pressure determined by off-gas concentrations and head pressure measurement as described above. Samples were taken every hour in order to determine biomass concentration. The results presented in Figure 8 demonstrate, that the biomass yield per mol carbon dioxide with a partial pressure ratio of $H_2/CO_2$ of $2\pm1$:1 (parts hydrogen : parts carbon dioxide) was significantly higher than with a partial pressure ratio above 5:1 (parts hydrogen : parts carbon dioxide).

## 12. Process stability

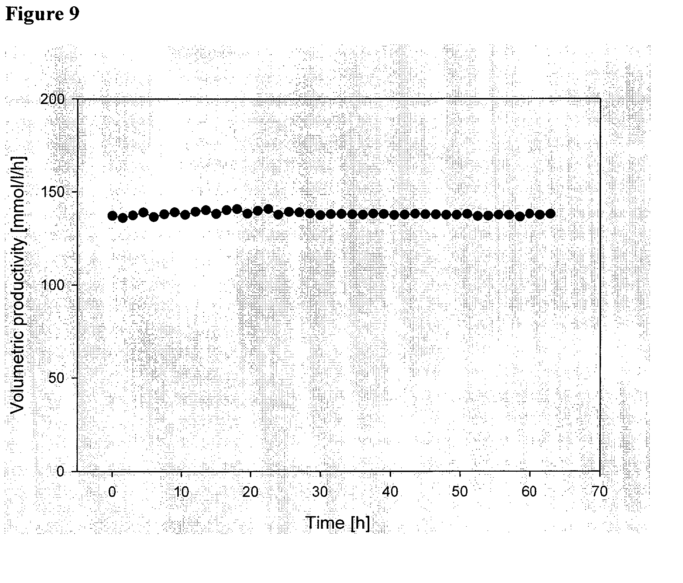

A cell culture of *M. marburgensis* in continuous mode with a constant feed rate of the medium of a dilution rate of 0.05 L/h was set up. Different partial pressure ratios of $H_2/CO_2$ were adjusted by varying the in-gas feed of $H_2/CO_2$ according to the partial pressure determined by off-gas concentrations and head pressure measurement. Figure 9 presents the volumetric productivity trajectory in continuous mode. The partial pressure ratio of $H_2/CO_2$ in the off-gas was maintained around $18\pm1$:1 (parts hydrogen : parts carbon dioxide). All cultures showed robust performance over several days and weeks. In the concrete example shown in Figure 9, the methanogenic microorganisms showed stable performance over 42 days. Around 130 mmol to 145 mmol of methane were stably produced per litre cell culture volume per hour over the whole period of time.

## 13. Volumetric productivity depending on gas feed rate, general experimental set-up without differentiation of methane production phase or cell growth phase

All experiments were carried out with *Methanothermobacter marburgensis* (*M. marburgensis*) strain DSM 2133 in a 10 L Biostat® C+ laboratory reactor (Sartorius Stedim biotech AG, Göttingen,Germany). *M. marburgensis* cultures were stored at 4 °C and 0.5 barg of a $H_2/CO_2$ atmosphere in pressure resistant bottles, sealed with a rubber gum stop. The gas phase was exchanged once a week by releasing the overpressure in the bottle and refilling the bottle with $H_2/CO_2$ to a pressure of 0.5 barg (i.e. about 1.5 bar$\pm$0.05 bar).

For fermentation, a slightly adapted medium according to Schoenheit *et al.* (1980) was used, containing the following ingredients per litre: 2.1 g $NH_4Cl$, 6.8 g $KH_2PO_4$, 3.6 g $Na_2CO_3$, 0.09 g Titriplex I, 0.04 g $MgCl_2 \cdot 6H_2O$, 0.01 g $FeCl_2 \cdot 4H_2O$, 0.2 mg $CoCl_2 \cdot 6H_2O$, 1.2 mg $NiCl_2 \cdot 6H_2O$, 0.2 mg $NaMoO_4 \cdot 2H_2O$.

The same medium was used as feed medium for continuous cultivation mode with the addition of 100 µl/L antifoam agent.

To ensure anaerobic conditions inside the reaction vessel, the whole system was flushed with an $H_2/CO_2$ mixture for several minutes prior to inoculation. For inoculation, 50 mL of the *M. marburgensis* stock suspension per litre of medium were transferred anaerobically to the reactor using an anaerobic gas tight syringe. Cultivation was performed at 65 °C and a stirrer speed of 1500 rpm. The pH was measured by pH probe (Mettler Toledo GmbH, Vienna, Austria; and Hamilton Bonaduz AG, Bonaduz, Switzerland) and kept constant at a pH value of 7.00$\pm$1 by addition of a 1 M $(NH_4)_2CO_3$ solution in order to compensate acidification during microbial growth.

The oxidation reduction potential (ORP) was measured by a redox probe (Mettler Toledo GmbH, Vienna, Austria) and always kept below -350mV. A 0.5 M $Na_2S*9H_2O$ solution was used as sulphur source and fed constantly to the reaction vessel at rates between 4 mL/h and 15 mL/h. The addition of base and $Na_2S$ solution was recorded gravimetrically. The feed medium was applied using an analogue peristaltic pump (Preciflow, Lambda Laboratory Instruments, Zurich, Switzerland). The feed rate was recorded gravimetrically and kept constant by controlling the pump speed. The bioreactor vessel volume was kept constant by withdrawing culture suspension over an immersion pipe using a peristaltic pump controlled on a fixed reactor weight. The withdrawn suspension was collected in a harvest bottle and its volume recorded gravimetrically. All solutions were made anaerobic by flushing with $N_2$ or $H_2/CO_2$. To maintain anaerobic conditions, all bottles were pressurised with 1 bar $N_2$. Pure $H_2$ and $CO_2$ were used as substrates for *M. marburgensis.* Gas feeds were adjusted individually via thermal mass flow controllers (Brooks Instrument, Hatfield, USA). The ratio between $H_2$ and $CO_2$ in the in-gas was kept constant at 4:1.

Off-gas concentrations of $CH_4$, $CO_2$ and $H_2$ were measured online using IR ($CH_4$, $CO_2$) and thermal sensors ($H_2$), respectively (BlueSens gas sensor GmbH, Herten, Germany) and were value corrected according to the manufacturer's instructions. Biomass dry weight was determined in quadruplicates by transferring 10 mL of the culture broth into a pre-weighed glass tube. The sample was centrifuged for 15 min at 4000 rpm (Sigma Signum 4K15 centrifuge, rotor 11156, SIGMA Laborzentrifugen GmbH, Osterode am Harz) and the supernatant was discarded. The pellet was then washed in 5 mL of deionised water and centrifuged again at 5000 rpm for 10 min. The supernatant was discarded and the pellet dried at 110 °C for 3 days. The weight of the dried biomass was then determined gravimetrically. All fermentation parameters, feed rates and setpoints were controlled and recorded using the process information management system (PIMS) Lucullus (SecureCell AG, Schlieren, Switzerland). In line with above described general experimental set-up, the following experiments were performed. Variations in process parameter and resulting % $CH_4$ in the off-gas and methane evolution rate (MER) are summarised in Tables 3 to 5:

Table 3: Process variations I. Here, a bioreactor comprising a 10 L reaction vessel (reaction medium volume ~5 L) and three rotor blades was used. Total in-gas feed rate is the total gas feed rate of hydrogen and carbon dioxide. The pressure inside the reaction vessel is given in barg, thus, the absolute pressure which was applied inside the reaction vessel during the experiments can be calculated as follows: pressure [barg] + $1\pm0.05$ bar = pressure [bar absolute]. $1\pm0.05$ bar is used as normal atmospheric pressure, however, may need adaptation depending on the location of the operator of the method.

| No. | Total in-gas feed rate [vvm] | Partial pressure ratio $H_2/CO_2$ | Pressure [barg] | % $CH_4$ in off-gas | Volumetric productivity [mmol/L/h] |
|---|---|---|---|---|---|
| 24 | 0.25 | 5.89 | 0.00 | 54.7 | 115 |
| 25 | 0.50 | 4.47 | 0.00 | 35.5 | 197 |
| 26 | 0.50 | 4.87 | 0.50 | 54.0 | 229 |
| 27 | 0.51 | 8.24 | 1.00 | 61.3 | 243 |
| 28 | 0.75 | 6.25 | 1.00 | 54.2 | 345 |
| 29 | 0.75 | 6.66 | 1.25 | 59.2 | 355 |
| 30 | 1.00 | 5.93 | 1.25 | 48.1 | 442 |
| 31 | 1.26 | 5.10 | 1.25 | 38.1 | 507 |
| 32 | 1.26 | 5.23 | 1.50 | 40.1 | 518 |
| 33 | 0.50 | 4.85 | 1.00 | 67.5 | 245 |
| 34 | 1.51 | 5.00 | 1.25 | 35.5 | 593 |
| 35 | 1.76 | 4.80 | 1.25 | 30.0 | 642 |
| 36 | 0.50 | 4.22 | 1.23 | 71.2 | 250 |
| 37 | 0.50 | 4.54 | 1.14 | 70.7 | 248 |
| 38 | 0.40 | 4.76 | 1.25 | 76.0 | 202 |
| 39 | 0.30 | 14.12 | 1.25 | 79.6 | 154 |

In Experiments Nos. 24 to 39, the trace elements in the fermentation medium were twice as concentrated as in standard fermentation medium as mentioned above, i.e. contained the following ingredients per litre: 2.1 g $NH_4Cl$, 6.8 g $KH_2PO_4$, 3.6 g $Na_2CO_3$, 0.09 g Titriplex I, 0.08 g $MgCl_2 \cdot 6H_2O$, 0.02 g $FeCl_2 \cdot 4H_2O$, 0.4 mg $CoCl_2 \cdot 6H_2O$, 2.4 mg $NiCl_2 \cdot 6H_2O$, 0.4 mg $NaMoO_4 \cdot 2H_2O$.

Table 4: Process variations II. Here, a bioreactor comprising a 10 L reaction vessel (reaction medium volume ~3.5 L) and one rotor blades was used. Total gas feed rate is the total gas feed rate of hydrogen and carbon dioxide. The pressure inside the reaction vessel is given in barg, thus, the absolute pressure which was applied inside the reaction vessel during the experiments can be calculated as follows: pressure [barg] + $1\pm0.05$ bar = pressure [bar absolute]. $1\pm0.05$ bar is used as normal atmospheric pressure, however, may need adaptation depending on the location of the operator of the method.

| No. | Total gas feed rate [vvm] | Partial pressure ratio $H_2/CO_2$ | Pressure [barg] | % $CH_4$ in off-gas | Volumetric productivity [mmol/L/h] |
|---|---|---|---|---|---|
| 40 | 0.29 | 7.49 | 0.06 | 56.3 | 135 |
| 41 | 0.29 | 14.73 | 0.52 | 63.7 | 140 |
| 42 | 0.29 | 17.55 | 0.75 | 64.2 | 140 |
| 43 | 0.43 | 6.39 | 0.75 | 57.6 | 203 |
| 44 | 0.43 | 5.63 | 0.49 | 53.5 | 198 |
| 45 | 0.43 | 5.07 | 0.25 | 47.2 | 189 |
| 46 | 0.43 | 4.60 | 0.00 | 37.4 | 173 |

(continued)

| No. | Total gas feed rate [vvm] | Partial pressure ratio H$_2$/CO$_2$ | Pressure [barg] | % CH$_4$ in off-gas | Volumetric productivity [mmol/L/h] |
|---|---|---|---|---|---|
| 47 | 0.72 | 4.17 | 0.00 | 18.7 | 206 |
| 48 | 0.72 | 4.40 | 0.25 | 21.1 | 221 |
| 49 | 0.73 | 4.46 | 0.50 | 22.0 | 228 |
| 50 | 0.73 | 4.48 | 0.75 | 22.1 | 229 |

The trace elements in the fermentation medium in Experiments Nos. 40 to 50 were twice as concentrated as in standard fermentation medium as mentioned above, i.e. contained the following ingredients per litre: 2.1 g NH$_4$Cl, 6.8 g KH$_2$PO$_4$, 3.6 g Na$_2$CO$_3$, 0.09 g Titriplex I, 0.08 g MgCl$_2$•6H$_2$O, 0.02 g FeCl$_2$•4H$_2$O, 0.4 mg CoCl$_2$•6H$_2$O, 2.4 mg NiCl$_2$•6H$_2$O, 0.4 mg NaMoO$_4$•2H$_2$O.In summary, one can see that the volumetric productivity is higher when a higher gas feed rate is applied.

Table 5: Process variations III. Here, a bioreactor comprising a 10 L reaction vessel (reaction medium volume ~5L) and three rotor blades was used. Total gas feed rate is the total gas feed rate of hydrogen and carbon dioxide. The pressure inside the reaction vessel is given in barg, thus, the absolute pressure which was applied inside the reaction vessel during the experiments can be calculated as follows: pressure [barg] + 1±0.05 bar = pressure [bar absolute]. 1 ±0.05 bar is used as normal atmospheric pressure, however, may need adaptation depending on the location of the operator of the method.

| No. | Total gas feed rate [vvm] | Partial pressure ratio H$_2$/CO$_2$ | Pressure [barg] | % CH$_4$ in off-gas | Volumetric productivity [mmol/L/h] |
|---|---|---|---|---|---|
| 51 | 0.50 | 4.67 | 0.00 | 43.6 | 213 |
| 52 | 0.50 | 4.16 | 0.00 | 43.9 | 214 |
| 53 | 0.50 | 4.25 | 0.00 | 44.4 | 214 |
| 54 | 0.50 | 4.57 | 1.00 | 69.0 | 246 |
| 55 | 0.50 | 4.32 | 1.00 | 70.4 | 248 |
| 56 | 0.50 | 4.75 | 1.50 | 76.3 | 251 |
| 57 | 1.51 | 4.00 | 1.00 | 57.4 | 703 |
| 58 | 2.01 | 6.36 | 1.75 | 60.2 | 950 |

In Experiment Nos. 51 to 56 a 0.5 M Na$_2$S*9H$_2$O solution was used at a feed rate of 2 g/h, and the trace elements in the fermentation medium were twice as concentrated as in standard fermentation medium as mentioned above, i.e. contained the following ingredients per litre: 2.1 g NH$_4$Cl, 6.8 g KH$_2$PO$_4$, 3.6 g Na$_2$CO$_3$, 0.09 g Titriplex I, 0.08 g MgCl$_2$•6H$_2$O, 0.02 g FeCl$_2$•4H$_2$O, 0.4 mg CoCl$_2$•6H$_2$O, 2.4 mg NiCl$_2$•6H$_2$O, 0.4 mg NaMoO$_4$•2H$_2$O. In Experiment No. 57, a 0.5 M Na$_2$S*9H$_2$O solution was used at a feed rate of 10 g/h, and the trace elements in the fermentation medium were 8 times as concentrated as in standard fermentation medium as mentioned above, i.e. contained the following ingredients per litre: 2.1 g NH$_4$Cl, 6.8 g KH$_2$PO$_4$, 3.6 g Na$_2$CO$_3$, 0.09 g Titriplex I, 0.32 g MgCl$_2$•6H$_2$O, 0.08 g FeCl$_2$•4H$_2$O, 1.6 mg CoCl$_2$•6H$_2$O, 9.6 mg NiCl$_2$•6H$_2$O, 1.6 mg NaMoO$_4$•2H$_2$O. In Experiment No. 58, a 0.5 M Na$_2$S*9H$_2$O solution was used at a feed rate of 15 g/h, and the trace elements in the fermentation medium were 20 times as concentrated as in standard fermentation medium as mentioned above, i.e. contained the following ingredients per litre: 2.1 g NH$_4$Cl, 6.8 g KH$_2$PO$_4$, 3.6 g Na$_2$CO$_3$, 0.09 g Titriplex I, 0.8 g MgCl$_2$•6H$_2$O, 0.2 g FeCl$_2$•4H$_2$O, 4 mg CoCl$_2$•6H$_2$O, 24 mg NiCl$_2$•6H$_2$O, 4 mg NaMoO$_4$•2H$_2$O.

## Claims

1. A method for producing methane comprising contacting methanogenic microorganisms in a reaction vessel with hydrogen and carbon dioxide, wherein the gas feed rate of hydrogen and carbon dioxide into the reaction vessel is at least 1.2 vvm in total, and wherein inside the reaction vessel an absolute pressure of at least 5.0 bar (4.0 barg) is applied.

2. The method according to claim 1, wherein the method comprises one or more of the following steps:

   a) generating electric energy using a renewable energy source; and/or
   b) using partially or completely said electric energy for the electrolysis of water and/or brine, thereby producing hydrogen and/or oxygen; and/or
   c) using partially or completely said hydrogen and carbon dioxide, and optionally partially or completely said electric energy for producing methane.

3. The method according to claim 2, wherein the renewable energy source comprises solar energy, wind power, wave power, tidal power, water or hydro power, geothermal energy, biomass and biofuel combustion, or combinations thereof.

4. The method according to claim 2 or 3, further comprising step d), wherein partially or completely the oxygen is used for producing partially or completely the carbon dioxide by oxygen enriched combustion or gasification.

5. The method according to any one of the preceding claims, wherein the methanogenic microorganisms are methanogenic archaea selected from the group consisting of *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arbor iphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus* and *Methanopyrus kandleri,* and mixtures thereof.

6. The method according to any one of the preceding claims, wherein the gas feed rate of hydrogen and carbon dioxide into the reaction vessel is at least 2.0 vvm in total or is in the range of 1.2 vvm to 25 vvm in total.

7. The method according to any one of the preceding claims, wherein the gas feed rate of hydrogen and carbon dioxide into the reaction vessel is in the range of 2.0 vvm to 8.0 vvm in total and the pressure inside the reaction vessel is adjusted to a value in the range of 5 bar to 1000 bar.

8. The method according to any one of claims 1 to 6, wherein the gas feed rate of hydrogen and carbon dioxide into the reaction vessel is at least 20 vvm in total.

9. The method according to any one of the preceding claims, further comprising at least one methane production phase, wherein in the at least one methane production phase the ratio of the partial pressure of hydrogen to the partial pressure of carbon dioxide inside the reaction vessel is maintained at 5:1 or higher (parts hydrogen : parts carbon dioxide).

10. The method according to any one of the preceding claims, wherein the volumetric ratio of hydrogen to carbon dioxide in the gas feed introduced into the reaction vessel is 4:1.

11. The method according to any one of the preceding claims, wherein the method is carried out using a system comprising

   at least one device for generating electric energy from a renewable and/or non-renewable energy source,
   at least one device for producing hydrogen and/or oxygen by the electrolysis of water and/or brine, and
   at least one bioreactor comprising a reaction vessel suitable for growing, fermenting and/or culturing methanogenic microorganisms and at least one device for measuring and/or controlling a gas feed of hydrogen and carbon dioxide into the reaction vessel,
wherein the at least one bioreactor further comprises a device for controlling and/or measuring an absolute pressure inside the reaction vessel of at least 5 bar.

12. The method according to claim 11, wherein the system further comprises at least one device for oxygen enriched combustion or gasification for producing carbon dioxide, said carbon dioxide being transferred to the bioreactor, and/or further comprising at least one device for recovery, purification, enriching, storing, recycling and/or further processing of off-gas, hydrogen, water, oxygen, chlorine, carbon dioxide, $H_2S$, sodium sulphite, methanogenic microorganisms, used medium and medium components, methane and other substances of the process from the reaction vessel and bioreactor.

13. The method according to claim 12, wherein the device for oxygen enriched combustion or gasification uses the oxygen produced by the electrolysis of water.

14. The method according to any one of claims 11 to 13, wherein the renewable energy source comprises solar energy, wind power, wave power, tidal power, water/hydro power, geothermal energy, biomass and/or biofuel combustion.

15. The method according to any one of claims 11 to 14, wherein the methanogenic microorganisms are methanogenic archaea.

## Patentansprüche

1. Ein Verfahren zur Herstellung von Methan, umfassend das Inkontaktbringen von methanogenen Mikroorganismen in ein mit Wasserstoff und Kohlendioxid, wobei die Gaszufuhrrate von Wasserstoff und Kohlendioxid in das Reaktionsgefäß mindestens 1,2 vvm insgesamt ist, und wobei im Reaktionsgefäß ein Absolutdruck von mindestens 5,0 bar (4,0 barg) angelegt ist.

2. Das Verfahren nach Anspruch 1, wobei das Verfahren einen oder mehrere der folgenden Schritte umfasst:

   a) Erzeugen von elektrischer Energie unter Verwendung einer erneuerbaren Energiequelle; und/oder
   b) teilweises oder vollständiges Verwenden der elektrischen Energie für die Elektrolyse von Wasser und/oder Salzlake, wodurch Wasserstoff und/oder Sauerstoff erzeugt werden; und/oder
   c) teilweises oder vollständiges Nutzen des Wasserstoffs und Kohlendioxids, und optional teilweises oder vollständiges Nutzen der elektrischen Energie zur Erzeugung von Methan.

3. Das Verfahren nach Anspruch 2, wobei die erneuerbare Energiequelle Sonnenenergie, Windkraft, Wellenkraft, Gezeitenkraft, Wasser- oder Hydrokraft, Erdwärme, Biomasse- und/oder Biokraftstoff- Verbrennung, oder Kombinationen davon umfasst.

4. Das Verfahren nach Anspruch 2 oder 3, das ferner Schritt d) umfasst, wobei der Sauerstoff teilweise oder vollständig zur teilweisen oder vollständigen Erzeugung von Kohlendioxid durch sauerstoffangereicherte Verbrennung oder Vergasung verwendet wird.

5. Das Verfahren nach einem der vorangehenden Ansprüche, wobei die methanogenen Mikroorganismen methanogene Archaeen sind, ausgewählt aus der Gruppe bestehend aus *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arbor iphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus* und *Methanopyrus kandleri,* sowie Gemische davon.

6. Das Verfahren nach einem der vorangehenden Ansprüche, wobei die Gaszufuhrrate von Wasserstoff und Kohlendioxid in das Reaktionsgefäß mindestens 2,0 vvm insgesamt ist oder im Bereich von 1,2 vvm bis 25 vvm insgesamt ist.

7. Das Verfahren nach einem der vorangehenden Ansprüche, wobei die Gaszufuhrrate von Wasserstoff und Kohlendioxid in das Reaktionsgefäß im Bereich von 2,0 vvm bis 8,0 vvm insgesamt ist und der Druck im Reaktionsgefäß auf einen Wert im Bereich von 5 bar bis 1000 bar eingestellt ist.

8. Das Verfahren nach einem der Ansprüche 1 bis 6, wobei die Gaszufuhrrate von Wasserstoff und Kohlendioxid in das Reaktionsgefäß mindestens 20 vvm insgesamt ist.

9. Das Verfahren nach einem der vorangehenden Ansprüche, ferner umfassend mindestens eine Methanproduktionsphase, wobei in der mindestens einen Methanproduktionsphase das Verhältnis des Partialdrucks von Wasserstoff zu dem Partialdruck von Kohlendioxid im Reaktionsgefäß bei 5:1 oder höher gehalten wird (Teile Wasserstoff : Teile Kohlendioxid).

10. Das Verfahren nach einem der vorangehenden Ansprüche, wobei das Volumenverhältnis von Wasserstoff zu Kohlendioxid in der Gaszufuhr, die in das Reaktionsgefäß eingeführt wird, 4:1 ist.

11. Das Verfahren nach einem der vorangehenden Ansprüche, wobei das Verfahren unter Verwendung eines Systems durchgeführt wird, das umfasst

mindestens eine Vorrichtung zum Erzeugen elektrischer Energie aus einer erneuerbaren und/oder nicht erneuerbaren Energiequelle,
mindestens eine Vorrichtung zum Erzeugen von Wasserstoff und/oder Sauerstoff durch die Elektrolyse von Wasser und/oder Salzlake, und
mindestens einen Bioreaktor umfassend ein Reaktionsgefäß, das zum Züchten, Fermentieren und/oder Kultivieren von methanogenen Mikroorganismen geeignet ist, und mindestens eine Vorrichtung zum Messen und/oder Kontrollieren einer Gaszufuhr von Wasserstoff und Kohlendioxid in das Reaktionsgefäß,
wobei der mindestens eine Bioreaktor ferner eine Vorrichtung zum Kontrollieren und/oder Messen eines Absolutdrucks in dem Reaktionsgefäß von mindestens 5 bar umfasst.

12. Das Verfahren nach Anspruch 11, wobei das System ferner mindestens eine Vorrichtung zur sauerstoffangereicherten Verbrennung oder Vergasung zur Erzeugung von Kohlendioxid umfasst, wobei das Kohlendioxid in den Bioreaktor überführt wird, und/oder ferner mindestens eine Vorrichtung zur Rückgewinnung, zur Reinigung, zum Anreichern, Speichern, Wiederverwerten und/oder zum Weiterverarbeiten von Abgas, Wasserstoff, Wasser, Sauerstoff, Chlor, Kohlendioxid, $H_2S$, Natriumsulfit, methanogene Mikroorganismen, verwendete Medien und Medienkomponenten, Methan und anderer Substanzen des Prozesses aus dem Reaktionsgefäß und dem Bioreaktor umfasst.

13. Das Verfahren nach Anspruch 12, wobei die Vorrichtung zur sauerstoffangereicherten Verbrennung oder Vergasung den durch Elektrolyse von Wasser erzeugten Sauerstoff verwendet.

14. Das Verfahren nach einem der Ansprüche 11 bis 13, wobei die erneuerbare Energiequelle Sonnenenergie, Windkraft, Wellenkraft, Gezeitenkraft, Wasser-/Hydrokraft, Erdwärme, Biomasse- und/oder Biokraftstoff-Verbrennung umfasst.

15. Das Verfahren nach einem der Ansprüche 11 bis 14, wobei die methanogenen Mikroorganismen methanogene Archaeen sind.

## Revendications

1. Procédé pour la production de méthane comprenant la mise en contact de micro-organismes méthanogènes dans une cuve de réaction avec de l'hydrogène et du dioxyde de carbone, dans lequel le débit d'alimentation en gaz d'hydrogène et de dioxyde de carbone dans la cuve de réaction est d'au moins 1,2 vvm au total, et dans lequel, à l'intérieur de la cuve de réaction, une pression absolue d'au moins 5,0 bar (4,0 barg) est appliquée.

2. Procédé selon la revendication 1, dans lequel le procédé comprend l'une ou plusieurs des étapes suivantes :

a) la génération d'énergie électrique à l'aide d'une source d'énergie renouvelable ; et/ou

b) l'utilisation, partiellement ou complètement, de ladite énergie électrique pour l'électrolyse d'eau et/ou de saumure, ce qui permet de produire de l'hydrogène et/ou de l'oxygène ; et/ou

c) l'utilisation, partiellement ou complètement, dudit hydrogène et dudit dioxyde de carbone, et facultativement, partiellement ou complètement, de ladite énergie électrique pour la production de méthane.

3. Procédé selon la revendication 2, dans lequel la source d'énergie renouvelable comprend l'énergie solaire, l'énergie éolienne, l'énergie des vagues, l'énergie marémotrice, l'énergie de l'eau ou hydroélectrique, l'énergie géothermique, la combustion de biomasse et de biocarburant, ou des combinaisons de celles-ci.

4. Procédé selon la revendication 2 ou 3, comprenant en outre l'étape d), dans laquelle, partiellement ou complètement, l'oxygène est utilisé pour la production, partiellement ou complètement, du dioxyde de carbone par combustion ou gazéification enrichie en oxygène.

5. Procédé selon l'une quelconque des revendications précédentes, dans lequel les micro-organismes méthanogènes sont des archées méthanogènes sélectionnées parmi le groupe constitué de *Methanobacterium alcaliphilum, Methanobacterium bryantii, Methanobacterium congolense, Methanobacterium defluvii, Methanobacterium espanolae, Methanobacterium formicicum, Methanobacterium ivanovii, Methanobacterium palustre, Methanobacterium thermaggregans, Methanobacterium uliginosum, Methanobrevibacter acididurans, Methanobrevibacter arbor iphilicus, Methanobrevibacter gottschalkii, Methanobrevibacter olleyae, Methanobrevibacter ruminantium, Methanobrevibacter smithii, Methanobrevibacter woesei, Methanobrevibacter wolinii, Methanothermobacter marburgensis, Methanothermobacter thermoautotrophicus, Methanobacterium thermoautotrophicus, Methanothermobacter thermoflexus, Methanothermobacter thermophilics, Methanothermobacter wolfeii, Methanothermus sociabilis, Methanocorpusculum bavaricum, Methanocorpusculum parvum, Methanoculleus chikuoensis, Methanoculleus submarinus, Methanogenium frigidum, Methanogenium liminatans, Methanogenium marinum, Methanosarcina acetivorans, Methanosarcina barkeri, Methanosarcina mazei, Methanosarcina thermophila, Methanomicrobium mobile, Methanocaldococcus jannaschii, Methanococcus aeolicus, Methanococcus maripaludis, Methanococcus vannielii, Methanococcus voltaei, Methanothermococcus thermolithotrophicus* et *Methanopyrus kandleri,* et des mélanges de celles-ci.

6. Procédé selon l'une quelconque des revendications précédentes, dans lequel le débit d'alimentation en gaz d'hydrogène et de dioxyde de carbone dans la cuve de réaction est d'au moins 2,0 vvm au total ou est dans la plage de 1,2 vvm à 25 vvm au total.

7. Procédé selon l'une quelconque des revendications précédentes, dans lequel le débit d'alimentation en gaz d'hydrogène et de dioxyde de carbone dans la cuve de réaction est dans la plage de 2,0 vvm à 8,0 vvm au total et la pression à l'intérieur de la cuve de réaction est réglée sur une valeur dans la plage de 5 bar à 1000 bar.

8. Procédé selon l'une quelconque des revendications 1 à 6, dans lequel le débit d'alimentation en gaz d'hydrogène et de dioxyde de carbone dans la cuve de réaction est d'au moins 20 vvm au total.

9. Procédé selon l'une quelconque des revendications précédentes, comprenant en outre au moins une phase de production de méthane, dans lequel, lors de l'au moins une phase de production de méthane, le rapport de la pression partielle d'hydrogène sur la pression partielle de dioxyde de carbone à l'intérieur de la cuve de réaction est maintenu à 5:1 ou plus (parties hydrogène : parties dioxyde de carbone).

10. Procédé selon l'une quelconque des revendications précédentes, dans lequel le rapport volumétrique d'hydrogène sur dioxyde de carbone dans l'alimentation en gaz introduite dans la cuve de réaction est de 4:1.

11. Procédé selon l'une quelconque des revendications précédentes, dans lequel le procédé est mis en œuvre à l'aide d'un système comprenant au moins un dispositif pour la génération d'énergie électrique à partir d'une source d'énergie renouvelable et/ou non renouvelable,

au moins un dispositif pour la production d'hydrogène et/ou d'oxygène par l'électrolyse d'eau et/ou de saumure, et

au moins un bioréacteur comprenant une cuve de réaction appropriée pour la croissance, la fermentation et/ou la culture de micro-organismes méthanogènes et au moins un dispositif pour la mesure et/ou la régulation d'une alimentation en gaz d'hydrogène et de dioxyde de carbone dans la cuve de réaction,

dans lequel l'au moins un bioréacteur comprend en outre un dispositif pour la régulation et/ou la mesure d'une pression absolue à l'intérieur de la cuve de réaction d'au moins 5 bar.

12. Procédé selon la revendication 11, dans lequel le système comprend en outre au moins un dispositif pour une combustion ou une gazéification enrichie en oxygène pour la production de dioxyde de carbone, ledit dioxyde de carbone étant transféré au bioréacteur, et/ou comprenant en outre au moins un dispositif pour la récupération, la purification, l'enrichissement, le stockage, le recyclage et/ou le traitement ultérieur d'un effluent gazeux, d'hydrogène, d'eau, d'oxygène, de chlore, de dioxyde de carbone, de $H_2S$, de sulfite de sodium, de micro-organismes méthanogènes, d'un milieu usagé et de composants de milieu usagés, de méthane et d'autres substances du traitement provenant de la cuve de réaction et du bioréacteur.

13. Procédé selon la revendication 12, dans lequel le dispositif pour une combustion ou une gazéification enrichie en oxygène utilise l'oxygène produit par l'électrolyse d'eau.

14. Procédé selon l'une quelconque des revendications 11 à 13, dans lequel la source d'énergie renouvelable comprend l'énergie solaire, l'énergie éolienne, l'énergie des vagues, l'énergie marémotrice, l'énergie de l'eau/hydroélectrique, l'énergie géothermique, la combustion de biomasse et/ou de biocarburant.

15. Procédé selon l'une quelconque des revendications 11 à 14, dans lequel les micro-organismes méthanogènes sont des archées méthanogènes.

**Figure 1**

**Figure 2**

**Figure 3**

**Figure 4**

Figure 5

Figure 6

**Figure 7**

**Figure 8**

Figure 9

**Figure 10**

# Figure 11

**Figure 12**

**Figure 13**

**Figure 14**

**Figure 15**

**Figure 16**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100269498 A
- DE 102007037672
- US 4883753 A
- WO 2008094282 A
- EP 1574581 A
- US 20090130734 A
- WO 2012110256 A

**Non-patent literature cited in the description**

- **RITTMANN et al.** *Biomass and Bioenergy*, 2012, vol. 36, 293-301
- **YANO et al.** *Journal of Fermentation Technology*, 1986, vol. 64 (5), 383-387
- **NISHIMURA et al.** *Journal of Fermentation and Bioengineering*, 1991, vol. 72 (4), 280-284
- Water Treatment, Principles and Design. John Wiley & Sons, 2005
- **BAILEY, OLLIS**. Biochemical Engineering Fundamentals. McGraw-Hill Science, 1986